# EUROPEAN PATENT APPLICATION

(11) **EP 3 578 636 A1**
(43) Date of publication of application: **11.12.2019**
(21) Application number: 18176325.1
(22) Date of filing: 06.06.2018
(51) Int. Cl.: C12N 1/16, C12P 7/44, C12R 1/645

(54) **MORPHOLOGIC ENGINEERING IN BASIDIOMYCOTA**

(71) Applicant: Rheinisch-Westfälische Technische Hochschule Aachen (RWTH), 52062 Aachen (DE)
(72) Inventor: Hosseinpour Tehrani, Hamed, 52068 Aachen (DE); Blank, Lars M., 44227 Dortmund (DE); Wierckx, Nick, 6268NV Bemelen (NL)
(74) Representative: Schiweck Weinzierl Koch Patentanwälte Partnerschaft mbB

(57) **Abstract**

The present invention relates to a method of producing a secreted, non-proteinaceous biomolecule, comprising culturing a fungal host cell belonging to the division of Basidiomycota in culture medium under conditions allowing the production of said biomolecule, wherein the fungal host cell is due to genetic engineering not capable to switch from budding growth to filamentous growth in its vegetative stage during said culturing; and obtaining the biomolecule produced by the fungal host cell from the supernatant of said culture medium. Further, the present invention relates to the use of a fungal host cell for the production of a biomolecule.

## Description

This application contains a Sequence Listing in computer readable form, which is incorporated herein by reference.

### TECHNICAL FIELD OF THE INVENTION

The present invention relates to a method of producing a secreted, non-proteinaceous biomolecule, comprising culturing a fungal host cell belonging to the division of Basidiomycota in culture medium under conditions allowing the production of said biomolecule, wherein the fungal host cell is due to genetic engineering not capable to switch from budding growth to filamentous growth in its vegetative stage during said culturing; and obtaining the biomolecule produced by the fungal host cell from the supernatant of said culture medium. Further, the present invention relates to the use of a fungal host cell for the production of a biomolecule.

### BACKGROUND ART

The biotechnological production of different biomolecules such as organic acids is an emerging approach to the production of building block chemicals. The industrial and fermentative production of biomolecules such as organic acids as an important class of the biomolecules is mostly produced industrially by filamentous fungi such as *Aspergillus niger* or *Aspergillus terreus.* Due to the heavily process-controlled fermentation, the production of biomolecules incurs high costs. pH values play an important role for the production of many biomolecules such as organic acids, since low pH values, which are used in production have an extremely advantage on the downstream process, thus having a significant influence on production costs. Further, low pH values also reduce the risk of contamination.

However, *Aspergillus* for example is a filamentous fungus, and this filamentous growth influences the production of said biomolecule such as organic acids. Thus, *Aspergillus* only produces certain biomolecules under certain morphological conditions, which results in the overall regulation and control of the process for the production of said biomolecule.

There are several disadvantages related to fermentation of filamentous fungi in general, and with *Aspergillus terreus* specifically (Willke T, and Vorlop K.D., Appl Microbiol Biotechnol. (2001) 56, 3-4, 289-295). Filamentous fungi are particularly problematic in that their morphology can be difficult to be controlled in fermentation systems.

Besides *Aspergillus* the division of Basidiomycota (e.g., the family of Ustilaginaceae, the family of Dacrymycetacea etc.) may also be promising candidates for producing biomolecules such as organic acids, in particular itaconic acid. However, while some Basidiomycota such as *Ustilago maydis* grow in a yeast-like form, they can change their morphology into filamentous growth under certain stress conditions, also resulting in a negative influence on the production of biomolecules.

It is therefore highly important to investigate alternative means and methods for producing biomoleucles such as organic acids by Basidiomycota.

Therefore, the objective of the present invention is to comply with this need.

The solution of the present invention is described in the following, exemplified in the appended examples, illustrated in the figures and reflected in the claims.

### SUMMARY OF THE INVENTION

The present invention is based on the elucidation of the production of a secreted, non-proteinaceous biomolecule in a fungal host cell belonging to the division of Basidiomycota. Here, while allowing for the production of said biomolecule during the culture process of said fungal host cell, the fungal host cell is genetically engineered, thus not being capable to switch from yeast-similar growth (budding growth) to filamentous growth in its vegetative stage. The present inventors found that this is achieved by knocking-out one or more polynucleotide sequence(s) encoding a polypeptide being involved in a signal transduction pathway which regulates the switching from budding growth to filamentous growth of said fungus. Thus, by knocking-out specific proteins involved in said particular signal transduction pathway, a positive effect on the production of said biomolecule was demonstrated. Further, by morphologic engineering of said fungal host cell, production processes of said biomolecule are being notably simplified, also having considerable influence on the process chain and on process costs in general.

Thus, the present invention relates to a method of producing a secreted, non-proteinaceous biomolecule, comprising culturing a fungal host cell belonging to the division of Basidiomycota in culture medium under conditions allowing the production of said biomolecule, wherein the fungal host cell is due to genetic engineering not capable to switch from budding growth to filamentous growth in its vegetative stage during said culturing; and obtaining the biomolecule produced by the fungal host cell from the supernatant of said culture medium.

Additionally, the present invention may comprise the method as mentioned above, wherein the biomolecule is an organic acid, a polyol, or a glycolipid.

The organic acid may be itaconic acid, malic acid, hydroxyparaconic acid, citramalic acid, aconitic acid or itatararic acid. The polyol may be erythritol or mannitol. The glycolipid may be ustilagic acid or mannosylerythritol lipid.

The present invention may further comprise the method of producing a secreted, non-proteinaceous biomolecule, wherein the fungal host cell belonging to the division of Basidiomycota is from the family of Ustilaginaceae, the family of Dacrymycetaceae, or from the family of Moniliellaceae. The fungal host cell from the family of Ustilaginaceae may be from the genus *Ustilago, Pseudozyma, or Sporisorium.* Further, the fungal host cell from the genus *Ustilago* may be *Ustilago maydis, Ustilago cynodontis, Ustilago trichophora, Ustilago vetiveriae* or *Ustilago xerochloae.*
The fungal host cell from the family of Dacrymycetaceae may be from the genus *Dacryopinax, Dacrymyces, Ditiola, Guepiniopsis* or *Femsjonia.* The fungal host cell from the genus *Dacryopinax* may be *Dacryopinax spathularia* or *Dacryopinax primogenitus.* The fungal host cell from the genus *Dacrymyces* may be *Dacrymyces stillatus* or *Dacrymyces chrysocomus.* The fungal host cell from the genus *Guepiniopsis* may be *Guepiniopsis buccina* and the fungal host cell from the genus *Femsjonia* may be *Femsjonia luteo-alba.*

Further, also contemplated by the present invention may be the method as indicated above, wherein the fungal host cell which is no longer capable of switching from budding growth to filamentous growth in its vegetative stage during said culturing is genetically engineered by knocking-out one or more polynucleotide sequence(s) encoding a polypeptide being involved in a signal transduction pathway which regulates the switching from budding growth to filamentous growth of said fungus.

The present invention may comprise the method above, wherein the one or more polynucleotide sequence(s) encoding a polypeptide is
a) a polynucleotide sequence encoding a polypeptide having an amino acid sequence having at least 25% sequence identity to the amino acid sequence of SEQ ID NO. 1, said polynucleotide sequence encoding a polypeptide or fragment thereof having pheromone-responsive MAP kinase kinase activity,
b) a polynucleotide sequence encoding a polypeptide having an amino acid sequence having at least 25% sequence identity to the amino acid sequence of SEQ ID NO. 2, said polynucleotide sequence encoding a polypeptide or fragment thereof having pheromone-responsive MAP kinase pathway-interacting activity,
c) a polynucleotide sequence encoding a polypeptide having an amino acid sequence having at least 25% sequence identity to the amino acid sequence of SEQ ID NO. 3, said polynucleotide sequence encoding a polypeptide or fragment thereof having pheromone-responsive MAP kinase activity,
d) a polynucleotide sequence encoding a polypeptide having an amino acid sequence having at least 25% sequence identity to the amino acid sequence of SEQ ID NO. 4, said polynucleotide sequence encoding a polypeptide or fragment thereof having cAMP-dependent protein kinase regulator activity,
e) a polynucleotide sequence encoding a polypeptide having an amino acid sequence having at least 25% sequence identity to the amino acid sequence of SEQ ID NO. 5, said polynucleotide sequence encoding a polypeptide or fragment thereof having pheromone-responsive MAP kinase pathway-interacting activity,
f) a polynucleotide sequence encoding a polypeptide having an amino acid sequence having at least 25% sequence identity to the amino acid sequence of SEQ ID NO. 6, said polynucleotide sequence encoding a polypeptide or fragment thereof having pheromone-responsive MAPKK kinase activity, or
g) a polynucleotide sequence encoding a polypeptide having an amino acid sequence having at least 25% sequence identity to the amino acid sequence of SEQ ID NO. 7, said polynucleotide sequence encoding a polypeptide or fragment thereof having pheromone-responsive transcription factor activity.

Preferably, the one or more polynucleotide sequence(s) encoding a polypeptide is
a) polynucleotide sequence encoding a polypeptide having an amino acid sequence having at least 25% sequence identity to the amino acid sequence of SEQ ID NO. 1, said polynucleotide sequence encoding a polypeptide or fragment thereof having pheromone-responsive MAP kinase kinase activity,
b) a polynucleotide sequence encoding a polypeptide having an amino acid sequence having at least 25% sequence identity to the amino acid sequence of SEQ ID NO. 2, said polynucleotide sequence encoding a polypeptide or fragment thereof having pheromone-responsive MAP kinase pathway-interacting activity, or
c) a polynucleotide sequence encoding a polypeptide having an amino acid sequence having at least 25% sequence identity to the amino acid sequence of SEQ ID NO. 3, said polynucleotide sequence encoding a polypeptide or fragment thereof having pheromone-responsive MAP kinase activity.

Additionally, the present invention may comprise the method as indicated above, wherein the capability of switching from budding growth to filamentous growth is determined by the influence of sunflower oil, low pH, high glucose concentration or glycerol concentration during culturing of the fungal host cell.

Further, the present invention comprises use of a fungal host cell belonging to the division of Basidiomycota for the production of a biomolecule, wherein the fungal host cell is due to genetic engineering not capable of switching from budding growth to filamentous growth in its vegetative stage.

### BRIEF DESCRIPTION OF THE FIGURES

**Fig. 1****: Maps of deletion constructs for ras2, fuz7 and ubc3.**
   Plasmid map comprising pJET1.2FRT-KO-ras2 which contains the deletion construct for the gene *ras2* (**A**), pJET1.2FRT-KO-*fuz7* which contains the deletion construct for the gen *fuz7* (**B**) and pJET1.2FRT-KO-*ubc3* which contains the deletion construct for the gene *ubc3* (**C**).
**Fig. 2****: Microscopy images of *U. cynodontis* NBRC 9727, *U. cynodontis* Δras2**, ***U*. *cynodontis* Δfuz7 and *U. cynodontis* Δubc3 in production medium A after 48h.**
   In production medium A *U. cynodontis* NBRC9727 already showed a strong filamentous behavior after 24 h and kept it for the whole cultivation time (for 95 hours in total). Compared to the wildtype, the deletion strains did not show any filamentous growth behavior in medium A, instead they showed a yeast like morphology for the whole cultivation time.

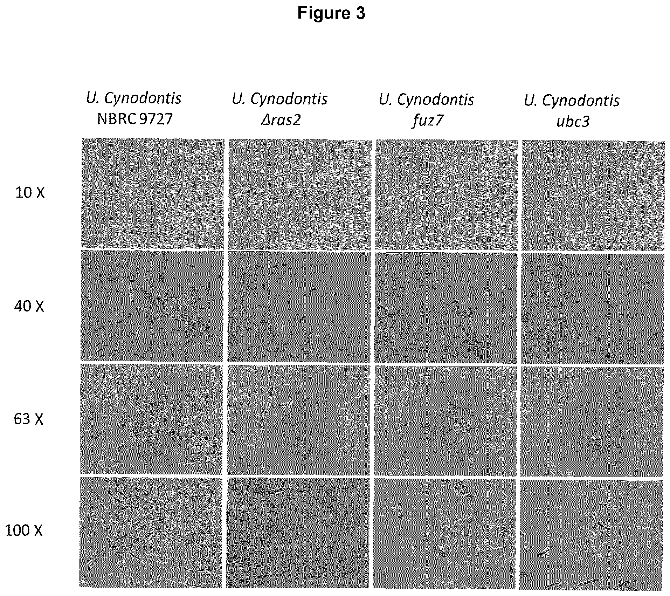

**Fig. 3****: Microscopy images of *U. cynodontis* NBRC 9727, *U. cynodontis* Δras2, *U*. *cynodontis* Δfuz7 and *U. cynodontis* Δubc3 in production medium D after 72h.**
   In production medium D strong filamentous growth was observed for *U. cynodontis* NBRC 9727. At pH-values below 3 stronger filamentous growth behavior could be observed for *U. cynodontis* NBRC 9727. In contrast, no filamentous growth was observed for *U. cynodontis* Δ*ras2, U. cynodontis* Δ*fuz7,* and *U. cynodontis* Δ*ubc3.*

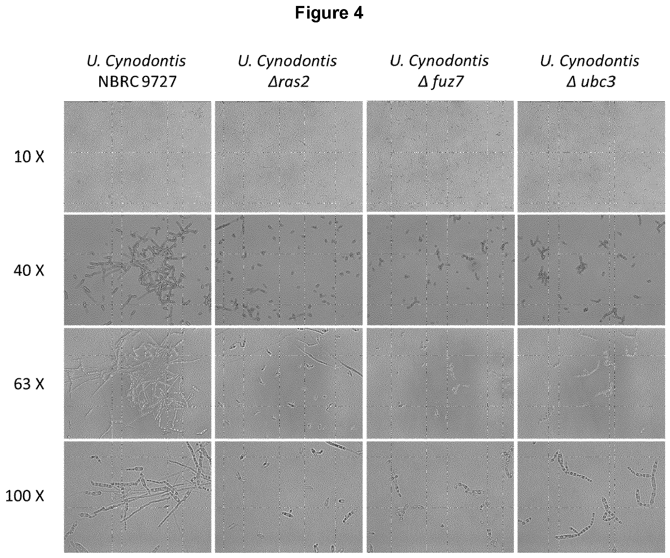

**Fig. 4****: Microscopy images of *U. cynodontis* NBRC 9727, *U. cynodontis* Aras2, *U*. *cynodontis* Δfuz7 and *U. cynodontis* Δubc3 in production medium C after 72h.**
   In production medium C strong filamentous growth was observed for *U. cynodontis* NBRC 9727. At pH-values below 3 stronger filamentous growth behavior could be observed for *U. cynodontis* NBRC 9727. In contrast, no filamentous growth was observed for *U. cynodontis* Δ*ras2, U. cynodontis* Δ*fuz7,* and *U. cynodontis* Δ*ubc3.*

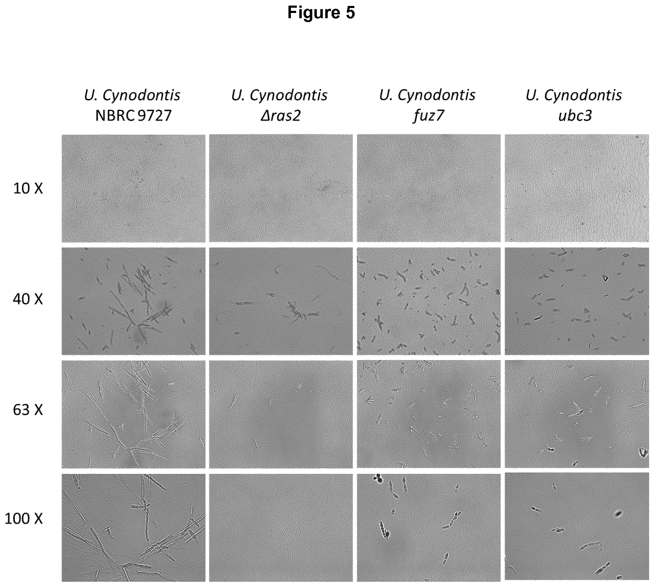

**Fig. 5****: Microscopy images of *U. cynodontis* NBRC 9727, *U. cynodontis* Aras2, *U*. *cynodontis* Δfuz7 and *U. cynodontis* Δubc3 in production medium B after 72h.** In production medium B strong filamentous growth was observed for *U. cynodontis* NBRC 9727.
   At pH-values below 3 stronger filamentous growth behavior could be observed for *U. cynodontis* NBRC 9727. In contrast, no filamentous growth was observed for *U. cynodontis* Δ*ras2, U. cynodontis* Δ*fuz7,* and *U. cynodontis* Δ*ubc3.*

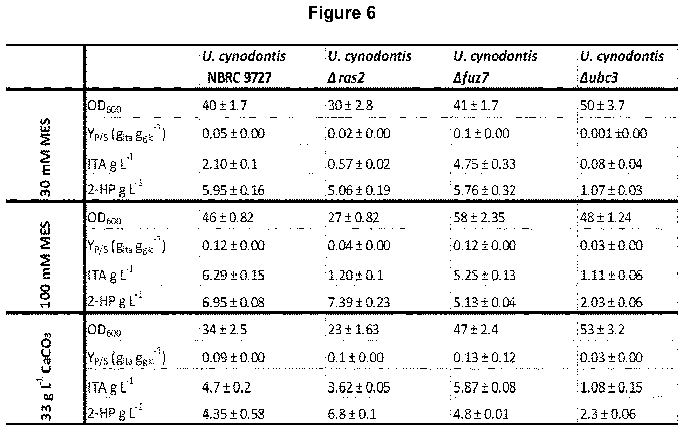

**Fig. 6****: Various end point values for *U. cynodontis* NBRC 9727, *U. cynodontis* Aras2, *U. cynodontis* Δfuz7 and *U. cynodontis* Δubc3 in production medium B, C and D after 96h.**
   All strains produce biomolecules under the selected conditions. Notably, the production of some biomolecules is improved by the deletion of *ras2, fuz7,* and *ubc3.* For instance, *U. cynodontis* Δ*fuz7* produces over two-fold more itaconate than *U. cynodontis* NBRC 9727 in medium with 30 mM MES buffer.

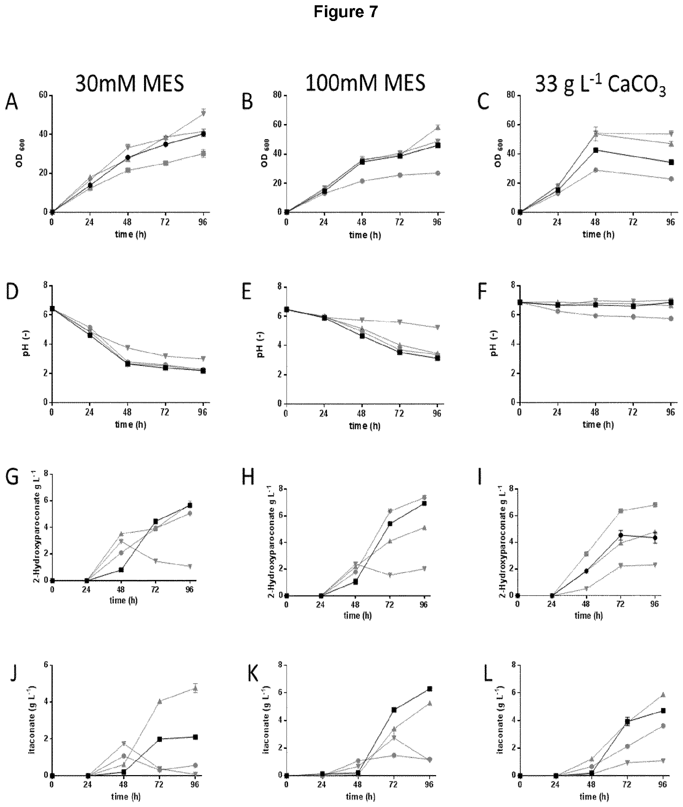

**Fig. 7****: Process values for OD₆₀₀, pH, 2-hydroxyparaconate and itaconate over time for *U. cynodontis* NBRC 9727, *U. cynodontis* Δras2, *U. cynodontis* Δfuz7 and *U. cynodontis* Δubc3 in production medium B, C and D.**
   *U. cynodontis* NBRC 9727 is depicted as circle (●), *U. cynodontis* Δ*ras2* as square (▪), *U. cynodontis* Δ*fuz7* as triangle (▲) and *U. cynodontis* Δ*ubc3* as triangle (▼). OD values are depicted in A, B and C, pH values in D, E and F, Hydroxyparaconate production in H, H and I and itaconic production in J, K and L. In all three production mediums (B, C and D) *U. cynodontis* Δ*ubc3* showed the highest OD600 values for biomass, but also showed significant lower itaconic acid concentrations compared to the wildtype. This phenotype was also observed for *U. cynodontis* Δ*ras2.* In contrast, *U. cynodontis* Δ*fuz7* showed in all production media (B, C and D) higher values for itaconic acid compared to the wildtype.

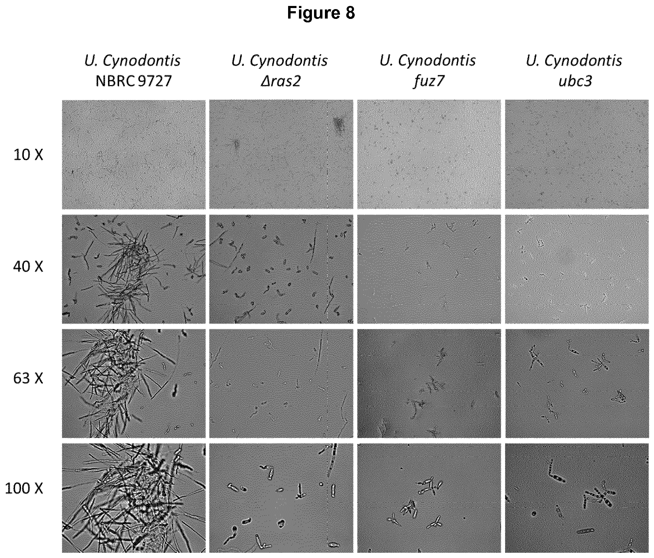

**Fig. 8****: Microscopy images of *U. cynodontis* NBRC 9727, *U. cynodontis* Aras2, *U*. *cynodontis* Δfuz7 and *U. cynodontis* Δubc3 in production medium G after 72h.**
   In production medium G *U. cynodontis* NBRC 9727 showed a strong filamentous growth behavior. Further, *U. cynodontis* Δ*ras2, U. cynodontis* Δ*fuz7* and *U. cynodontis* Δ*ubc3* grew yeast-like in said medium.

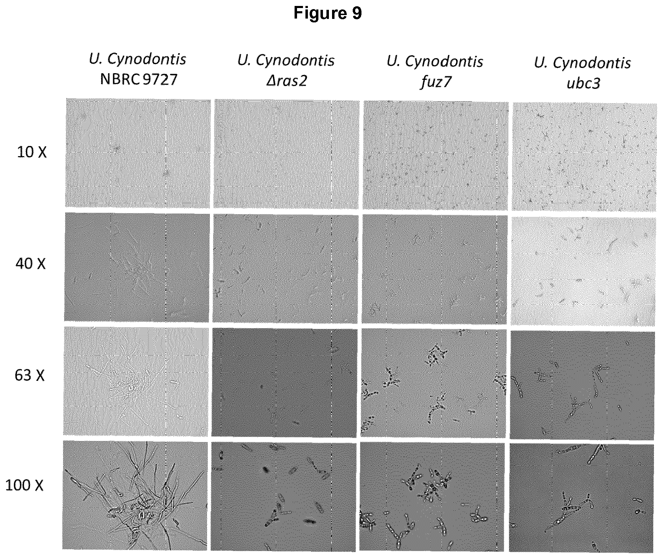

**Fig. 9****: Microscopy images of *U. cynodontis* NBRC 9727, *U. cynodontis* Δras2, *U*. *cynodontis* Δfuz7 and *U. cynodontis* Δubc3 in production medium F after 72h.**
   In production medium F *U. cynodontis* NBRC 9727 showed a strong filamentous growth behavior. Further, *U. cynodontis* Δ*ras2, U. cynodontis* Δ*fuz7* and *U. cynodontis* Δ*ubc3* grew yeast-like in said medium.

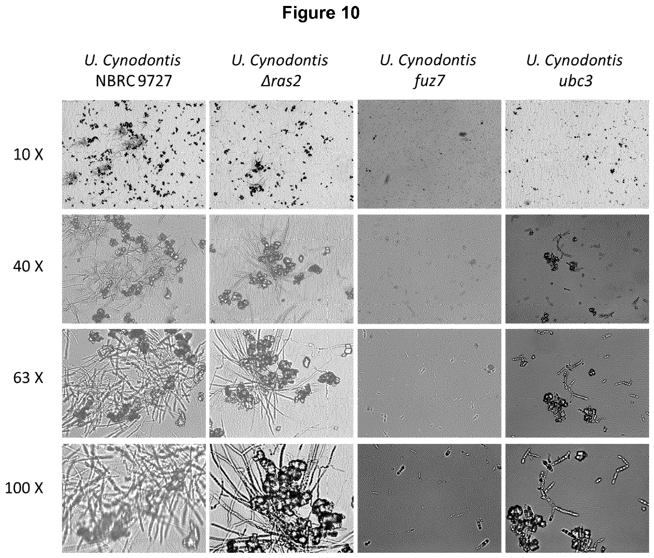

**Fig. 10****: Microscopy images of *U. cynodontis* NBRC 9727, *U. cynodontis* Aras2, *U*. *cynodontis* Δfuz7 and *U. cynodontis* Δubc3 in production medium E after 72h.**
   In production medium E *U. cynodontis* NBRC 9727 showed a strong filamentous growth behavior. Further, *U. cynodontis* Δ*ras2* also showed significant filamentous growth. However, *U*. *cynodontis* Δ*fuz7* and *U. cynodontis* Δ*ubc3* grew yeast-like in said medium.

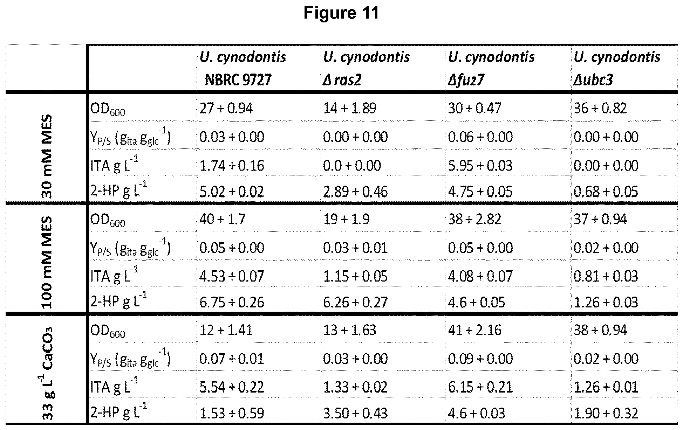

**Fig. 11****: Various end point values for *U. cynodontis* NBRC 9727, *U. cynodontis* Aras2, *U. cynodontis* Δfuz7 and *U. cynodontis* Δubc3 in production medium E, F and G after 96h.**
   All strains produce biomolecules under the selected conditions. Notably, the production of some biomolecules is improved by the deletion of *ras2, fuz7,* and *ubc3.* For instance, *U. cynodontis* Δ*fuz7* produces over two-fold more itaconate than *U. cynodontis* NBRC 9727 in medium with 30 mM MES buffer.

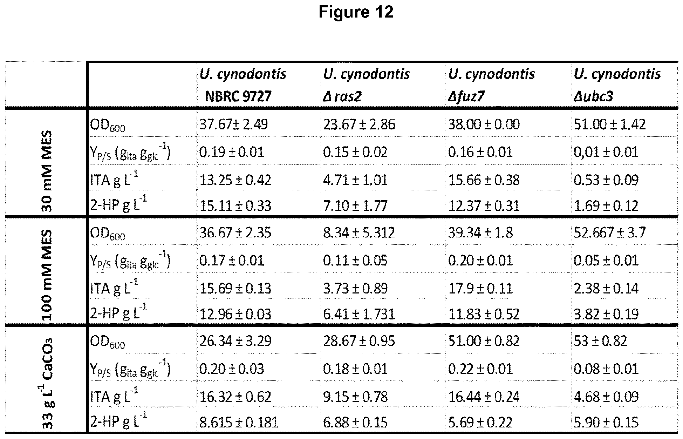

**Fig. 12****: Various end point values for *U. cynodontis* NBRC 9727, *U. cynodontis* Δras2, *U. cynodontis* Δfuz7 and *U. cynodontis* Δubc3 in production medium E, F and G after 216h.**
   All strains produce biomolecules under the selected conditions. Notably, the production of some biomolecules is improved by the deletion of *ras2, fuz7,* and *ubc3.* For instance, *U. cynodontis* Δ*fuz7* produces more itaconate than *U. cynodontis* NBRC 9727 in medium with 30 mM MES buffer.

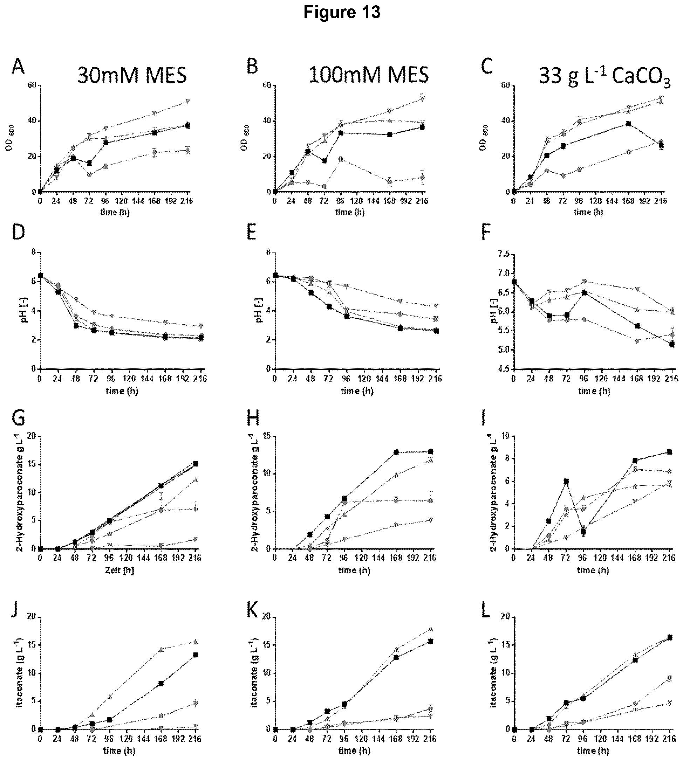

**Fig. 13****: Process values for OD₆₀₀, pH, 2-hydroxyparaconate and itaconate over time for *U. cynodontis* NBRC 9727, *U. cynodontis* Aras2, *U. cynodontis* Δfuz7 and *U. cynodontis* Δubc3 in production medium E, F and G.**
   *U. cynodontis* NBRC 9727 is depicted as a circle (●), *U. cynodontis* Δ*ras2* as a square (■), *U. cynodontis* Δ*fuz7* as a triangle (▲) and *U. cynodontis* Δ*ubc3* as a triangle (▼). OD values are depicted in A, B and C, pH values in D, E and F, Hydroxyparaconate production in H, H and I and itaconic production in J, K and L. The higher initial concentration of glucose (200 g L⁻¹) had an influence on biomass formation and on 2-hydroxyparaconate, but no significant differences in itaconic acid production compared to the lower initial glucose concentrations (50 g L⁻¹ glucose) was observed.

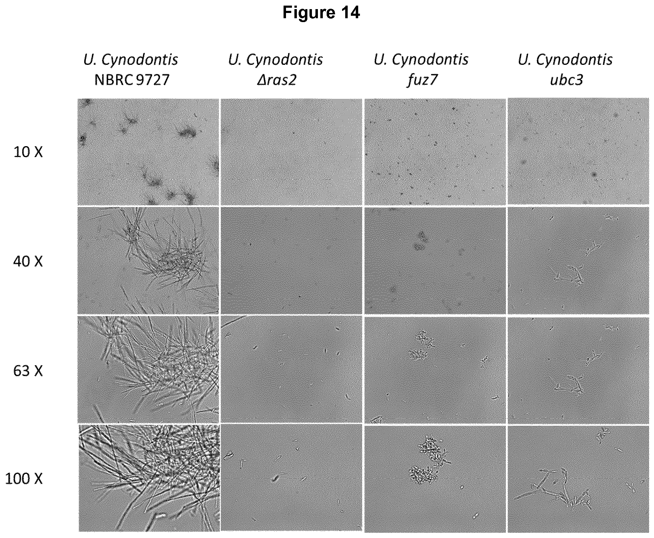

**Fig. 14****: Microscopy images of *U. cynodontis* NBRC 9727, *U. cynodontis* Aras2, *U*. *cynodontis* Δfuz7 and *U. cynodontis* Δubc3 in production medium H after 48h.**
   Strong filamentous growth could be observed during the whole cultivation for *U. cynodontis* NBRC 9727, whereas *U. cynodontis* Δ*ras2, U. cynodontis* Δ*fuz7 and U. cynodontis* Δ*ubc3* grew yeast-like. In particular, *U. cynodontis* Δ*fuz7* grew yeast-like, where the cells were organized in a clustered formation.

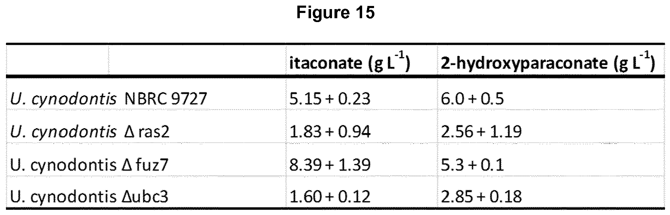

**Fig. 15****: Various end point values for *U. cynodontis* NBRC 9727, *U. cynodontis* Aras2, *U. cynodontis* Δfuz7 and *U. cynodontis* Δubc3 in production medium H after 384 h.**
   Itaconic and Hydroxyparaconate production for *U. cynodontis* NBRC 9727, *U. cynodontis* Δras2, *U. cynodontis* Δfuz7 and *U. cynodontis* Δubc3. For itaconic acid production *U. cynodontis* Δ*fuz7* reached the highest titer with 8.39 g L⁻¹.

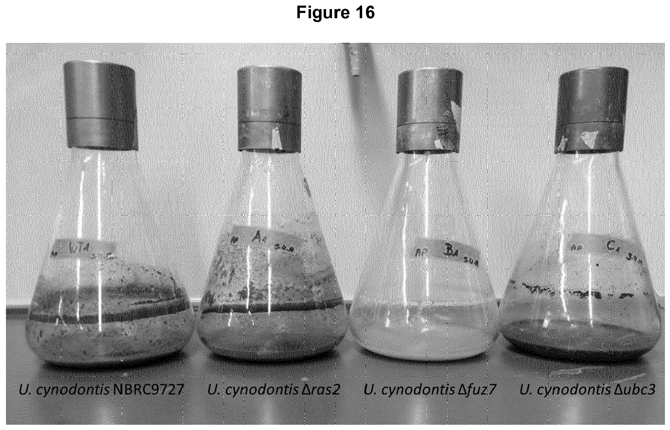

**Fig. 16****: Shaking flasks after 384 h cultivation in production medium H.**
   *U. cynodontis* Δ*fuz7* kept its yellow color, while *U. cynodontis* NBRC 9727, *U. cynodontis* Δ*ras2 and U. cynodontis* Δ*ubc3* turned black over time. Black coloration can be considered a sign of microbial stress, indicating that *U. cynodontis* Δ*fuz7* is more stress-resistant.

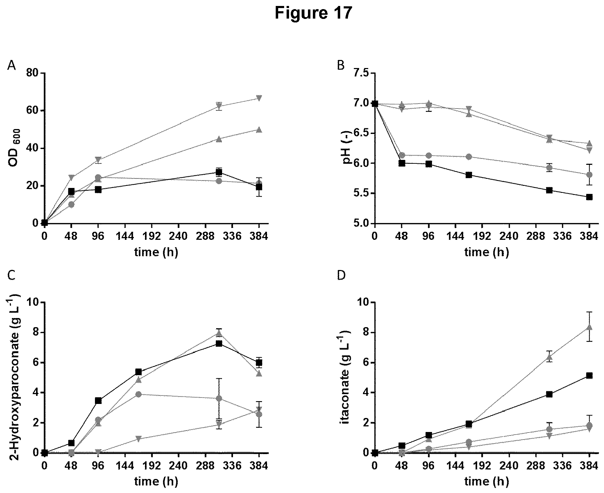

**Fig. 17****: Process values for OD₆₀₀, pH, 2-hydroxyparaconate and itaconate over time for *U. cynodontis* NBRC 9727, *U. cynodontis* Δras2, *U. cynodontis* Δfuz7 and *U. cynodontis* Δubc3 in production medium H.**
   *U. cynodontis* NBRC 9727 is depicted as circle (●), *U. cynodontis* Δ*ras2* as square (■), *U. cynodontis* Δ*fuz7* as triangle (▲) and *U. cynodontis* Δ*ubc3* as triangle (▼). OD values are depicted in A, pH values in B, hydroxyparaconate production in C and itaconate production in D. *U. cynodontis* Δ*fuz7* showed a stable single cell growth behavior in medium H and the best values for itaconic acid production.

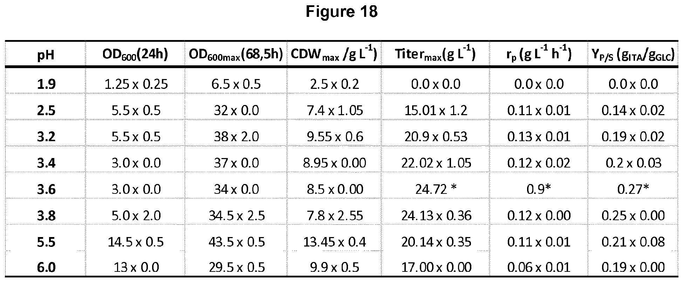

**Fig. 18****: Various end point values for *U. cynodontis* Δfuz7 in a bioreactor in production medium H after 284 h.**
   Depicted are OD₆₀₀ after 24h, Od_{Max} after 68.5, CDW_{Max} (g L⁻¹), Titer_{Max} for itaconate in g L⁻¹, productivity in g L⁻¹ h⁻¹ and the yield for itaconate in g_{ITA}/g_{GLC}. *U. cynodontis* Δ*fuz7* showed at pH = 5.5 the highest rates for biomass formation after 24 h. Said pH of 5.5 was chosen as the optimum for biomass formation. Best production condition for itaconate was at a pH at 3.6. (OD₆₀₀: optical density at 600nm; CDW: cell dry weight)

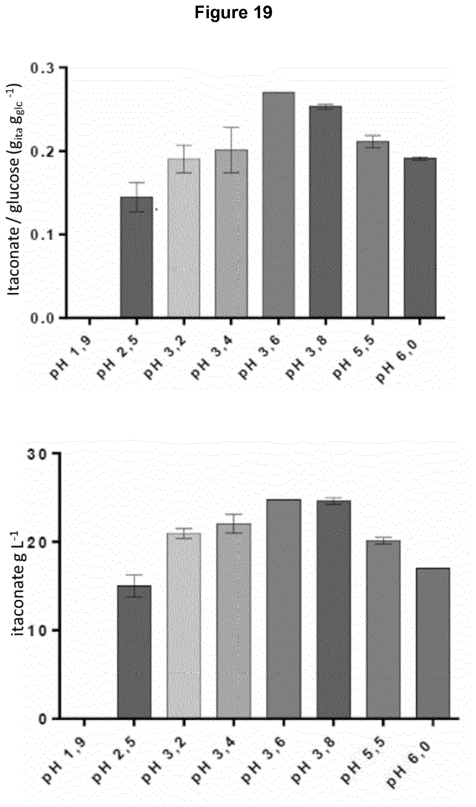

**Fig. 19****: Process values for *U. cynodontis* Δfuz7 at different pH values in** a **bioreactor in production medium H after 284 h.**
   Depicted are yield and titer for itaconic acid production at different pH values for fed batch fermentations. The highest titer of 24.72 g L-1 and a yield of 0.27 gᵢₜₐ g_{glc}⁻¹ could be reached at pH = 3.6. Said pH of 3.6 was therefore chosen as the optimum for itaconic acid production.

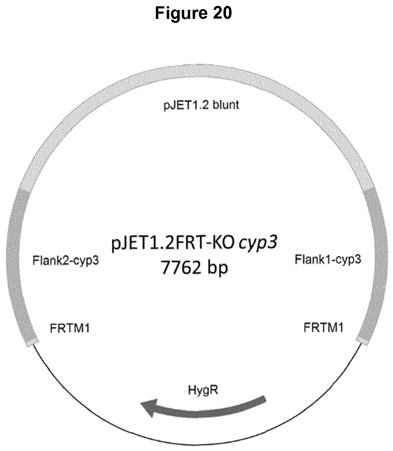

**Fig. 20****: Plasmid map of pJET1.2FRT-KO cyp3.**
   Plasmid map of pJET1.2FRT-KO *cyp3* which contains the deletion construct for the gene *cyp3.*

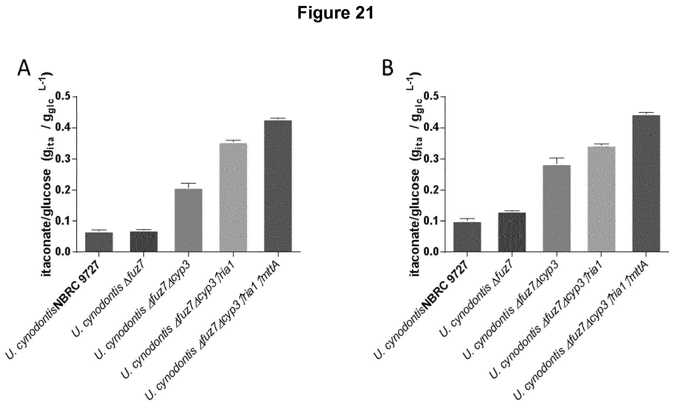

**Fig. 21** **Titer and yield for various *U. cynodontis* strains in production medium C and D.**
   Depicted is the yield for itaconate for buffered medium with 30mM (A) and 100mM (B). It is shown that with metabolic engineering enhancement of itaconic acid is possible in *U. cynodontis.*

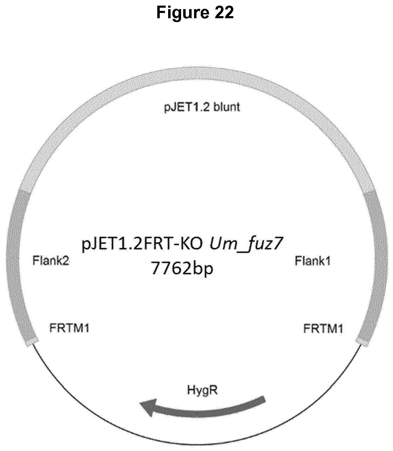

**Fig. 22****: Map of deletion construct for Um_fuz7.**
   Plasmid map of pJET1.2FRT-KO-Um_fuz7 which contains the deletion construct for *fuz7*.

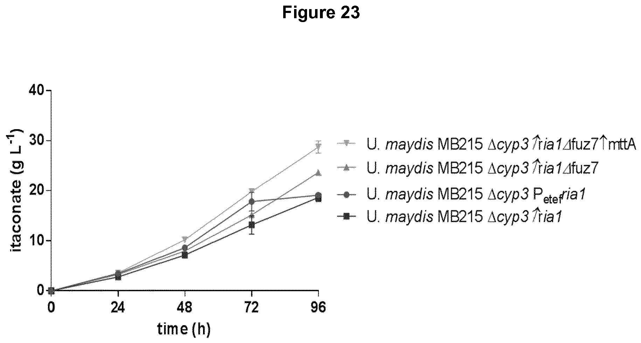

**Fig. 23****: Itaconic acid production in *U. maydis* over time in production medium J.**
   Depicted is itaconic acid titer for various *U. maydis* strains.The newly designed strain *U. maydis* MB215 Δcyp3↑*ria1* reached the same end titer of approximately 19 g L⁻¹ in comparison to the published strain *U. maydis* Δ*cyp3*P_{etef}*ria1* (Geiser et al., 2016 ,https://doi.org/10.1016/j.ymben.2016.10.006). Deletion of *fuz7* in *U. maydis* MB215 Δcyp3↑*ria1* resulted in an increase of itaconate up to 23.6 g L⁻¹ itaconate. Also expression of *mttA* resulted in an increase of itaconate up to 28.4 g L⁻¹.

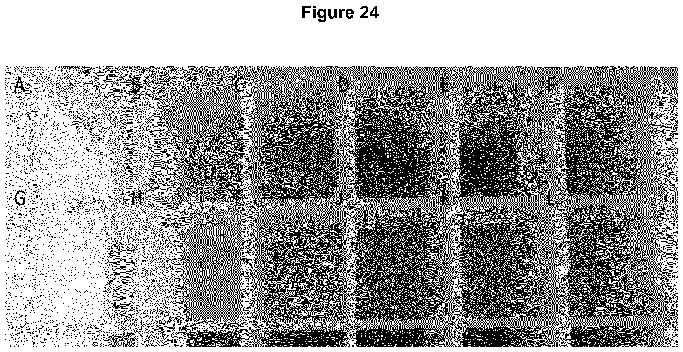

**Fig. 24****: System Duetz® (24 well plates) in production medium J after 48 h.**
   *U. maydis* Δcyp3P_{etef}ria1 (**A**, **B** and **C**), *U. maydis* MB215 Δcyp3↑ria1 (**D**, **E** and **F**), *U. maydis* MB215 Δcyp3↑ria1Δfuz7 (**G**, **H** and **I**) and *U. maydis* MB215 Δcyp3↑ria1Δfuz7↑mttA (**J**, **K** and **L**). Like it is in *U. cynodontis,* deletion of *fuz7* stops the filamentous growth behavior in *U. maydis* and further stops cell wall growing in well plates and shaking flasks.

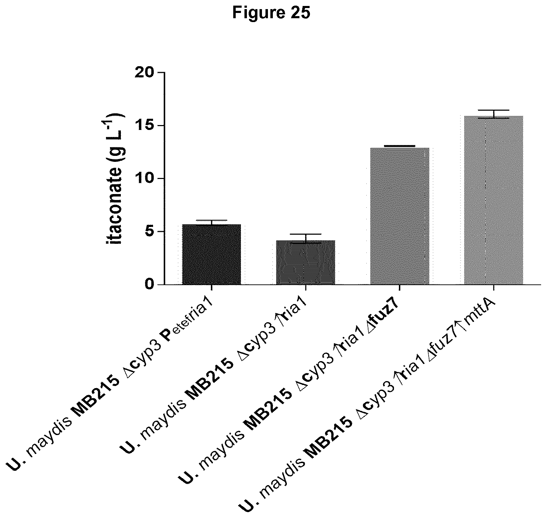

**Fig. 25****: Itaconic acid production after 168 h in *U. maydis* in production medium I.** Itaconic acid production vor various *U. maydis* strains are depicted. Just the deletion of *fuz7* resulted in an increase of itaconic acid production by three-fold.

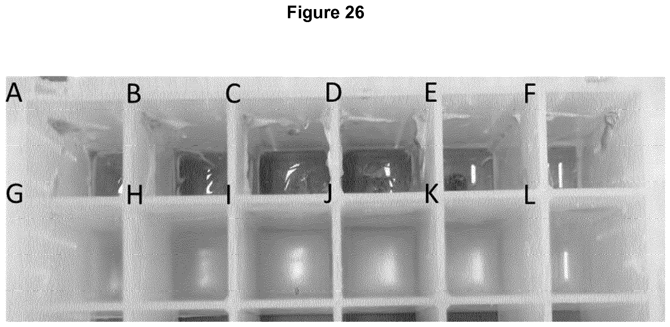

**Fig. 26****: System Duetz® (24 well plates) in production medium I after 48 h.** *U. maydis* Δcyp3P_{etef}ria1 (**A**, **B** and **C**), *U. maydis* MB215 Δcyp3↑ria1 (**D**, **E** and **F**), *U. maydis* MB215 Δcyp3↑ria1Δfuz7 (**G**, **H** and **I**) and *U. maydis* MB215 Δcyp3↑ria1Δfuz7↑mttA (**J**, **K** and **L**). Again, *fuz7* deletion shows no filamentous growth behavior and no cell wall growing.

### DETAILED DESCRIPTION OF THE INVENTION

The present invention provides a method of producing a secreted, non-proteinaceous biomolecule, comprising (a) culturing a fungal host cell belonging to the division of Basidiomycota in culture medium under conditions allowing the production of said biomolecule, wherein the fungal host cell is due to genetic engineering not capable to switch from budding growth to filamentous growth in its vegetative stage during said culturing; and (b) obtaining the biomolecule produced by the fungal host cell from the supernatant of said culture medium.

The fungal host cell being used in the method of the present invention needs to be genetically engineered in order to be not capable of switching from budding growth to filamentous growth in its vegetative stage any more, which has a positive effect on the production of said biomolecule as indicated elsewhere herein.
This morphological transition between budding and filamentous growth in fungi is called dimorphism. The morphological transition is involved in the formation of three cell types in fungi, including budding haploid cells, filamentous dikaryons, and diploid telispores. Haploid cells grow saprophytically and may participate in mating interactions when compatible partners exchange pheromone signals and may form the conjugation tubes (hyphae) required for cell fusion. The product of fusion is an infectious dikaryotic cell type (dicaryon) that penetrates and proliferates within the infected host tissue such as plants. Host plants may react with formation of tumors in which the fungus proliferates and produces diploid teliospores. These spores are surrounded by an echinulated and meanized cell wall and are produced in large numbers during infection. After teliospore germination, meiosis occurs resulting in the generation of haploid cells (sporidiae/basidiospores) by successive budding from probasidia (comprising the four haploid nuclei).

A fungal life cycle of a fungal host cell comprises a vegetative, a dicaryon and a sexual phase/stage (comprising karyogamy).
A "vegetative stage/phase" refers to the asexual reproduction in fungi of the present invention, the stage in which no karyokinetic changes are taking place. Said vegetative stage refers to budding growth of the haploid basidiospores and the formation of conjugation hyphae as indicated above. Said vegetative stage encompasses haploid and/or diploid cells which grow yeast-like on solid PD-medium and/or in liquid YEPS-medium when being cultured for not longer than 12h at 28°C with continous shaking, when the culture is inoculated with about 1 ml of OD₆₀₀=1 (about 10⁹ cells/ml). It is, however, preferred that the vegetative stage encompasses cells which may be diploid, but do not have compatible a-alleles, e.g. *a1* and *a2*. It is, however, also preferred that the vegetative stage encompasses haploid cells only. For avoidance of doubt, the vegetative stage nevertheless encompasses both haploid and diploid cells which may show pseudo-filamentous growth, i.e. cells grow filamentous but no septum/septa is/are formed.
In this context, the term "PD-medium" comprises 24g potato dextrose broth filled up with H₂O to 1L and then being autoclaved. In this context, the term "YEPS-medium" comprises 10g yeast extract, 10g pepton, 10g saccharose filled up with H₂O to 1L.
In this context, the term "pseudo-filamentous growth" refers to a string of budding yeast-like cells that fail to completely divide.

The term "budding growth" refers to asexual reproduction of haploid basidiospores (sporidiae), which are successively separated from the probasidia being generated from a teliospore. In general, budding growth in yeast comprises developing of a new organism from a bud due to cell division at one particular site. The small bulb like projection coming out from yeat cells refers to a bud, being attached as the new organism (yeast cell) grows, finally separating from the parent yeast cell only when it is mature. Thus, in the present invention yeast-like growth may be used interchangeably with the term budding growth in fungi.
In general, those haploid sporidiae being characterized by budding growth in fungi of the present invention may have different alleles of the mating type loci. A successful mating interaction comprising cell-cell-recognition and cell fusion and the additional formation of infectious filamentous dikaryons may only be observed when two cells have different specificities at both the a and *b* loci. The a locus being present in two alleles (*a1* and *a2*) may be important for mutual recognition and cell fusion. The *a1*-locus encodes a phermomone called *mfa1* and a phermomone receptor *pra1* and the a2-locus encodes a phermomone called *mfa2* and and a phermomone receptor *pra2* (Bölker et al. 1992). By responding to the pheromone of the other mating type the formation of long, irregularlly formed conjugation hyphae in the vegetative stage of the fungal life cycle may occur, resulting in the formation of filamentous dikaryons (Spellig et al. 1994). Responsible for the formation of the filamentous dikaryons and the following pathogenic development are the multiallelic *b*-locus comprising at least about 33 alleles. It encodes two divergent genes, *bW* and *bE,* which are separated by a promoter region. Thus, said *b*-locus encodes two homeodomain proteins, bE and bW, which are responsible for maintencance of the infectious dikaryon and completion of the life cycle of said fungus (Gillissen et al. 1992).

In the present invention due to genetic engineering and due to the fact that also haploid and diploid cells which may show pseudo-filamentous growth, are *inter alia* encompassed by the vegetative stage, the fungus of the present invention will never reach the dikaryotic stage of the fungus's lifecycle by mating.

Further, in the present invention the term "filamentous growth" refers to the formation of a tubular, elongated and thread-like structure/shape, which extends by growing at the tip of said structure and may also concomitantly form retraction septa at the distal end of said structure. Thus, for cells growing filamentously the term "growing like a tube" can be used.

In a first step (a) of the method of the present invention a fungal host cell of the present invention needs to be cultured in culture medium under conditions allowing the production of said biomolecule, wherein the fungal host cell is due to genetic engineering not capable to switch from budding growth to filamentous growth in its vegetative stage during said culturing.

In this context, the term "culturing" refers to the *in vitro* maintenance, differentiation, growth, proliferation and/or propagation of the fungal host cell under suitable conditions in a culture medium.

Preferably, a culture medium being used for culturing said fungal host cell under conditions allowing the production of said biomolecule in the method of the present invention comprises glucose (as a C-source), ammonium chloride (NH₄Cl) (as N-source), and a buffer. More preferably, said culture medium comprises at least 20 g L⁻¹ glucose, such as 20, 25, 30, 35, 40, 45, 50, 60, 70, 80, 90, 100, 150, 160, 170, 180, 190, 200, 230, 250, 280, 300 g L⁻¹ glucose, 0.8 g L⁻¹ ammonium chloride as an initial low concentration and MES buffer or calcium carbonate (CaCO₃). Most preferably, said culture medium of the present invention comprises 50 g L⁻¹ glucose, 0.8 g L⁻¹ ammonium chloride (as an initial low concentration) and 30 mM MES buffer.

The term "under conditions allowing the production of said biomolecule" may also comprise besides the specific culture medium as indicated above, a certain pH of said culture medium, whereby the pH has a positive influence on the yield / titer of said biomolecule. Preferably, a pH of at least 3.0, such as 3.0, 3.1, 3.2, 3.3, 3.4, 3.5, 3.6, 3.7, 3.8, 3.9, 4.0, 4.5, 5.0 increases the yield / titer of said biomolecule in a fungal host cell of the present invention. Even more preferably, a pH of 3.6 increases the yield / titer of said biomolecule in a fungal host cell of the present invention being genetically engineered as indicated elsewhere herein in comparison to a fungal host cell not being genetically engineered / prior to engineering as indicated elsewhere herein.

In this context, the term "yield" refers to the amount of a biomolecule as described herein, in particular itaconic acid, respectively, which is, for example, harvested from the engineered host cell, and increased yields can be due to increased amounts of production or secretion of the biomolecule by the host cell. Yield may be presented by g biomolecule/g biomass (measured as dry cell weight or wet cell weight) of a host cell. The term "titer" when used herein refers similarly to the amount of produced biomolecule, in particular itaconic acid, presented as g biomolecule/L culture supernatant. An increase in yield or in titer can be determined when the yield / titer obtained from an engineered host cell is compared to the yield / titer obtained from a host cell prior to engineering, i.e., from a non-engineered host cell. The yield / titer of the biomolecule being produced from the fungal host cell being genetically engineered as indicated elsewhere herein in the method of the present invention may be increased by at least 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 200, 250, 300, 350, 400, or even 500% in comparison to a fungal host cell prior to engineering. Thus, the present invention comprises a method of increasing the yield / titer of a secreted, non-proteinaceous biomolecule by at least 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 200, 250, 300, 350, 400, or even 500% by a fungal host cell of the present invention being genetically engineered in comparison to a host cell prior to engineering.

"Prior to engineering" or "prior to manipulation" when used in the context of fungal host cells of the present invention means that such host cells are not engineered by knocking-out one or more polynucleotide sequence(s) encoding a polypeptide being involved in a signal transduction pathway which regulates the switching from budding growth to filamentous growth of said fungus.

Further, also contemplated by the present invention is a method of producing a secreted, non-proteinaceous biomolecule of the present invention of at least 25 g L⁻¹ comprising step (a) and (b) as indicated above.

Additionally, culturing may be performed by incubating the culture (e.g., shake flask culture) or inoculating the culture (e.g., System Duetz® cultures) under certain conditions, preferably at a temperature of 30°C, at 200 or 300 rpm (centrifugation) and a relative air humidity of 75%.

After incubating / inoculating at certain conditions as indicated above, a clear liquid above the solid residue after centrifugation is being visible, which refers to the supernatant of the culture medium, where the biomolecule is being obtained from after the fungal host cell has produced / generated and then secreted said biomolecule into the culture medium. Said supernatant may also be achieved by precipitation, crystallization or just settling. The liquid (supernatant) is normally free of precipitate and has a lower density. The process leading to the supernatant formation is used in separating the several components making up a complex mixture.

After culturing the fungal host cell of the present invention in culture medium under conditions allowing the production of said biomolecule as indicated in step (a) of the method as indicated above, the biomolecule may be recovered as indicated in step (b) of the method of the present invention.

However, a convenient way to harvest a biomolecule of interest is its isolation from the supernatant of the culture medium. The biomolecule may be isolated (obtained) from the supernatant of the culture medium in which the fungal host cell is cultured by lysis or any other measure applied to break the cell membrane and/or cell wall of a fungal host cell and subsequently be purified. Also it is possible, that the host cells are removed from the medium before isolation of said biomolecule takes place. Methods to isolate molecules such as itaconic acid are known to the person skilled in the art. Examples of such methods for isolating (obtaining) said biomolecule from the supernatant of the culture medium is achieved by precipitation, crystallization, adsorption or extraction, preferably by precipitation. In the case of isolating the biomolecule from the culture medium, the biomolecule of interest must be secreted by the host. In the present invention said biomolecules were isolated from the medium in which the host cell is cultured *via* high pressure liquid chromatography (HPLC).

In general, secretion of a biomolecule may be achieved by the use of a transporter, such as the Mtt1 transporter encoded by the *mtt1* gene with locus tag UMAG_05079 in *Ustilago maydis* (Geiser et al. 2016 Microbial Biotechnology 9(1), 116-126).

Thus, the term "produced by the fungal host cell" means that the fungal host cell of the present invention generates said biomolecule as indicated elsewhere herein and then secretes the biomolecule into the supernatant of the culture medium, where the biomolecule is being able to be isolated / obtained from.

The findings of the present inventors are rather surprising, since the specific genetically engineering of the present invention as indicated elsewhere herein was to the best of one's knowledge up to the present invention not brought in connection with the method of the present invention of producing a secreted, non-proteinaceous biomolecule of interest in a fungal host cell.

The biomolecule being produced by the method as described above refers to a "small molecule that is present in an organism, essential to some typically biological process and denotes an organic molecule comprising at least two carbon atoms, but preferably not more than 8, 12, 15, 17, 20 or 25, 30, 35, 40, 50, 60 rotatable carbon bonds (excluding carbon-hydrogen bonds), more preferably not more than 8, 12, 15, 17 or 20 rotatable carbon bonds, even more preferably not more than 8, 12, or 15 rotatable carbon bonds, having a molecular weight in the range between 100 and 2000 Dalton (DA), preferably between 100 and 1000 Dalton, and optionally including one or two metal atoms.

In this contect, the biomolecule may be secreted and non-proteinaceous. The term "non-proteinaceous" does not refer of, relate to, contain, resemble or does not refer to a protein. Thus, said biomolecule being produced by the method of the present invention does not refer to a protein.

The biomolecule being produced by the method as described above may be an organic acid and/or salts thereof, and/or an ester thereof. Preferably, the organic acid is itaconic acid, malic acid, hydroxyparaconic acid, citramalic acid, aconitic acid or itatararic acid. Most preferably, the organic acid is itaconic acid, malic acid, hydroxyparaconic acid, more preferably the organic acid is itaconic acid.

Itaconic acid (or 2-methylidenebutanedioic acid) as a biomolecule of the present invention being produced in the method of the present invention having the formula C₅H₆O₄ has the following structure: 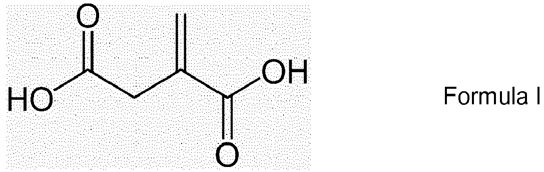 Itaconic acid (ITA) may have a molecular weight of about 130.10 g/mol and may have not more than 5 rotatable carbon bonds. Itaconic acid is an unsaturated organic dicarboxylic acid which has a high biotechnological potential. It is a top-value biobased chemical building block used for the production of polymers, pharmaceuticals and fuels. The major application of this acid is in the production of synthetic fibers, but it is also used in the pharmaceutical field. Itaconic acid is currently produced *via* fermentation of the filamentous ascomycete *Aspergillus terreus.* Biosynthesis of itaconic acid in *A. terreus* occurs by decarboxylation of cis-aconitate, a common intermediate of the citric acid cycle, by a cis-aconitate decarboxylase (CAD). Due to the heavily process-controlled fermentation of *A. terreus,* the production of itaconic acid incurs high costs. *Ustilaginaceae* are therefore further promising candidates for itaconic acid production since they have numerous advantages over *A. terreus.* In *U. maydis,* two enzymes are important for itaconic acid production: Aconitate-delta-isomerase (ADI) being involved in ITA formation by converting the TCA cycle intermediate cis-aconitic acid into trans-aconitic acid and trans-aconitate decarboxylase (TAD) being able to catalyze the second step of ITA formation by converting trans-aconitic acid into itaconic acid.

Malic acid (or 2-hydroxybutanedioic acid) as a biomolecule of the present invention being produced in the method of the present invention having the formula of C₄H₆O₅ has the following structure: 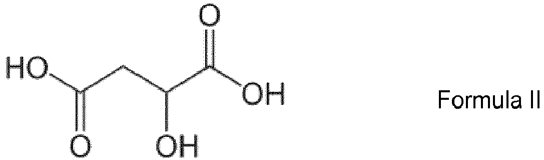 Malic acid may have a molecular weight of about 134.09 g/mol and may have not more than 6 rotatable carbon bonds. Malic acid may mainly be used in food-, beer- and sweet industry, but also in the pharmaceutical sector.

Hydroxyparaconic acid as a biomolecule being produced in the method of the present invention having the formula of C₅H₆O₅ has the following structure: 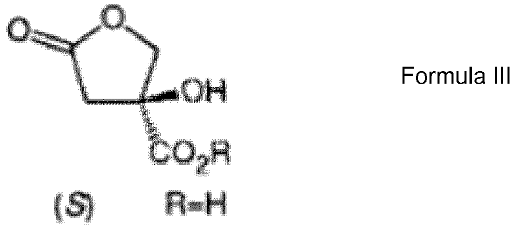 Hydroxyparaconic acid may have a molecular weight of about 146.098 g/mol and may have not more than 3 rotatable carbon bonds. Hydroxyparaconat, a chiral lactone, may be used as a precursor for the production of medical applicable pheromons (Mori and Fukamatsu 1992).

Citramalic acid as a biomolecule being produced in the method of the present invention having the formula HO₂CCH₂C(CH₃)(OH)CO₂H has the following structure:

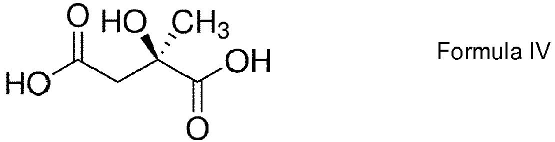

Citramalic acid acid may have a molecular weight of about 148.11 g/mol and may have not more than 7 rotatable carbon bonds.

Aconitic acid as a biomolecule being produced in the method of the present invention having the formula of C₆H₆O₆ has the following structure in *cis* and *trans:*

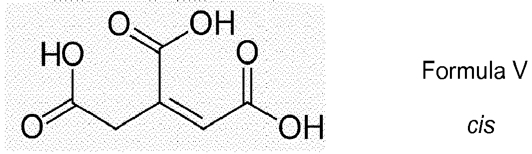

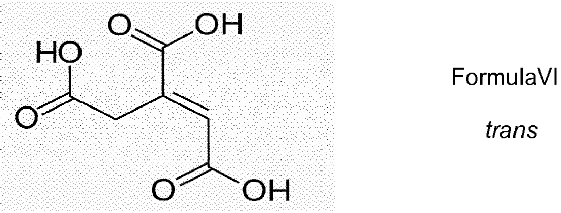

Aconitic acid acid may have a molecular weight of about 174.11 g/mol and may have not more than 7 rotatable carbon bonds.

Itatararic acid (or 2-Hydroxy-2-(hydroxymethyl)butanedioic acid) as a biomolecule being produced in the method of the present invention having the formula of C₅H₈O₆ has the following structure:

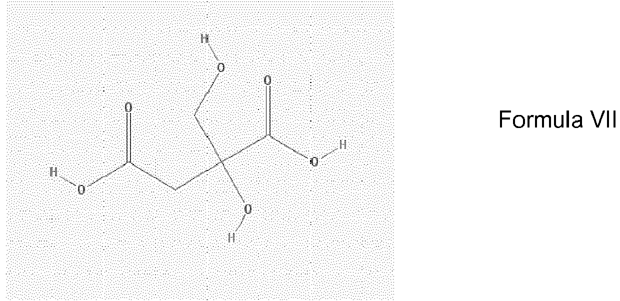

Itatararic acid may have a molecular weight of about 164.113 g/mol and may have not more than 8 rotatable carbon bonds.

The biomolecule being produced by the method described elsewhere herein may be a polyol and/or salts thereof, and/or an ester thereof. Preferably, a polyol is erythritol or mannitol, most preferably a polyol is erythritol.

Erythritol as a biomolecule being produced in the method of the present invention having the formula of C₄H₁₀O₄ has the following structure: 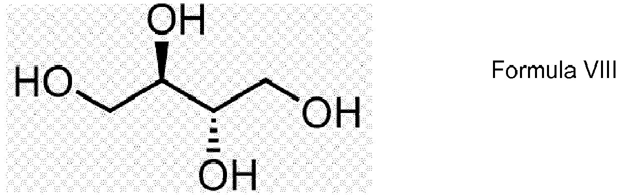 Erythritol may have a molecular weight of about 122.12 g/mol and may have not more than 7 rotatable carbon bonds.

Mannitol as a biomolecule being produced in the method of the present invention having the formula of C₆H₁₄O₆ has the following structure:

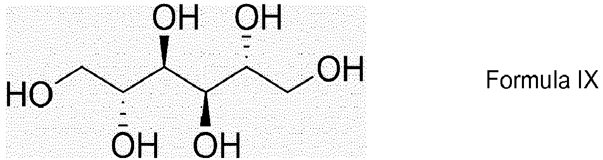

Mannitol may have a molecular weight of about 182.172 g/mol and may have not more than 11 rotatable carbon bonds.
Erythritol and mannitol may have a wide application in the fodd industry and nutraceutical industry (Moon et al. 2010; Wisselink et al. 2002).

The biomolecule being produced in the method of the present invention described elsewhere herein may be a glycolipid, and/or salts thereof, and/or esters thereof. Preferably, a glycolipid is ustilagic acid or mannosylerythritol lipid. A glycolipid may also be a trisaccharide lipid.

Ustilagic acid as a biomolecule being produced in the method of the present invention having the formula of C₃₆H₆₄O₁₈ has the following structure: 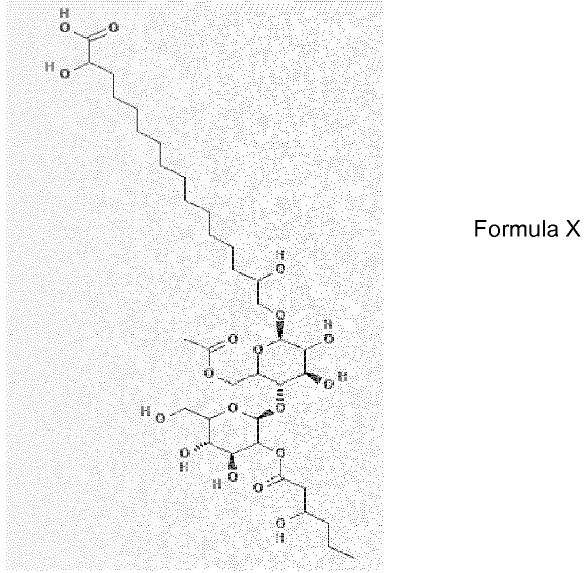 Ustilagic acid may have a molecular weight of about 784.89 g/mol and may have not more than 40 rotatable carbon bonds.

Mannosylerythritol lipid (MEL) as a biomolecule being produced in the method of the present invention has the following structure: 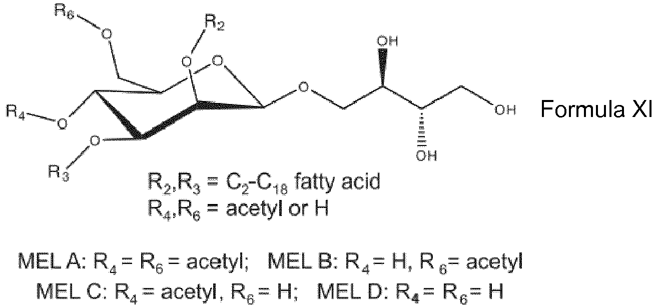 Mannosylerythritol lipids (MELs) consist of a mannosylerythritol disaccharide which is acylated with short-chain (C₂ to C₈) and medium-chain (C₁₀ to C₁₈) fatty acids at the mannosyl moiety. Depending on the number of acetyl groups, mannosylerythritol lipids can be differentiated into MEL A (fully acetylated), MEL B and MEL C (monoacetylated at R-6 and R-4, respectively), and the fully deacetylated MEL D (Hewald et al. 2006). Said MELs may have not more than 57 rotatable carbon bonds.
Ustilagic acid and mannosylerythritol lipid may be used in the pharmaceutical sector as well as in food industry (Feldbrugge, Kellner and Schpper 2013).

The trisaccharide lipid as a biomolecule being produced in the method of the present invention refers to a glycolipid component comprising at least one antimicrobial glycolipid having the following structure: 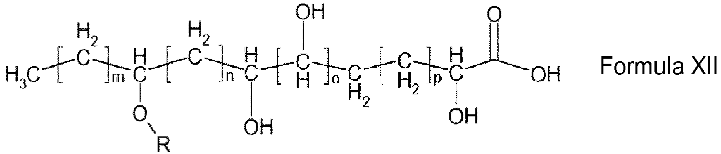 wherein
m is 3 to 5,
n is 2 to 5,
o is 0 or 1; and
p is 3 to 17,
with the proviso that the sum m + n + o + p is not less than 14; and
R is a carbohydrate moiety bound via one of its carbon atoms to the binding oxygen, and/or a physiologically, especially pharmaceutically or cosmetically, acceptable salt thereof, or an ester thereof as it is described in WO2012/167920.

Preferably, said trisaccharide lipid refers to the formula XII, wherein m is 3 to 5, n is 2 to 5, o is 0 or 1, p is 5 to 15; more preferably 11 or 13 and R is a moiety of the subformula XIII 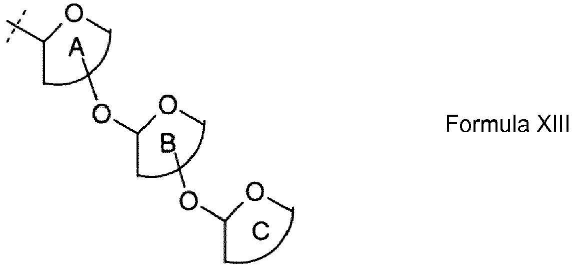 wherein the rings A, B and C are monosaccharide moieties each independently from the others with 5 or 6 ring members, wherein one or more of the hydroxyl groups may be acylated.

The term "host cell" or "fungal host cell" being used in the method of the present invention to produce said secreted, non-proteinaceous biomolecule as indicated above refers to a (fungal) cell which is genetically engineered, thus not being capable to switch from budding growth to filamentous growth in its vegetative stage, thus producing said biomolecule during culture of said host cell. Such host cell is applied in the method of the present invention. Examples of fungal host cells include, but are not limited to, cells belonging to the division of Basidiomycota.

Basidiomycota are typically filamentous fungi composed of hyphae. Basidiomycota is one of two large divisions that together with the Ascomycota constitute the subkingdom Dikarya (also being referred to "higher fungi") within the kingdom fungi. The obvious difference between Basidiomycota and Ascomycota is the way that they produce their spores following meiosis. The Basidiomycota produce their meiospores, called basidiospores, externally on basidia, while the Ascomycota produce theirs, called ascospores, in asci. Basidiospores are always borne externally on the basidium and ascospores are always borne internally in the ascus.

Preferably, the fungal host cell being used to produce the biomolecule in the method of the present invention belonging to the division of Basidiomycota is from the family of Ustilaginaceae, the family of Dacrymycetaceae, or from the family of Moniliellaceae. Most preferably, the fungal host cell being used to produce the biomolecule of the present invention is from the family of Ustilaginaceae.

The method of the present invention comprises a fungal host cell from the family of Ustilaginaceae, preferably a species from the genus *Ustilago, Pseudozyma, or Sporisorium,* more preferably from the genus *Ustilago.*

The fungal host cell from the genus *Ustilago* being used to produce the biomolecule in the method of the present invention may be *Ustilago maydis, Ustilago cynodontis, Ustilago trichophora, Ustilago vetiveriae, Ustilago xerochloae, Ustilago hordei, Ustilago bromivora, Ustilago esculenta.* Preferably, the fungal host cell from the genus *Ustilago* being used to produce the biomolecule in the method of the present invention is *Ustilago maydis, Ustilago cynodontis, Ustilago trichophora, Ustilago vetiveriae, or Ustilago xerochloae,* more preferably, *Ustilago maydis or Ustilago cynodontis.* Especially preferred are *Ustilago maydis* strain MB215. Especially preferred are *Ustilago cynodontis* strain NBRC 9727.
In another embodiment, the fungal host cell from the genus *Pseudozyma* being used to produce the biomolecule in the method of the present invention may be *Pseudozyma hubeiensis, Pseudozyma antarctica, Pseudozyma tsukubaensis, Pseudozyma (Moesziomyces) aphidis, Pseudozyma (Kalmanozyma) brasiliensis, Pseudozyma (Anthracocystis) flocculosa.*
In another embodiment, the fungal host cell from the genus *Sporisorium* being used to produce the biomolecule in the method of the present invention may be *Sporisorium scitamineum, Sporisorium iseilematis-ciliati.*

In another embodiment, the method of the present invention comprises a fungal host cell from the family of Dacrymycetaceae, preferably a species from the genus *Dacryopinax, Dacrymyces, Ditiola, Guepiniopsis,* or *Femsjonia.*
The fungal host cell from the genus *Dacryopinax* being used to produce the biomolecule in the method of the present invention may be *Dacryopinax aurantiaca, Dacryopinax crenata, Dacryopinax dennisii, Dacryopinax elegans, Dacryopinax felloi, Dacryopinax fissus, Dacryopinax foliacea, Dacryopinax formosus, Dacryopinax imazekiana, Dacryopinax indacocheae, Dacryopinax lowyi, Dacryopinax macrospora, Dacryopinax martinii, Dacryopinax maxidorii, Dacryopinax parmastoensis, Dacryopinax petaliformis, Dacryopinax spathularia, Dacryopinax primogenitus Dacryopinax sphenocarpa, Dacryopinax taibaishanensis, Dacryopinax xizangensis,* or *Dacryopinax yungensis.* Preferably, the fungal host cell from the genus *Dacryopinax* being used to produce the biomolecule in the method of the present invention is *Dacryopinax spathularia or Dacryopinax primogenitus,* more preferably *Dacryopinax spathularia.* Especially preferred are *Dacryopinax spathularia* strain MUCL 53181, *Dacryopinax spathularia* strain MUCL 53182.
The fungal host cell from the genus *Dacrymyces* being used to produce the biomolecule in the method of the present invention may be *Dacrymyces stillatus, Dacrymyces chrysocomus, Dacrymyces ancyleus, Dacrymyces aureosporus, Dacrymyces australis, Dacrymyces capitatus, Dacrymyces chrysospermus, Dacrymyces cupularis, Dacrymyces dictyosporus, Dacrymyces enatus, Dacrymyces flabelliformis, Dacrymyces intermedius, Dacrymyces lacrymalis, Dacrymyces macnabbii, Dacrymyces minor, Dacrymyces novae-zelandiae, Dacrymyces ovisporus, Dacrymyces palmatus, Dacrymyces paraphysatus, Dacrymyces pinacearum, Dacrymyces punctiformis, Dacrymyces subarcticus, Dacrymyces tortus, or Dacrymyces variisporus.* Preferably, the fungal host cell from the genus *Dacrymyces* being used to produce the biomolecule in the method of the present invention is *Dacrymyces stillatus* or *Dacrymyces chrysocomus.* Especially preferred is *Dacrymyces chrysocomus* strain CBS280.84 and XX.
The fungal host cell from the genus *Ditiola* being used to produce the biomolecule in the method of the present invention may be *Ditiola radicata, Ditiola nuda, Ditiola abieticola, Ditiola brasiliensis; Ditiola coccinea, Ditiola obliqua, Ditiola orientalis, or Ditiola peziziformis.* Especially preferred are *Ditiola radicata* strain MUCL 53180 and *Ditiola nuda* strain MUCL 53179.
The fungal host cell from the genus *Guepiniopsis* being used to produce the biomolecule in the method of the present invention may be *Guepiniopsis buccina, Guepiniopsis alpina, Guepiniopsis estonica, Guepiniopsis fulva, Guepiniopsis oresbia, Guepiniopsis ovispora, Guepiniopsis pedunculata, or Guepiniopsis suecica.* Preferably, the fungal host cell from the genus *Guepiniopsis* being used to produce the biomolecule in the method of the present invention is *Guepiniopsis buccina.*
The fungal host cell from the genus *Femsjonia* being used to produce the biomolecule in the method of the present invention may be *Femsjonia luteo-alba (*= *Ditiola*/*Femsjonia pezizaeformis).* Especially preferred is *Femsjonia luteo-alba* strain MUCL 53500.

In an especially preferred embodiment the method of the present invention comprises a fungal host cell, wherein the fungal host cell is *Ustilago maydis, Ustilago cynodontis or Dacryopinax spathularia,* even more preferably *Ustilago maydis* or *Ustilago cynodontis,* most preferably *Ustilago cynodontis.*

*Ustilago cynodontis* strain NBRC 9727 and *Ustilago maydis* strain MB215 were found to be among the best strains so far for producing the biomolecule and/or salts and/or esters thereof, whereby the strains were genetically engineered, thus being able to switch from filamentous growth to yeast-like growth.

By morphologic engineering, the fungus of the present invention is not only not capable from switching from budding growth to filamentous growth, but also is able to stay in its yeast-like growth under stress conditions. The genetically modified strain having a (single) deletion(s) of a polynucleotide sequence(s) encoding a polypeptide being involved in a signal transduction pathway regulating the switching from budding growth to filamentous growth of said fungus is not capable to change its morphology any more in comparison to other fungi. This may result in a considerable influence on the production of said biomolecule.

Thus, the present invention may comprise a method of producing said biomolecule of the present invention, wherein the fungal host cell of the present invention and as indicated elsewhere herein, which is no longer capable of switching from budding growth to filamentous growth in its vegetative stage during said culturing, is genetically engineered by knocking-out one or more polynucleotide sequence(s) encoding a polypeptide being involved in a signal transduction pathway which regulates the switching from budding growth to filamentous growth of said fungus.

In general, after binding of pheromones (such as *mfa1* or *mfa2*) to the pheromone receptor (*pra1* or *pra2*) of the other mating loci in a fungus as mentioned above, whereby said pheromone response is responsible for cell-cell recognition and cell fusion, activation of the pheromone response transcription factor Prf1, which regulates the transcription of genes located at the a and *b* loci, occurs. More specific, through regulation by a mitogen-activated protein (MAP) kinase cascade, said activation of Prf1 occurs. Many components of the MAP kinase cascade have been identified including the pheromone-responsive MAP kinase pathway adaptor Ubc2, the MAP kinase kinase kinase Ubc4, the MAP kinase kinase Fuz7, the MAP kinase Ubc3, and the pheromone response transcription factor Prf1. Another component of the MAP kinase cascade is the Ras protein belonging to a family of small GTPases which are able to affect the MAP kinase pathway. There are two Ras proteins, Ras1 and Ras2. Activated ras2 allele may promote filamentation in a Fuz7/-Ubc3 dependent manner, being upstream of Fu7 in the MAPK cascade.

Ras protein also affects the cAMP pathway, respectively. Said signal transduction pathway is also responsible for regulation of cell fusion and pathogenic development by formation of filamentous dikaryons in a fungal host cell. The signal being transferred by said cascade is not known. It might be the pheromone signal resulting in the activation of Prf1 in the MAP kinase cascade. The cAMP pathway consists of Gpa3, the α-subunit of the heterotrimeric G-protein, adenylatcyclase Uac1 and the regulatory and the catalytic subunit of PKA, namely Ubc1 and Adr1. Activation of the herterotrimeric G-protein results in the dissociation of the α-subunit Gpa3 from the βγ-subunits and thus in the activation of Uac1. Thereby, an increase of intracellular cAMP-concentration occurs, leading to the dissociation of the Ubc1/Adr1 complex, whereby Adr1 may phosphorylate target proteins.

The inventors found that by genetically engineering said fungal host cell by knocking-out one or more polynucleotide sequence(s) encoding a polypeptide being involved in a signal transduction pathway, in particular being involved in the MAP kinase pathway or in the cAMP pathway, said biomolecule of the present invention may be produced.

Thus, the present invention comprises a method of producing said biomolecule of the present invention, wherein the fungal host cell which is no longer capable of switching from budding growth to filamentous growth in its vegetative stage during said culturing is genetically engineered by knocking-out one, two, three, four, five, six or seven polynucleotide sequence(s) encoding a polypeptide being involved in a signal transduction pathway, in particular being involved in the MAP kinase pathway or in the cAMP pathway, which regulate the switching from budding growth to filamentous growth of said fungus.

In a preferred embodiment, knocking-out only one polynucleotide sequence encoding a polypeptide being involved in a signal transduction pathway, in particular being involved in the MAP kinase pathway or in the cAMP pathway is sufficient. In an even more preferred embodiment knocking-out only one polynucleotide sequence encoding a polypeptide being involved in the MAP kinase pathway is sufficient for a fungal host cell being no longer capable of switching from budding growth to filamentous growth in its vegetative stage during said culturing in the method of the present invention.

As used herein, "engineered" host cells are host cells which have been manipulated using genetic engineering, i.e. by human intervention. When a fungal host cell is "engineered by knocking-out" a given polynucleotide sequence, the host cell is manipulated such that there is no expression of the given protein compared to the host cell under the same condition prior to manipulation (or "prior to engineering").

In this context, by knocking-out one or more polynucleotide sequence(s) encoding a polypeptide being involved in said signal transduction pathway which regulates the switching from budding growth to filamentous growth of said fungus, deleting the coding sequence of said polypeptide or fragments thereof being involved in a signal transduction pathway which regulates the switching from budding growth to filamentous growth of said fungus may be included. Further, deleting a regulatory sequence required for expression of the polypeptide (e.g. the promoter) may also be comprised for knocking-out one or more polynucleotide sequence(s) encoding a polypeptide being involved in said signal transduction pathway. A gene can be knocked out by deleting the entire or partial coding sequence. Methods of making gene knockouts are known in the art, e.g., see Kuhn and Wurst (Eds.) Gene Knockout Protocols (Methods in Molecular Biology) Humana Press (March 27, 2009). A gene can also be knocked out by removing part or all of the gene sequence. Alternatively, a gene can be knocked-out or inactivated by the insertion of a nucleotide sequence, such as a resistance gene. Alternatively, a gene can be knocked-out or inactivated by inactivating its promoter.
Preferably, deletion (knock-out) of a polynucleotide sequence encoding a polypeptide being involved in a signal transduction pathway which regulates the switching from budding growth to filamentous growth is achieved by FLP/FRT mediated recombination system from Khrunyk et al. 2010 and as also indicated in **Example 15.**

The term "promoter" as used herein refers to a region of DNA upstream from the translational start codon and which is involved in recognition and binding of RNA polymerase and other proteins to initiate transcription. Promoters contain specific DNA sequences called response elements that make sure a secure initial binding site for RNA polymerase as well as for proteins called transcription factors recruiting RNA polymerase is provided.

A polynucleotide refers to nucleotides, either ribonucleotides or deoxyribonucleotides or a combination of both, in a polymeric unbranched form of any length. Preferably, a polynucleotide refers to deoxyribonucleotides in a polymeric unbranched form of any length. Here, nucleotides consist of a pentose sugar (deoxyribose), a nitrogenous base (adenine, guanine, cytosine or thymine) and a phosphate group. The terms "polynucleotide", "polynucleotide sequence" and "nucleotide sequence" can thus be interchangeably used.

The term "polypeptide" refers to a polymer of amino acid residues and is not limited to a certain minimum length of the product. However, peptidomimetics of such proteins/ polypeptides, wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. As mentioned, the terms polypeptide and protein are often used interchangeably herein. The term polypeptide also refers to, and does not exclude, modifications of the polypeptide. Modifications include glycosylation, acetylation, acylation, phosphorylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cysteine, formation of pyroglutamate, formulation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination.

In a preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 1 (fuz7 of *U. maydis*), said polynucleotide sequence encoding a polypeptide or fragment thereof having pheromone-responsive MAP kinase kinase activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% or even 100% sequence identity to the amino acid sequence of SEQ ID NO. 1 (fuz7 of *U. maydis*), said polynucleotide sequence encoding a polypeptide or fragment thereof having pheromone-responsive MAP kinase kinase activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 1 (fuz7 of *U. maydis*), said polynucleotide sequence encoding a polypeptide or fragment thereof having pheromone-responsive MAP kinase kinase activity.

In another preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 2 (ras2 of *U. maydis*), said polynucleotide sequence encoding a polypeptide or fragment thereof having pheromone-responsive MAP kinase pathway-interacting activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 2 (ras2 of *U. maydis*), said polynucleotide sequence encoding a polypeptide or fragment thereof having pheromone-responsive MAP kinase pathway-interacting activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 2 (ras2 of *U. maydis*), said polynucleotide sequence encoding a polypeptide or fragment thereof having pheromone-responsive MAP kinase pathway-interacting activity.

In another preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 3 (ubc3 of *U. maydis*), said polynucleotide sequence encoding a polypeptide or fragment thereof having pheromone-responsive MAP kinase activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 3 (ubc3 of *U. maydis*), said polynucleotide sequence encoding a polypeptide or fragment thereof having pheromone-responsive MAP kinase activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 3 (ubc3 of *U. maydis*), said polynucleotide sequence encoding a polypeptide or fragment thereof having pheromone-responsive MAP kinase activity.

In another preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 4 (ubc1 of *U. maydis*), said polynucleotide sequence encoding a polypeptide or fragment thereof having cAMP-dependent protein kinase regulator activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 4 (ubc1 of *U. maydis*), said polynucleotide sequence encoding a polypeptide or fragment thereof having cAMP-dependent protein kinase regulator activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 4 (ubc1 of *U. maydis*), said polynucleotide sequence encoding a polypeptide or fragment thereof having cAMP-dependent protein kinase regulator activity.

In another preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 5 (ubc2 of *U. maydis*), said polynucleotide sequence encoding a polypeptide or fragment thereof having pheromone-responsive MAP kinase pathway-interacting activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 5 (ubc2 of *U. maydis*), said polynucleotide sequence encoding a polypeptide or fragment thereof having pheromone-responsive MAP kinase pathway-interacting activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 5 (ubc2 of *U. maydis*), said polynucleotide sequence encoding a polypeptide or fragment thereof having pheromone-responsive MAP kinase pathway-interacting activity.

In another preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 6 (ubc4 of *U. maydis*), said polynucleotide sequence encoding a polypeptide or fragment thereof having pheromone-responsive MAPKK kinase activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 6 (ubc4 of *U. maydis*), said polynucleotide sequence encoding a polypeptide or fragment thereof having pheromone-responsive MAPKK kinase activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 6 (ubc4 of *U. maydis*), said polynucleotide sequence encoding a polypeptide or fragment thereof having pheromone-responsive MAPKK kinase activity.

In another preferred embodiment the present invention comprises the method for producing said biomolecule, wherein the one or more polynucleotide sequence(s) encoding a polypeptide is a polynucleotide sequence encoding a polypeptide having an amino acid sequence having at least 25% sequence identity to the amino acid sequence of SEQ ID NO. 7 (prf1 of *U. maydis*), said polynucleotide sequence encoding a polypeptide or fragment thereof having pheromone-responsive transcription factor activity. Even more preferred as the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 7 (prf1 of *U. maydis*), said polynucleotide sequence encoding a polypeptide or fragment thereof having pheromone-responsive transcription factor activity. Even more preferred as the one or more polynucleotide sequence(s) encoding a polypeptide is a polynucleotide sequence encoding a polypeptide having an amino acid sequence having 25%, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 7 (prf1 of *U. maydis*), said polynucleotide sequence encoding a polypeptide or fragment thereof having pheromone-responsive transcription factor activity.

A polypeptide has "identity", or is "identical" to a second polypeptide if the polynucleotide sequence that encodes the polypeptide has a similar or identical sequence to the polynucleotide sequence that encodes the second polypeptide. Also, a polypeptide has identity to a second polypeptide if the two proteins have "similar" amino acid sequences. Thus, the term "identical polypeptide/proteins" is defined to mean that the two polypeptides/proteins have similar or identical amino acid sequences. In a preferred embodiment, an identical protein is one that exhibits at least 25%, 30%, 40%, 50%, 60%, 70%, 80%, 85%, 90% or more such as 95%, 97%, 98% or 99% sequence identity to the wild type protein. In another preferred embodiment, an identical protein is one that exhibits at least 60% sequence identity to the wild type protein, more preferred is at least 70% sequence identity. Even more preferred are identical proteins that exhibit at least 80%, 85% or 90% sequence identity to the wild type protein. In a yet more preferred embodiment, an identical protein exhibits at least 95%, 96%, 97%, 98% or 99% sequence identity. As used herein, identity between two regions of amino acid sequence (especially with respect to predicted structural similarities) is interpreted as implying similarity in function.

Sequence identity for polypeptides, which is also referred to as percent sequence identity, is typically measured using sequence analysis software. See, e.g., the Sequence Analysis Software Package of the Genetics Computer Group (GCG), University of Wisconsin Biotechnology Center, 910 University Avenue, Madison, Wisconsin 53705. Protein analysis software matches similar sequences using measure of identity assigned to various substitutions, deletions and other modifications, including conservative amino acid substitutions. For instance, GCG contains programs such as "Gap" and "Bestfit" which can be used with default parameters to determine sequence identity or sequence identity between closely related polypeptides, such as identical polypeptides from different species of organisms or between a wild type protein and a mutein thereof. See, e. g., GCG Version 6.1. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The same is true for (poly)nucleotide sequences disclosed herein.

By "fragment" in reference to a polypeptide as described herein is meant any amino acid sequence present in a polypeptide as described herein, as long as it is shorter than the full length sequence and as long as it is capable of performing the function of a protein involved in the signal transduction pathway which regulates the switching from budding growth to filamentous growth of said fungus as described herein. Preferred fragments have at least 20, 40, 60, 80, 100, 120, 150, 200, 250, 300, 350, 400, 450, 500, 550, or 600 amino acids. Such preferred fragments have the function of a protein involved in the signal transduction pathway which regulates the switching from budding growth to filamentous growth of said fungus as described herein. Thus, for example a fragment of fuz7 protein has the same function as the fuz7 protein having the amino acid sequence of SEQ ID NO. 1.

Preferably, the one or more polynucleotide sequence(s) encoding a polypeptide is a polynucleotide sequence encoding a polypeptide having an amino acid sequence as set out in any one of SEQ ID NOs. 1, 2, 3, 4, 5, 6, 7, 8 (fuz7 of *U. cynodontis*), 9 (ras2 of *U. cynodontis*), 10 (ubc3 of *U. cynodontis*), 11 (ubc1 of *U. cynodontis),* 12 (ubc2 of *U. cynodontis*), 13 (ubc4 of *U. cynodontis*), or 14 (prf1 of *U. cynodontis*).

Said polynucleotide sequence encoding a polypeptide having an amino acid sequence of SEQ ID NO. 1 refers to SEQ ID NO. 15 (DNA sequence of fuz7 *U. maydis*). Said polynucleotide sequence encoding a polypeptide having an amino acid sequence of SEQ ID NO. 2 refers to SEQ ID NO. 16 (DNA sequence of ras2 *U. maydis*). Said polynucleotide sequence encoding a polypeptide having an amino acid sequence of SEQ ID NO. 3 refers to SEQ ID NO. 17 (DNA sequence of ubc3 *U. maydis*). Said polynucleotide sequence encoding a polypeptide having an amino acid sequence of SEQ ID NO. 4 refers to SEQ ID NO. 18 (DNA sequence of ubc1 *U. maydis*). Said polynucleotide sequence encoding a polypeptide having an amino acid sequence of SEQ ID NO. 5 refers to SEQ ID NO. 19 (DNA sequence of ubc2 *U*. *maydis*). Said polynucleotide sequence encoding a polypeptide having an amino acid sequence of SEQ ID NO. 6 refers to SEQ ID NO. 20 (DNA sequence of ubc4 *U. maydis*). Said polynucleotide sequence encoding a polypeptide having an amino acid sequence of SEQ ID NO. 7 refers to SEQ ID NO. 21 (DNA sequence of Prf1 *U. maydis*).
Said polynucleotide sequence encoding a polypeptide having an amino acid sequence of SEQ ID NO. 8 refers to SEQ ID NO. 22 (DNA sequence of fuz7 *U. cynodontis*). Said polynucleotide sequence encoding a polypeptide having an amino acid sequence of SEQ ID NO. 9 refers to SEQ ID NO. 23 (DNA sequence of ras2 *U. cynodontis*). Said polynucleotide sequence encoding a polypeptide having an amino acid sequence of SEQ ID NO. 10 refers to SEQ ID NO. 24 (DNA sequence of ubc3 *U. cynodontis*). Said polynucleotide sequence encoding a polypeptide having an amino acid sequence of SEQ ID NO. 11 refers to SEQ ID NO. 25 (DNA sequence of ubc1 *U*. *cynodontis*). Said polynucleotide sequence encoding a polypeptide having an amino acid sequence of SEQ ID NO. 12 refers to SEQ ID NO. 26 (DNA sequence of ubc2 *U. cynodontis*). Said polynucleotide sequence encoding a polypeptide having an amino acid sequence of SEQ ID NO. 13 refers to SEQ ID NO. 27 (DNA sequence of ubc4 *U. cynodontis*). Said polynucleotide sequence encoding a polypeptide having an amino acid sequence of SEQ ID NO. 14 refers to SEQ ID NO. 28 (DNA sequence of prf1 *U. cynodontis*).

The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the sequence of SEQ ID NO. 15, preferably having at least 60%, 70%, 80%, 90%, 95%, or even 98% sequence identity to the sequence of SEQ ID NO. 15. The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the sequence of SEQ ID NO. 16, preferably having at least 60%, 70%, 80%, 90%, 95%, or even 98% % sequence identity to the sequence of SEQ ID NO. 16. The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the sequence of SEQ ID NO. 17, preferably having at least 60%, 70%, 80%, 90%, 95%, or even 98% sequence identity to the sequence of SEQ ID NO. 17. The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the sequence of SEQ ID NO. 18, preferably having at least 60%, 70%, 80%, 90%, 95%, or even 98% sequence identity to the sequence of SEQ ID NO. 18. The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the sequence of SEQ ID NO. 19, preferably having at least 60%, 70%, 80%, 90%, 95%, or even 98% sequence identity to the sequence of SEQ ID NO. 19. The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the sequence of SEQ ID NO. 20, preferably having at least 60%, 70%, 80%, 90%, 95%, or even 98% sequence identity to the sequence of SEQ ID NO. 20. The present invention may also comprise the method as indicated elsewhere herein, wherein the one or more polynucleotide sequence(s) is a polynucleotide sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to sequence of SEQ ID NO. 21, preferably having at least 60%, 70%, 80%, 90%, 95%, or even 98% sequence identity to the sequence of SEQ ID NO. 21.

Thus, the present invention may also comprise the method as indicated elsewhere herein, wherein the one or more polynucleotide sequence(s) is a polynucleotide sequence having any one of SEQ ID NOs. 15, 16, 17, 18, 19, 20, 21, 22, 23, 24, 25, 26, 27, or 28.

In an even more preferred embodiment of the present invention the one or more polynucleotide sequence(s) encoding a polypeptide is a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 1, said polynucleotide sequence encoding a polypeptide or fragment thereof having pheromone-responsive MAP kinase kinase activity;
or a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 2, said polynucleotide sequence encoding a polypeptide or fragment thereof having pheromone-responsive MAP kinase pathway-interacting activity;
or a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 3, said polynucleotide sequence encoding a polypeptide or fragment thereof having pheromone-responsive MAP kinase activity.
More preferably, the one or more polynucleotide sequence(s) encoding a polypeptide is a polynucleotide sequence encoding a polypeptide having an amino acid sequence as set out in any one of SEQ ID NOs. 1, 2, 3, 8, 9, or 10.

Especially preferred in the present invention is as the one or more polynucleotide sequence(s) encoding a polypeptide, a polynucleotide sequence encoding a polypeptide having an amino acid sequence having at least 25%, at least 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or even 100% sequence identity to the amino acid sequence of SEQ ID NO. 1. Mostly preferred in the present invention is a polynucleotide sequence encoding a polypeptide having an amino acid sequence of SEQ ID NO. 8. Thus, the present invention refers to the method as indicated elsewhere herein, wherein the one or more polynucleotide sequence(s) encoding a polypeptide is a polynucleotide sequence encoding a polypeptide having an amino acid sequence of SEQ ID NO. 1 or 8.

By genetically engineering said fungal host cell of the present invention due to knocking-out / deleting one or more polynucleotide sequence(s) encoding a protein as indicated above, preferably by knocking-out one polynucleotide sequence encoding a polypeptide having an amino acid sequence having at least 25 % sequence identity to the amino acid sequence of SEQ ID NO. 1, the fungal host cell of the present invention is not capable of switching from budding growth to filamentous growth in its vegetative stage.

Thus, the present invention may comprise under which stress conditions a fungal host cell of the present invention without being genetically engineered may be capable to switch its morphology. The capability of switching from budding growth to filamentous growth is determined by the influence of sunflower oil, low pH, high glucose concentration or glycerol concentration during culturing of the fungal host cell.

The inventors found that by culturing the fungal host cell being genetically engineered by knocking-out one or more polynucleotide sequence(s) encoding a polypeptide as indicated above (also referring to the term "deletion strain") in a culture medium comprising at least 5%, such as 5, 5.5, 6, 6.5, 6.6, 6.7, 6.8, 6.9, 7% sunflower oil, said deletion strain, in particular said deletion strain *Δras2, Δfuz7* and *Δubc3* demonstrated budding growth in comparison to said wild type strain (as a reference) not being genetically engineered by knocking-out one or more polynucleotide sequence(s) encoding a polypeptide as indicated above, which grew filamentously under those stess conditions of using sunflower oil. In particular, the capability of switching from budding growth to filamentous growth may be determined by the influence of a culture medium comprising 50 g L⁻¹ glucose, 7% (w/v) sunflower oil and 0.8 g L⁻¹ NH₄Cl, being buffered by 100mM MES (pH 6.5) during culturing of the fungal host cell.

Further, it was found that by culturing the fungal host cell being genetically engineered by knocking-out one or more polynucleotide sequence(s) encoding a polypeptide as indicated above (also referring to the term "deletion strain") in a culture medium comprising at least 20 g L⁻¹ CaCO₃, such as 20, 25, 30, 31, 32, or 33 g L⁻¹ CaCO₃; or comprising at least 50mM MES (pH 6.5), such as 50, 55, 60, 65, 70, 75, 80, 85, 90, 95 or 100mM MES (pH 6.5); or comprising at least 15mM MES (pH 6.5), such as 15, 20, 25, or 30mM MES (pH 6.5), said deletion strain, in particular said deletion strain *Δras2, Δfuz7* and *Δubc3* demonstrated budding growth in comparison to said wild type strain (as a reference) not being genetically engineered by knocking-out one or more polynucleotide sequence(s) encoding a polypeptide as indicated above, which grew filamentously under those stess conditions at low pH-values. Preferably, using a cuture medium comprising at least 15mM, such as 15, 20, 25, or 30mM MES (pH 6.5), in particular 30mM MES (pH 6.5) induces the lowest pH-values of below 3, thereby showing an increase in forming filaments in the wild type strain in comparison to the deletion strains showing budding growth under lowest pH conditions. The capability of switching from budding growth to filamentous growth may be determined by the influence of a culture medium comprising 50 g L⁻¹ glucose and either 33 g L⁻¹ CaCO₃ (w/v), or 100mM MES buffer (pH 6.5), or 30mM MES buffer (pH 6.5) during culturing of the fungal host cell. In particular, the capability of switching from budding growth to filamentous growth may be determined by the influence of a culture medium comprising 50 g L⁻¹ glucose, 30mM MES (w/v) and 0.8 g L⁻¹ NH₄Cl during culturing of the fungal host cell.

Additionally, it was demonstrated that by culturing the fungal host cell being genetically engineered by knocking-out one or more polynucleotide sequence(s) encoding a polypeptide as indicated above (also referring to the term "deletion strain") in a culture medium comprising at least 100, 120, 130, 140, 150, 160, 170, 180, 190, or 200 g L⁻¹ glucose and comprising either 33 g L⁻¹ CaCO₃, or 100mM MES or 30mM MES, said deletion strain, in particular said deletion strain *Δras2, Δfuz7* and *Δubc3* demonstrated budding growth in comparison to said wild type strain (as a reference) not being genetically engineered by knocking-out one or more polynucleotide sequence(s) encoding a polypeptide as indicated above, which grew filamentously under those stess conditions using high glucose concentrations. The capability of switching from budding growth to filamentous growth may be determined by the influence of a culture medium comprising 200 g L⁻¹ glucose and either 33 g L⁻¹ CaCO₃ (w/v), or 100mM MES buffer (pH 6.5), or 30mM MES buffer (pH 6.5) during culturing of the fungal host cell. In particular, the capability of switching from budding growth to filamentous growth may be determined by the influence of a culture medium comprising 200 g L⁻¹ glucose, 30mM MES (w/v) and 0.8 g L⁻¹ NH₄Cl during culturing of the fungal host cell.

Finally, the present inventors found that by culturing the fungal host cell being genetically engineered by knocking-out one or more polynucleotide sequence(s) encoding a polypeptide as indicated above (also referring to the term "deletion strain") in a culture medium comprising at least 50, 60, 70, 80, 90, or 100 g L⁻¹ glycerol as alternative C-source and 33 g L⁻¹ CaCO₃ (w/v), said deletion strain, in particular said deletion strain *Δras2, Δfuz7* and *Δubc3* demonstrated budding growth in comparison to said wild type strain (as a reference) not being genetically engineered by knocking-out one or more polynucleotide sequence(s) encoding a polypeptide as indicated above, which grew filamentously under those stess conditions using glycerol concentrations. The capability of switching from budding growth to filamentous growth may be determined by the influence of a culture medium comprising 100 g L⁻¹ glycerol and 33 g L⁻¹ CaCO₃ (w/v) during culturing of the fungal host cell. In particular, the capability of switching from budding growth to filamentous growth may be determined by the influence of a culture medium comprising 100 g L⁻¹ glycerol, 33 g L⁻¹ CaCO₃ (w/v) and 0.8 g L⁻¹ NH₄Cl during culturing of the fungal host cell.

Also contemplated by the present invention is the use of a fungal host cell belonging to the division of Basidiomycota for the production of a biomolecule, wherein the fungal host cell is due to genetic engineering not capable of switching from budding growth to filamentous growth in its vegetative stage.

The fungal host cell belonging to the division of Basidiomycota as being used herein may be from the family of Ustilaginaceae, the family of Dacrymycetaceae, or from the family of Moniliellaceae. Most preferably, the fungal host cell being used is from the family of Ustilaginaceae as indicated for the method of the present invention.

The definitions concerning the method of the present invention may also apply to said use of the present invention.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. The term "at least one" refers to one or more such as two, three, four, five, six, seven, eight, nine, ten and more. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

The term "about" means plus or minus 10%, preferably plus or minus 5%, more preferably plur or minus 2%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

A better understanding of the present invention and of its advantages will be gained from the following examples, offered for illustrative purposes only. The examples are not intended to limit the scope of the present invention in any way.

### EXAMPLES OF THE INVENTION

The following Examples illustrate the invention, but are not to be construed as limiting the scope of the invention.

### I. Experimental Procedures

### Example 1: Plasmid Isolation form E. coli.

Plasmids were isolated using the NEB Monarch nucleic acid purification miniprep kit according to the manual (New England Biolabs GmbH, Frankfurt - Germany).

### Example 2: Transformation of E. coli.

To transform and sub-clone plasmids into *E. coli,* purchased chemically competent cells from NEB were used. The transformation was carried out according to the chemically competent cells transformation protocol of Gibson Assembly® Master Mix Instruction Manual NEB #2611S/L from NEB.

### Example 3: Production of Protoplasts in U. maydis MB215 and U. cynodontis NBRC 9727.

Production of protoplasts was carried out according to Schulz et al. 1990 with minor modifications. As preculture cells were growing in 50 mL YEPS medium containing 10 g L⁻¹ sucrose, 10 g L⁻¹ peptone and 10 g L⁻¹ yeast extract for 36 hours, at 30 °C and 200 rpm, with a shaking diameter of 25 mm and a relative air humidity of 75 % in 500 mL flasks. Afterwards 50 mL YEPS medium containing 10 g L⁻¹ sucrose, 10 g L⁻¹ peptone and 10 g L⁻¹ yeast extract were inoculated with the preculture to an OD₆₀₀ = 0.2 at 30 °C and 200 rpm, with a shaking diameter of 25 mm and a relative air humidity of 75 % in 500 mL flasks. After the mainculture reached an OD₆₀₀ = 0.8, the whole culture (50mL) was centrifuged at 4000 rpm at 4 °C for 10 minutes. The pellet was re-suspended in 25 mL SCS (1 M sorbitol, 20 mM sodium citrate, pH 5.8) and again centrifuged at 4000 rpm at 4 °C for 10 minutes. The pellet was then re-suspended in 2 mL SCS/Lysing-enzyme (20 mg mL⁻¹ from Trichoderma harzianum, Sigma Aldrich Chemie GmbH, Taufkrichen, Germany) and incubated at room temperature for 6-12 minutes until 80 % of the cells reached the state of protoplastation, which was audited using microscopy. To stop the lysing reaction 10 mL of SCS were added. The cells were centrifuged at 2300 rpm at 4 °C for 10 minutes and the resulting pellet was washed three times with SCS with the same centrifugation settings. After the last washing step, the pellet was re-suspended in STC-buffer (100 mM calcium chloride, 1 M sorbitol in 10 mM Tris-HCI, pH 7.5) and aliquots of 50 µL were stored at - 80°C.

### Example 4: Transformation of U. maydis MB215 and U. cynodontis NBRC 9727 by protoplasts.

Transformation of Ustilago by protoplasts was carried out according to Tsukuda et al. 1988. Aliquots of protoplasts were thawed on ice for 10 minutes. Afterwards 500 to 1500 ng of DNA were added, followed by incubation on ice for 10 minutes. Afterwards 500 µL of STC-PEG solution (40 % /w/v) PEG 4,000 in STC) were added and the cells were incubated for 10 minutes on ice. The suspension was then spread on REG-agar plates containing the necessary antibiotics. Colonies were obtained after two to four days of incubation at 30 °C.

### Example 5: Plasmid isolation from U. cynodontis NBRC 9729.

For plasmid isolation from *U. cynodontis* NBRC 9727 protoplastation as described in 3 was performed. Afterwards the supernatant was decanted, and the plasmid isolation was performed according to **Example 1** with one modification. In the last step 30 µL of elution buffer were used. Subsequently the whole isolated plasmid was used for transformation in *E*. *coli* competent cells. Afterwards for following work the plasmid could be isolated from *E. coli.*

### Example 6: Colony PCR in U. maydis MB215 and U. cynodontis NBRC 9727.

Colony PCR was carried out according to Blount et al. with minor modifications (Blount et al. 2016). Therefore 5 % (w/v) Chelex was prepared and 100 µL were given in tubes with glass beads (0,5mm). Afterwards, cell material of Ustilago strains from agar plates were added to the suspension. For decomposition of the cells the tubes were vortexed for ten minutes and incubated at 100 °C for six minutes. Afterwards, centrifugation occurred at maximum g for one minute. At the end 1 µL of the supernatant were used for PCR reaction.

### Example 7: Isolation of genomic DNA in U. maydis MB215 and U. cynodontis NBRC 9727.

Genomic DNA was isolated as described by Hoffman and Winston 1987 (Hoffman and Winston 1987). Cells were grown 24 h at 30°C in 1.5mL YEPS medium containing 10 g L-1 sucrose, 10 g L-1 yeast extract and 10 g L-1 peptone in System Duetz® (24 well plate), at 30°C and 300 rpm with a shaking diameter of 50 mm and a relative air humidity of 75 %. The cells were separated from the media by centrifugation at 5000 rpm for 5 minutes. The resulting pellet was washed with demineralized water two times. Afterwards the supernatant was decanted, and the pellet was re-suspended in the remaining water. Then, 400 µL of lysing buffer /7 %(w/v) TritonX-100, 1 % /w/v) SDS, 100 mM NaCl, 10 mM Tris-HCI pH 8.0, 1 mM EDTA), 400 µl phenol-chloroform-isoamylalcohol (PCI 24:24:1) and glass beads with a diameter of 0.5 mm were added. The mixture was then vortexed for 10 minutes. Afterwards 200 µl of TE-buffer (10 mM Tris-HCI, 1 mM EDTA pH 8.0) were added and the mixture was centrifuged at 12000 rpm for 5 minutes. The resulting aquatic phase was transferred into a new tube and 1000 µl ice cold 100 % ethanol were added. After inversion the tube was centrifuged at 1200 rpm for 2 minutes and the resulting pellet was dissolved in 400 µl TE-buffer containing 5 µl RNAseA (10 mg mL⁻¹ in TE-buffer). The solution was incubated for 1 hour at 37°C. Then 500 µL of PCI were added followed by strong vortexing for 20 seconds. The resulting aquatic phase was transferred into a new tube and 500 µL of chloroform-isoamylalcohol (CI 24:1) were added. The mixture was again strongly vortexed for 20 seconds and centrifuged at 12000 rpm for 10 minutes. After transferring the aquatic phase into a new tube, the last step was repeated twice. Then, the aquatic phase was transferred into a new tube and 0.3 M ammonium-acetate dissolved in ethanol (2.5 fold of sample volume) were added. After inverting and centrifugation at 12000 rpm for two minutes the supernatant was decanted and the dried pellet re-suspended in 50 µL TE-buffer and stored at 4 °C.

### Example 8: Microscopy.

For culture observation microscope Leica DM750 was used (Leica Microsystems, Wetzlar, Germany) with 10x, 40x, 63x and 100x oil immersion objectives. For images all samples were set to an OD₆₀₀ = 5 and 10 µL culture was used for microscopy.

### Example 9: Determination of biomass.

The optical density of cell cultures was measured using Ultrospec 10 Cell Density Meter (Amersham Biosciences, Chalfont St Giles, UK) at 600 nm (OD₆₀₀). Determination took place in 4 mL Rotilabo polystyrol Makro cuvettes from Carl Roth with distilled water as blank. Samples were diluted to an OD between 0.2 and 0.3. To determine cell dry weight, 1mL culture were centrifuged for three minutes at 13.3 rpm, afterwards supernatant was discarded and pellet was washed once with 2 mL 0.9 % NaCl and and afterwards resuspended in 200 µL 0.9 % NaCl. Afterwards the suspension was transferred to filter paper and was dried for 24 h at 100 °C. Afterwards cell dry weight was determined with an infrared dryer including analysis scales.

### Example 10: Quantification of glucose, glycerol, 2-hydroxyparaconate, erythritol and itaconate by HPLC.

Products and substrates were analyzed in a DIONEX UltiMate 3000 High Performance Liquid Chromatography System (Thermo Scientific, Germany) with an ISERA Metab AaC column 300 x 7.8 mm column (ISERA, Germany). As solvent 5 mM H2SO4 with a flow rate of 0.6 mL min-1 and a temperature of 40 °C was used. All samples were filtered with Acrodisc® Syringe Filters (GHP, 0,20 µm, Ø 13 mm). Itaconate, 2-hydroxyparaconate, erythritol, glycerol and glucose were determined with refractive index detector SHODEX RI-101 (Showa Denko Europe GmbH, Germany). Itaconate, 2-hydroxyparaconate, erythritol, glycerol and glucose were identified via retention time and UV/RI quotient comaperd to corresponding standards. Synthesized 2-hydroxyparaconate (purity ≈ 70%) was used as HPLC standard for quantification and indicated hydroxyparaconate values should be taken as rough estimates only.

### Example 11: Cultivation conditions.

As standard growth medium, modified Tabuchi medium (mTm) containing 0.8 g L⁻¹ NH₄Cl , 0,2 g L⁻¹ MgSO₄ x 7 H₂O, 0.5 g L⁻¹ FeSO₄ x 7 H₂O, 0.5 g L⁻¹ KH₂PO₄, 1 mL L⁻¹ vitamin solution, 1mL L⁻¹ trace element solution and differing concentrations of glucose and buffer systems were used.
Vitamin solution contained 0,005 g L⁻¹ D-biotin, 1 g L⁻¹ D-calcium penthotenate, 1 g L⁻¹ nicotinic acid, 25 g L⁻¹ myo-inositol, 1 g L⁻¹ thiamine hydrochloride, 1 g L⁻¹ pyridoxol hydrochloridem and 0.2 g L⁻¹ para-aminobenzoic acid.
Trace element solution contained 1.5 g L⁻¹ EDTA, 0.45 g L⁻¹ ZnSO₄ x 7 H₂O, 0.10 g L⁻¹ MnCl₂ x 4 H₂O, 0.03 g L⁻¹ CoCl₂ x 6 H₂O, 0.03 g L⁻¹ CuSO₄ x 5 H₂O, 0.04 g L⁻¹ Na2MoO₄ x 2 H₂O, 0.45 g L⁻¹ CaCl₂ x 2 H₂O, 0.3 g L⁻¹ FeSO₄ x 7 H₂O, 0.10 g L⁻¹ H₃BO₃ and 0.01 g L⁻¹ Kl.
As production medium A in shake flasks mTm containing 50 g L⁻¹ glucose, 7 % /(w/v) **sunflower oil** and 100 mM MES was used.
As production medium B in System Duetz® (24 well plates) mTm containing 50 g L⁻¹ glucose and **33 g L⁻¹ CaCO3** was used.
As production medium C in System Duetz® (24 well plates) mTm containing 50 g L⁻¹ glucose and **100 mM MES** (pH=6.5) was used.
As production medium D in System Duetz® (24 well plates) mTm containing 50 g L⁻¹ glucose and **30 mM MES** (pH=6.5) was used.
As production medium E in System Duetz® (24 well plates) mTm containing **200 g L⁻¹ glucose** and **33 g L⁻¹ CaCO3** was used.
As production medium F in System Duetz® (24 well plates) mTm containing **200 g L⁻¹ glucose** and **100 mM MES** (pH=6.5) was used.
As production medium G in System Duetz® (24 well plates) mTm containing **200 g L⁻¹ glucose** and **30 mM MES** (pH=6.5) was used.
As production medium H in shake flasks (500 mL) mTm containing **100 g L⁻¹ glycerol** and **33 g L⁻¹ CaCO3** was used.
As production medium I in System Duetz® (24 well plates) mTm containing **100 g L⁻¹ glycerol** and **33 g L⁻¹ CaCO3** was used.
As production medium J in System Duetz® (24 well plates) mTm containing **100 g L⁻¹ glucose** and **66 g L⁻¹ CaCO3** was used.
As preculture for production medium H 50 mL of mTm containing 50 g L⁻¹ glycerol and 100 mM MES were inoculated with 1 mL from a 36 h YEPS culture containing 10 g L⁻¹ yeas extract, 10 g L⁻¹ peptone and 10 g L⁻¹ sucrose. This culture was grown for 36 h and used to inoculate the production culture to a starting OD of 0.5.
As preculture for production medium A, B, C, D, E, F and G 1.5 mL of mTm containing 50 g L⁻¹ glucose and 100 mM MES were inoculated with cell material from a YEPS-plate containing 10 g L⁻¹ yeast extract, 10 g L⁻¹ peptone, 10 g L⁻¹ sucrose an 20 g L⁻¹ Agar. This culture was grown for 36h and used to inoculate the production culture to a starting OD of 0.5.
All shake flask cultures were incubated at 30°C shaking at 200 rpm, with a shaking diameter of 25 mm and a relative air humidity of 75% in 500 mL flasks with 50 mL filling volume.
All System Duetz® cultures were inoculated at 30°C shaking at 300 rpm, with a shaking diameter of 50 mm and a relative air humidity of 75 % in 24 well plates with 1.5 filling volume.

### Example 12: Bioreactor conditions.

Fed-batch fermentations were prepared in one liter vessel from Eppendorf containing 0.5 L mTm with 50 g L⁻¹ glucose. New Brunswick™ BioFlo110® was used as control station. For inoculation preculture containing mTm with 50 g L-1 glucose and 100 mM MES were used. Preculture was grown for 36 h in 500 mL shaking flask. To inoculate reactor, preculture was washed once with 0.9 % NaCl, afterwards the bioreactor was inoculate with a starting OD₆₀₀ of 0.75. Starting volume of the bioreactor was 500 mL, the stirrers were set on 1000 rpm, aeration rate was set to 1vvm and temperature was set on 30 °C. To regulate the pH in the beginning 1 M Hcl and 10 M NaOH were used. After inoculation pH just was regulated with 10 M NaOH, because of the produced acid. The pH-values were recorded by a 405-DPAS-SC-K8S-elctrode from Mettler Toledo and dO₂ signal was recorded with an oxygen-electrode from mettler Toledo. To keep the temperature constant at 30°C cooling system from the company lauda was used (type VC2000).

### II. Results

### Example 13: Examination of carboxin (cbx), hygromycin (hyg), phleomycin (phi) and nourseothricin (nat) sensitivity in U. cynodontis.

To be able to use antibiotics as a selection marker, first a drop-test was carried out to determine the sensitivity of *U. cynodontis* NBRC 9727 against cbx, hyg, phl and nat.
To find out the sensitivity on cbx Medium I was used containing 10 g L⁻¹ yeast extract, 10 g L⁻¹ peptone, 10 g L⁻¹ sucrose and 20 g L⁻¹ Agar which were supplement with 1, 2, 3, 5, 7.75, 10 or 15 µg mL⁻¹ cbx respectively.
To find out the sensitivity on hyg Medium J was used containing 10 g L⁻¹ yeast extract, 10 g L⁻¹ peptone, 10 g L⁻¹ sucrose and 20 g L⁻¹ Agar which were supplement with 100, 200, 300, 400 or 500 µg mL⁻¹ hyg respectively.
To find out the sensitivity on nat Medium K was used containing 10 g L⁻¹ yeast extract, 10 g L⁻¹ peptone, 10 g L⁻¹ sucrose and 20 g L⁻¹ Agar which were supplement with 1, 3, 5, 10, 20, 30, 40, 50, 75, 100 and 150 µg mL⁻¹ nat respectively.
To find out the sensitivity on phl Medium L was used containing 10 g L⁻¹ yeast extract, 10 g L⁻¹ peptone, 10 g L⁻¹ sucrose and 20 g L⁻¹ Agar which were supplement with 1, 5, 10, 13, 25, 50, 100, 200 and 300 µg mL⁻¹ phl respectively.

As positive control *U. cynodontis* NBRC 9727 was grown on YEPS plate containing 10 g L⁻¹ yeast extract, 10 g L⁻¹ peptone, 10 g L⁻¹ sucrose and 20 g L⁻¹ Agar.

As preculture *U. cynodontis* NBRC 9727 was grown for 24h in YEPS-medium containing 10 g L⁻¹ yeast extract, 10 g L⁻¹ peptone and 10 g L⁻¹ sucrose in 100 mL shaking flasks containing 10 mL culture. Culture was incubated at 30 °C at 200 rpm, with a shaking diameter of 25 mm and a relative air humidity of 75 %. From these preculture Medium I, J, K and L were inoculated. Each plate contained four inoculation spots (each 10 µL) with different OD₆₀₀ values of 1, 0.1, 0.01 and 0.001 as duplets. The physiology changes of the colonies were documented via photography every 24 hours for 4 days. The antibiotics concentration of the plates on which no cell growth could be detected were chosen as selection marker concentrations for further work. For *U. cynodontis* NBRC 9727 no growth was observed after 72 hours of 2 µg mL⁻¹ cbx, 10 µg mL⁻¹ phl, 10 µg mL⁻¹ nat and 200 µg mL⁻¹ hyg.

### Example 14: Introduction of a resistance cassette to U. cynodontis NBRC 9727.

To be able to use the mentioned antibiotics in **Example 13,** selection marker had to be established which can made *U. cynodontis* NBRC 9727 resistance against those antibiotics. To test if the corresponding selection markers are functional, protoplasts were transformed with the episomally replicating plasmids pNEBUC (cbx resistance cassette), pNEBUN (nat resistance cassette), pNEBUP (phl resistance cassette), and genome-integrated pSMUT (hyg resistance cassette). Resulting colonies on selective medium plates with the respective antibiotics were screened. Plasmid re-isolation (mentioned in **Example 5**) resulted in the correct plasmid for pNEBUN, pNEBUP and pNEBUC. Colony PCR resulted in the correct size for pSMUT.

### Example 15: Deletion of ras2, fuz7 and ubc3 in U. cynodontis NBRC 9727.

For deletion of *ras2* in *U. cynodontis* NBRC 9727 the FLP/FRT mediated recombination system was adapted from Khrunyk et al. 2010. In the first step plasmids had to be designed which contained the deletion constructs for *ras2, fuz7* and *ubc3.* Afterwards, the deletion construct for the gene of interest was integrated in the genomic locus of *U. cynodontis* NBRC 9727 by homologous recombination, yielding a hygromycin resistant transformant. PCR was used to allow identification of positive deletion, plus verification of the correct localization inside of the genome.

For the construction of the deletion constructs the flanking regions were amplified from *U. cynodontis* NBRC 9727 genome for every gene of interest (*ras2, fuz7, ubc3*).

For ras2 primer pair HT-168 and HT-169 for flanking region-1 and primer pair HT-172 and HT-173 for flanking region-2 were used, using Q5 as polymerase according to the manual (Q5® High-Fidelity DNA Polymerase / New England BioLabs, Frankfurt am Main). Both resulted in a PCR fragment of 1030 bp.

For *fuz7* primer pair HT-180 and HT-181 for flanking region-1 and primer pair HT-184 and HT-185 for flanking region-2 were used, using Q5 as polymerase according to the manual (Q5® High-Fidelity DNA Polymerase / New England BioLabs, Frankfurt am Main) . Both resulted in a PCR fragment of 1030 bp.

For *ubc3* primer pair HT-174 and HT-175 for flanking region-1 and primer pair HT-178 and HT-179 for flanking region-2 were used, using Q5 as polymerase according to the manual (Q5® High-Fidelity DNA Polymerase / New England BioLabs, Frankfurt am Main). Both resulted in a PCR fragment of 1030 bp.

PCR conditions for the Flanking region-1 and 2 of *ras2, fuz7* and *ubc3* are shown in **Tab.1.** Primer sequences are shown in **Tab. 3.**

**Tab. 1: PCR conditions for flanking regions 1 and 2 of ras2, fuz7 and ubc3.**

| | |
|---|---|
| 98°C | 300 seconds |
| 98°C | 5 seconds |
| 64°C | 5 seconds |
| 72°C | 30 seconds |
| 72°C | 60 seconds |

Further, the hygromycin cassette including FRT-sites was amplified from the plasmid pStorl_1rh_pTOa1522.

For ras2 primer pair HT-170 and HT-171 were used to amplify the hygromycin cassette including FRT-sites using Q5 as polymerase according to the manual (Q5® High-Fidelity DNA Polymerase / New England BioLabs, Frankfurt am Main), which resulted in a band of 2808 bp.

For *fuz7* primer pair HT-176 and HT-177 were used to amplify the hygromycin cassette including FRT-sites using Q5 as polymerase according to the manual (Q5® High-Fidelity DNA Polymerase / New England BioLabs, Frankfurt am Main), which resulted in a band of 2808 bp.

For *fuz7* primer pair HT-182 and HT-183 were used to amplify the hygromycin cassette including FRT-sites using Q5 as polymerase according to the manual (Q5® High-Fidelity DNA Polymerase / New England BioLabs, Frankfurt am Main), which resulted in a band of 2808 bp.

PCR conditions for the hygromycin cassette are shown in **Tab. 2.** Primer sequences are shown in **Tab. 3.**

**Tab. 2: PCR conditions for amplification of the hygromycin cassette from pStorl_1rh_pTOa1522.**

| | |
|---|---|
| 98°C | 30 seconds |
| 98°C | 5 seconds |
| 60°C | 5 seconds |
| 72°C | 120 seconds |
| 72°C | 60 seconds |

Afterwards all PCR products were purified and assembled via Gibson cloning, together with pJET1.2 blunt as backbone (Gibson Asembly® Master Mix from New England Biolabs (NEB) and the NEBuilder® Assembly Tool were used according to the manual. Afterwards the plasmid was sub-cloned into *E. coli* competent cells.

To verify that the deletion construct for the genes were assembled right, PCR analyses was used using OneTaq®-polymerase.

For *ras2* primer pair HT-100 and HT-101 were used, which resulted in a band of 4869 bp, further to verify the correct size of the whole plasmid (**Fig**. **1A**) restriction analysis with *Pstl* was used according to the manual, which resulted in one band at 2535 bp and one band at 5227 bp.

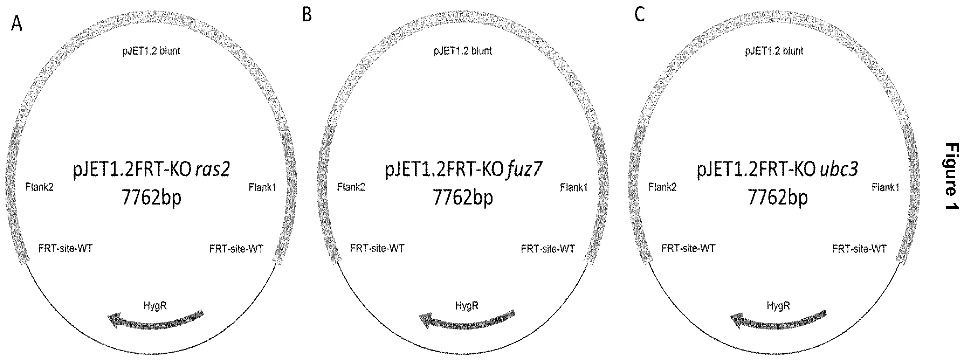

For *fuz7* primer pair HT-109 and HT-110 were used, which resulted in a band of 4786 bp, further to verify the correct size of the whole plasmid (**Fig. 1B**) restriction analysis with *Pstl* was used according to the manual, which resulted in a band at 970 bp, 1565 bp and 5227bp.

For *ubc3* primer pair HT-117 and HT-118 were used, which resulted in a band of 4754 bp, further to verify the correct size of the whole plasmid (**Fig. 1C**) restriction analysis with *Pstl* was used according to the manual, which resulted in a band at 727bp. 1808 bp, 2363 bp and 2864 bp.

PCR conditions for the verification are shown in **Tab. 4.** Primer sequences are shown in **Tab. 3.**

**Tab. 3: Necessary primer sequences to assemble deletion constructs of ras2, fuz7 and ubc3 in U. cynodontis NBRC 9727.**

| | |
|---|---|
| HT-168 | ctcgagtttttcagcaagatCTTCTACAAGCGTGATTTACAAAGG (SEQ ID NO. 29) |
| HT-169 | acttctggccGTGGTCAGTCAGAGGGCG (SEQ ID NO. 30) |
| HT-172 | acttctggccGTTGGGCAACACTGTATTC (SEQ ID NO. 31) |
| HT-173 | aggagatcttctagaaagatGTCTCTCTCTCAAGCACAC (SEQ ID NO. 32) |
| HT-180 | ctcgagtttttcagcaagatTCCGCTTTGAGGTACAGTTG (SEQ ID NO. 33) |
| HT-181 | acttctggccGGTCGCCCCTGTGATAGTG (SEQ ID NO. 34) |
| HT-184 | acttctggccGATGGCAGCCCTAATGAG (SEQ ID NO. 35) |
| HT-185 | aggagatcttctagaaagatGCCCACATTAGCAGGTGG (SEQ ID NO. 36) |
| HT-174 | ctcgagtttttcagcaagatCCAAATCCGCCGTCGGAG (SEQ ID NO. 37) |
| HT-175 | acttctggccAAGTGGAAGTGTTGGAAGCATTTC (SEQ ID NO. 38) |
| HT-178 | acttctggccTGAGCAAGAGAGTGTGTTC (SEQ ID NO. 39) |
| HT-179 | aggagatcttctagaaagatCACGATCACACTTCCATC (SEQ ID NO. 40) |
| HT-170 | gactgaccacGGCCAGAAGTTCCTATTC (SEQ ID NO. 41) |
| HT-171 | gttgcccaacGGCCAGAAGTTCCTATAC (SEQ ID NO. 42) |
| HT-176 | acttccacttGGCCAGAAGTTCCTATTC (SEQ ID NO. 43) |
| HT-177 | ctcttgctcaGGCCAGAAGTTCCTATAC (SEQ ID NO. 44) |
| HT-182 | aggggcgaccGGCCAGAAGTTCCTATTC (SEQ ID NO. 45) |
| HT-183 | ggctgccatcGGCCAGAAGTTCCTATAC (SEQ ID NO. 46) |
| HT-100 | AAGGGTGGCATCGAGGAGAG (SEQ ID NO. 47) |
| HT-101 | CTCGAGCCACTTAGCCTTTC (SEQ ID NO. 48) |
| HT-109 | TCCGCTTTGAGGTACAGTTG (SEQ ID NO. 49) |
| HT-110 | CCACATTAGCAGGTGGTATC (SEQ ID NO. 50) |
| HT-117 | GACAAGCTCAGTGCCCTCTC (SEQ ID NO. 51) |
| HT-118 | CACTTCCATCGGAACATGTG (SEQ ID NO. 52) |
| HT-119 | CGATCCCGCGATTATCAC (SEQ ID NO. 53) |
| HT-120 | CCAGTCACTCGCTCATTC (SEQ ID NO. 54) |
| HT-121 | ACGATTGTGCCAAGCTTC (SEQ ID NO. 55) |
| HT-122 | GCAGCTGATTGATGTTGG (SEQ ID NO. 56) |
| HT-123 | CCATGTCTAGCTCCTTTC (SEQ ID NO. 57) |
| HT-124 | CGAGACAGGTTGACTTTC (SEQ ID NO. 58) |

**Tab. 4: PCR conditions for verification of the deletion constructs for ras2, fuz7 and ubc3.**

| | |
|---|---|
| 94°C | 30 seconds |
| 94°C | 30 seconds |
| 53°C | 60 seconds |
| 68°C | 270 seconds |
| 68°C | 5 min |

After verification, deletion constructs were amplified from the corresponding plasmids to obtain a linearized DNA for transformation.

For *ras2* deletion construct primer pair HT-100 and HT-101 were used, which resulted in a band of 4869 bp, for *fuz7*deletion construct primer pair HT-109 and HT-110 were used, which resulted in a band of 4786 bp and for *ubc3* deletion construct primer pair HT-117 and HT-118 were used, which resulted in a band of 4754 bp. As polymerase Q5 was used according to the manual (Q5® High-Fidelity DNA Polymerase / New England BioLabs, Frankfurt am Main). PCR conditions for the verification are shown in **Tab. 5.** Primer sequences are shown in **Tab. 3.**

**Tab. 5: PCR conditions for amplification of deletion constructs for ras2, fuz7 and ubc3.**

| | |
|---|---|
| 98°C | 30 seconds |
| 98°C | 5 seconds |
| 64°C | 10 seconds |
| 72°C | 140 seconds |
| 72°C | 2 min |

The final linear DNA fragment was transformed into *U. cynodontis* NBRC 9727 according to **Example 4.** Cells which could grow on hygromycin containing REG-agar plates were picked and further analyzed by colony PCR.

To verify deletion of *ras2* primer pair HT-119 and HT-120 were used, which resulted in a band of 4902 bp. As negative control wildtype was used, which resulted in a band of 2693 bp.

To verify deletion of *fuz7* primer pair HT-121 and HT-122 were used, which resulted in a band of 4943 bp. As negative control wildtype was used, which resulted in a band of 3746 bp.

To verify deletion of *ubc3* primer pair HT-123 and HT-124 were used, which resulted in a band of 4865 bp. As negative control wildtype was used, which resulted in a band of 3141 bp.

As polymerase Q5 was used according to the manual (Q5® High-Fidelity DNA Polymerase / New England BioLabs, Frankfurt am Main). PCR conditions for the verification are shown in **Tab. 6.** Primer sequences are shown in **Tab. 3.**

**Tab. 6: PCR conditions for verification of deletion of ras2, fuz7 and ubc3 in U. cynodontis NBRC 9727.**

| | |
|---|---|
| 98°C | 30 seconds |
| 98°C | 10 seconds |
| 64°C | 30 seconds |
| 72°C | 2.4 min |
| 72°C | 2 min |

### Example 16: Cultivation studies in U. cynodontis ras2, U. cynodontis fuz7 and U. cynodontis ubc3.

After deletion strains were verified, cultivation studies in different media's were prepared. Where it was known that the wildtype *U. cynodontis* NBRC 9727 show a filamentous growth behavior, further media was used which was optimized for itaconic acid production in *U. maydis.*

### I Cultivation behavior in production medium A (for medium A see Example 11)

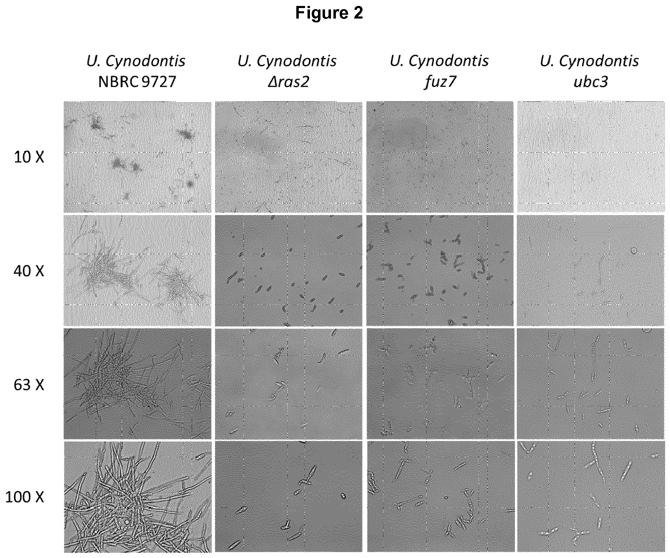

*U. cynodontis* NBRC 9727, *U. cynodontis* NBRC 9727 Δ*ras2, U. cynodontis* NBRC 9727 Δ*fuz7* and *U. cynodontis* NBRC 9727 Δ*ubc3* were cultivated in production medium A as triplicates. The cultivation took place for 95 hours, whereby samples were taken after 0 h, 24 h, 48 h and 95 h. **Fig. 2** show images of the cultures after 48h. After 24 h *U. cynodontis* NBRC9727 *already* showed a strong filamentous behavior and keept it for the whole cultivation time (for 95 hours). Compare to the wildtype, the deletion strains did not show any filamentous growth behaviour, instead they showed a yeast like morphology for the whole cultivation time.

### II Cultivation studies in production medium B, C and D (for medium B, C and D see Example 11)

In previous studies it could be shown that *U. cynodontis* NBRC 9727 produces more itaconic acid than *U. maydis,* especially at low pH-values. Unfortunately filamentous growth was observed too, which is not a benefit for the work in a bioreactor. In this part *U. cynodontis* Δ*ras2, U. cynodontis* Δ*fuz7, U. cynodontis* Δ*ubc3* were characterized in production medium B, C and D to see if filamentous growth is also abolished in production conditions for itaconic acid. As control strain wildtype *U. cynodontis* NBRC 9727 were cultivated too. The cultivations have been performed in triplicates and had a total duration of 96 h, whereby samples were taken after 24 h, 48 h, 72 h and 96 h. Microscopy images are shown in **Fig. 3****,** **4** and **5****.** Further in **Fig. 6** endpoint values of biomass formation, yield for itaconic acid, titer for itaconic acid and hydroxyparaconate after 96 h are shown. **Fig. 7** represents process values for OD₆₀₀, pH, 2-hydroxyparaconate and itaconic acid over time.

During cultivation with 33 g L⁻¹ CaCO₃ (production medium B) the pH values for all strains (*U*. *cynodontis* Δ*ras2, U. cynodontis* Δ*fuz7, U. cynodontis* Δ*ubc3* and *U. cynodontis* NBRC 9727) were between 6.5 and 7.5, whereas a strong decrease could be observed after 48 h when 30mM MES (production medium D) was used, pH value dropped from 6.5 to 2.7. In cultures where 100 mM MES (production medium C) was used, ph dropped constantly from 6.5 to 3.8 after 96 h **(****Fig. 7**). For all three production mediums (B, C and D) strong filamentous growth was observed for *U. cynodontis* NBRC 9727. At pH-values below 3 stronger filamentous growth behavior could be observed. In contrast no filamentous growth was observed for *U. cynodontis* Δ*ras2, U. cynodontis* Δ*fuz7,* and *U. cynodontis* Δ*ubc3.* Exemplary microscopy images are shown in **Fig. 3** (production medium D), **Fig. 4** (production medium C) and **Fig. 5** (production medium B) after 72 h cultivation. Even similar morphology could be observed for *U. cynodontis* Δ*ras2, U. cynodontis* Δ*fuz7* and *U. cynodontis* Δ*ubc3,* difference in biomass formation, itaconic acid production and 2-hydroxyparaconate production could be detected **(****Fig. 6** and **7**). In all three production mediums (B, C and D) *U. cynodontis* Δ*ubc3* showed the highest OD600 values for biomass, but also showed significant lower itaconic acid concentrations compared to the wildtype. This phenotype was also observed for *U. cynodontis* Δ*ras2.* In contrast *U. cynodontis* Δ*fuz7* showed in all production media (B, C and D) higher values for itaconic acid compared to the wildtype. In summary *U. cynodontis* Δ*fuz7* resulted in all three production media the highest yields (g_{ITA} g_{glc}⁻¹). Wildtype *U. cynodontis* NBRC 9727 showed a stronger filamentous character at lower pH values, compared to the deletion strains (*U. cynodontis* Δ*ras2, U. cynodontis* Δ*fuz7,* and *U. cynodontis* Δ*ubc3),* no dependent changes in morphology were observed.

### III. Cultivation studies in production medium E, F and G (for medium E, F and G see Example 11)

It is known that high glucose concentrations can influence biomass formation, yield and titer of itaconic acid in *Ustilago.* Further, high glucose concentrations resulted in osmotic stress which can induce filamentous growth. To test the effect of high glucose concentrations on *U*. *cynodontis* NBRC 9727, *U. cynodontis* Δ*ras2, U. cynodontis* Δ*fuz7 and U. cynodontis* Δ*ubc3 cultivation studies in production medium* E, F and G were performed. The cultivation were prepared in triplicates and had a total duration of 216 h, whereby samples were taken after 24 h, 48 h, 72 h, 96 h, 168 h and 216 h. Microscopy images are shown in **Fig. 8****,** **9** and **10****.** Further, in **Fig. 11** and **12** endpoint values of biomass formation, yield for itaconic acid, titer for itaconic acid and 2- hydroxyparaconate after 96h and 216 h are shown. Further, in **Fig. 13** process parameter for biomass formation, pH-values, 2-hydroxyparaconate and itaconic acid production over time are shown. In all three conditions (production medium E, F and G) *U. cynodontis* NBRC 9727 showed a strong filamentous growth behavior. Further, in medium E (see **Fig. 10**) *U. cynodontis* Δ*ras2* also showed significant filamentous growth. In medium F and G *U. cynodontis* Δ*ras2, U. cynodontis* Δ*fuz7* and *U. cynodontis* Δ*ubc3* grew yeast-like (see **Fig. 8** and **9**). The high initial concentration of glucose (200 g L⁻¹) had an influence on biomass formation and on 2-hydroxyparaconate, but no significant differences in itaconic acid production compared to low initial glucose concentrations (50 g L⁻¹) were observed (see **Fig. 11** and **12**).

### IV Cultivation studies in production medium H (for medium H see Example 11)

Glycerol is a waste product from biodiesel production and is an interesting alternative carbon source for biotechnological industry. Because it is known from previous work with *Ustilago* demonstrating that also glycerol can induce filamentous growth, cultivation studies in production medium H with *U. cynodontis* NBRC 9727, *U. cynodontis* Δ*ras2, U. cynodontis* Δ*fuz7 and U. cynodontis* Δ*ubc3* were performed. The cultivation was prepared in triplicates and had a total duration of 384 h. After 0, 48-, 96-, 168-, 312 and 384 h samples were taken. Microscopy images after 48 h are shown in **Fig. 14****.** Further, in **Fig. 15** endpoint values of itaconic acid and 2-hydroxyparaconate after 384 h are shown. Additionally, in **Fig. 16** used flasks after 384 h are shown and **Fig. 17** shows process parameter for biomass formation, pH-values, and titer of 2-hydroxyparaconate and itaconic acid production over time. Strong filamentous growth could be observed during the whole cultivation for *U. cynodontis* NBRC 9727, while, *U. cynodontis* Δ*ras2, U. cynodontis* Δ*fuz7 and U. cynodontis* Δ*ubc3* showed single cell growth. In particular, *U*. *cynodontis* Δ*fuz7* grew yeast-like, where the cells were organized in a clustered formation (see **Fig. 14**). For itaconic acid production *U. cynodontis* Δ*fuz7* reached the highest titer with 8.39 g L⁻¹ (see **Fig. 15**). Further, while *U. cynodontis* Δ*fuz7* kept its yellow color, *U. cynodontis* NBRC 9727, *U. cynodontis* Δ*ras2 and U. cynodontis* Δ*ubc3* turned black over time (see **Fig. 16**). That could be a sign for microbial stress, indicating that *U. cynodontis* Δ*fuz7* is more stress-resistant. *U. cynodontis* Δ*fuz7* showed a stable single cell growth behavior and the best values for itaconic acid production. Based on these findings, next experiments were performed with *U. cynodontis* Δ*fuz7.*

### Example 17: Determination of the optimal pH for itaconic acid production for U. cynodontis Δfuz7 in a bioreactor.

To obtain the optimal pH value for itaconic acid production in *U. cynodontis* Δ*fuz7* fed batch fermentation as described in **Example 12** were prepared. As pH values 1.9, 2.5, 3.2, 3.4, 3.6, 3.8, 5.5 and 6.0 were chosen. All fed batch were prepared as duplicates instead of the fed batch with pH = 3.6. A bioreactor which was controlled at a pH = 1.9 was fed with 100 mL of a 50 % glucose stock after 116.5 h, a bioreactor which was controlled at a pH = 2.5, 3.2, 3.4, 3.6, 3.8 and 5.5 was fed with 50 mL of a 50 % glucose stock solution after 69.5 h and 158.5 h and a bioreactor which was controlled at a pH = 6 was fed with 100 mL of a 50 % glucose stock solution after 27 h. Further the total duration of all bioreactors was 288 h, whereby everyday samples were taken. Values for OD₆₀₀ after 24h, Od_{Max} after 68.5, CDW_{Max} (g L⁻¹), Titer_{Max} for itaconate in g L⁻¹, productivity in g L⁻¹ h⁻¹ and the yield for itaconate in g_{ITA}/g_{GLC} are shown in **Fig. 18****.** Further, yield and titer for itaconic acid production after 284 h are shown in **Fig. 19****.**

*U. cynodontis* Δ*fuz7* showed at a pH = 5.5 the highest rates for biomass formation after 24 h. Best production condition for itaconate was at a pH at 3.6 (see **Fig. 18**). Said pH = 5.5 was chosen as optimum for biomass formation. Highest titer of 24.72 g L-1 and a yield of 0.27 gᵢₜₐ g_{glc}⁻¹ could be reached at pH = 3.6. Said pH of 3.6 was therefore chosen as optimum for itaconic acid production (see **Fig. 19**). Further for all bioreactor yeast-like morphology was observed.

### Example 18: Metabolic engineering to enhance itaconic acid production in U. cynodontis NBRC 9727Δfuz7.

To be able to use in later studies hygromycin cassette, in the first step the hygromycin cassette was recycled in *U. cynodontis* NBRC 9727Δ*fuz7.*

To recycle the hygromycin cassette in *U. cynodontis* NBRC 9727Δ*fuz7* plasmid pFLExpC was transformed in *U. cynodontis* NBRC 9727Δ*fuz7* protoplasts. The plasmid contains an autonomic replication site for *Ustilago,* a carboxin resistance cassette and a flipase which is arabinose inducible which recognize the FRT-sites. After transformation, transformants which could grow on hygromycin and carboxin were chosen. One transformant was inoculate in YEPS medium containing 10 g L⁻¹ yeast extract, 10 g L⁻¹ peptone, 10 g L⁻¹ sucrose and 2 µg mL⁻¹ cbx at 30 °C, 200 rpm with a shaking diameter of 25 mm for 30 hours. After 30 hours preculture was washed three times with 0.9 % Nacl and in the last step pellet was re-suspend in 5 mL 0.9 % NaCl. Ten mL main culture containing 10 g L⁻¹ yeast extract, 10 g L⁻¹ peptone, 10 g L⁻¹ arabinose and 2 µg mL⁻¹ cbx were inoculate than with an OD₆₀₀ = 0,7 and were cultivate for 24 h, at 30°C, 200 rpm with a shaking diameter of 25 mm. Afterwards the culture was diluted 1:200 and 50 µL was spread on agar plates containing 10 g L⁻¹ yeast extract, 10 g L⁻¹ peptone and 10 g L⁻¹ sucrose. Afterwards with replica plating transformants were screened which lost their hygromycin and carboxin resistance. Further to be sure that hygromycin cassette was recycled, PCR analysis was performed. As primer pair HT-121 and HT-122 were used with Q5 as polymerase according to the manual (Q5® High-Fidelity DNA Polymerase / New England BioLabs, Frankfurt am Main). PCR conditions for the verification are shown in **Tab. 7.** Primer sequences are shown in **Tab. 11.** If hygromycin cassette was successfully recycled a band of 2199 bp was expected otherwise a band of 4943 bp was observed.

**Tab. 7: PCR conditions for verification of recycled hygromycin cassette.**

| | |
|---|---|
| 98°C | 30 seconds |
| 98°C | 10 seconds |
| 62°C | 30 seconds |
| 72°C | 2.4 min |
| 72°C | 2 min |

In the next step the gene *cyp3* was deleted in *U. cynodontis* NBRC 9727Δ*fuz7* like in *U. maydis* to delete the side product 2-hydroxyparaconate.
For deletion of *cyp3* in *U. cynodontis* NBRC 9727 Δ*fuz7* the FLP/FRT mediated recombination system was adapted from Khrunyk et al. 2010. In the first step plasmid had to be designed which contained the deletion constructs for *cyp3.* Afterwards, the deletion construct for cyp3 was integrated in the genomic locus of *U. cynodontis* NBRC 9727 Δ*fuz7* by homologous recombination, yielding a hygromycin resistant transformant. PCR was used to identify positive mutants, to allow identification of positive deletion, plus verification of the correct localization inside of the genome.
For the construction of the deletion construct for *cyp3* the flanking regions were amplified from *U. cynodontis* NBRC 9727 genome.
For flanking region 1 primer pair HT-228 and HT-229 and for flanking region 2 primer pair HT-232a and HT-233 were used, using Q5 as polymerase according to the manual (Q5® High-Fidelity DNA Polymerase / New England BioLabs, Frankfurt am Main). Both resulted in a PCR fragment of 1030 bp. PCR conditions for the flanking regions are shown in **Tab. 8.** Primer sequences are shown in **Tab. 11.**

**Tab. 8: PCR conditions for flanking regions 1 and 2 of cyp3.**

| | |
|---|---|
| 98°C | 30 seconds |
| 98°C | 5 seconds |
| 64°C | 5 seconds |
| 72°C | 30 seconds |
| 72°C | 60 seconds |

Further the hygromycin cassette including FRT-sites was amplified from pStorl_1rh_pTOa1523. For PCR primer pair HT-230 and HT-231a were used to amplify the hygromycin cassette including DRT-sites using Q5 as polymerase according to the manual (Q5® High-Fidelity DNA Polymerase / New England BioLabs, Frankfurt am Main), which resulted in a band of 2808 bp. PCR conditions for the hygromycin cassette are shown in **Tab. 9.** Primer sequences are shown in **Tab. 11.**

**Tab. 9: PCR conditions for amplification of the hygromycin cassette from pStorl_1rh_pTOa1523.**

| | |
|---|---|
| 98°C | 30 seconds |
| 98°C | 5 seconds |
| 60°C | 5 seconds |
| 72°C | 90 seconds |
| 72°C | 60 seconds |

Afterwards all PCR products were purified and assembled via Gibson cloning, together with pJET1.2 blunt as backbone (Gibson Asembly® Master Mix from New Wngland Biolabs (NEB) and the NEBuilder® Assembly Tool were used according to the manual. Afterwards the plasmid (**Fig. 20**) was sub-cloned into *E. coli.*
To verify that the deletion construct for the genes were assembled right, PCR analyses was used using Q5 polymerase according to the manual (Q5® High-Fidelity DNA Polymerase / New England BioLabs, Frankfurt am Main) with primer pair HT-234 and HT-235, which resulted in a band of 4732 bp. PCR conditions are shown in **Tab. 10.** Primer sequences are shown in **Tab. 11.**

**Tab. 10: PCR conditions for verification of the deletion constructs for ras2, fuz7 and ubc3.**

| | |
|---|---|
| 98°C | 30 seconds |
| 98°C | 5 seconds |
| 64°C | 5 seconds |
| 72°C | 150 seconds |
| 72°C | 60 seconds |

**Tab. 11: Necessary primer sequences to assemble Deletion constructs of cyp3 in U. cynodontis Δfuz7.**

| | |
|---|---|
| HT-228 | ctcgagtttttcagcaagatTTACAATTCTTTGCCTCTC (SEQ ID NO. 59) |
| HT-229 | acttctggccCTCGCTATACTTGCAAATG (SEQ ID NO. 60) |
| HT-232a | acttcccgggGAACATGTTTCAGAATGCTGC (SEQ ID NO. 61) |
| HT-233 | aggagatcttctagaaagatTCGGGGATACCATCACGAG (SEQ ID NO. 62) |
| HT-230 | gtatagcgagGGCCAGAAGTTCCTATTC (SEQ ID NO. 63) |
| HT-231a | aaacatgttcGGCCAGAAGTTCCTATAC (SEQ ID NO. 64) |
| HT-234 | TGATCTTCTGCGAGCCGAAC (SEQ ID NO. 65) |
| HT-235 | GCTTGGAGGAAGCCGATCTG (SEQ ID NO. 66) |
| HT-236 | TTTGCCTGCCGTCAACACTG (SEQ ID NO. 67) |
| HT-237 | TTCGCCCACACGATGATCGG (SEQ ID NO. 68) |

After verification deletion construct was amplified with primer pair HT-234 and HT-235 from pJET1.2FRT-KO-cyp3 to obtain linearized DNA for transformation. As polymerase Q5 was used according to the manual (Q5® High-Fidelity DNA Polymerase / New England BioLabs, Frankfurt am Main). PCR conditions for the verification are shown in **Tab. 12.** Primer sequences are shown in **Tab. 11.**

**Tab. 12: PCR conditions for amplification of deletion constructs for cyp3.**

| | |
|---|---|
| 98°C | 30 seconds |
| 98°C | 5 seconds |
| 64°C | 5 seconds |
| 72°C | 150 seconds |
| 72°C | 60 seconds |

The final linear DNA fragment was transformed into *U. cynodontis* NBRC 9727Δ*fuz7* according to **Example 4.** Cells which could grow on hygromycin containing REG-Agar plates were picked and further analyzed by PCR with primer pair HT-236 and HT-237, which resulted in a band of 4871. As negative control wildtype was used, which resulted in a band at 4041 bp. As polymerase Q5 was used according to the manual (Q5® High-Fidelity DNA Polymerase / New England BioLabs, Frankfurt am Main). PCR conditions for the verification are shown in **Tab. 13.** Primer sequences are shown in **Tab. 11.**

**Tab. 13: PCR conditions for verification of deletion of cyp3 U. cynodontis Δfuz7.**

| | |
|---|---|
| 98°C | 30 seconds |
| 98°C | 5 seconds |
| 70°C | 5 seconds |
| 72°C | 150 seconds |
| 72°C | 60 seconds |

The next step was to recycle the hygromycin cassette in *U. cynodontis* NBRC 9727Δ*fuz7*Δ*cyp3*^{HR}*.* For that plasmid pFLExpC was transformed in *U. cynodontis* NBRC 9727Δ*fuz7*Δ*cyp3*^{HR} protoplasts. The plasmid contains an autonomic replication site for *Ustilago,* a carboxin resistance cassette and a flipase which is arabinose inducible which recognize the FRT-sites. After transformation, transformants which could grow on hygromycin and carboxin were chosen. One transformant was inoculate in YEPS medium 10 g L⁻¹ yeast extract, 10 g L⁻¹ peptone, 10 g L⁻¹ sucrose and 2 µg mL⁻¹ cbx at 30 °C, 200 rpm with a shaking diameter of 25 mm for 30 hours. After 30 hours preculture was washed three times with Nacl and in the last step pellet was resuspend in 5 mL NaCl. Ten mL main culture containing 10 g L⁻¹ yeast extract, 10 g L⁻¹ peptone, 10 g L⁻¹ arabinose and 2 µg mL⁻¹ cbx were inoculate than with an OD600 = 0,7 and were cultivate for 24 h, at 30°C, 200 rpm with a shaking diameter of 25 mm. Afterwards the culture was diluted 1:200 and 50 µL was spread on agar plates containing 10 g L⁻¹ yeast extract, 10 g L⁻¹ peptone and 10 g L⁻¹ sucrose. Afterwards with replica plating transformants were screened on agar plates containing containing 10 g L⁻¹ yeast extract, 10 g L⁻¹ peptone, 10 g L⁻¹ sucrose and 2 µg mL-1 cbx, 200 µg mL-1 hygromycin respectively, which lost their hygromycin and carboxin resistance. Further to be sure that hygromycin cassette was recycled, PCR analysis was performed. As primer pair HT-236 and HT-237 were used with Q5 as polymerase according to the manual (Q5® High-Fidelity DNA Polymerase / New England BioLabs, Frankfurt am Main). If hygromycin cassette was successfully recycled a band of 2127 bp was expected otherwise a band of 4871 bp was demonstrated. PCR conditions are shown in **Tab. 14.** Primer sequences are shown in **Tab. 11.**

**Tab.14: PCR conditions for verification of recycled hygromycin cassette.**

| | |
|---|---|
| 98°C | 30 seconds |
| 98°C | 5 seconds |
| 70°C | 5 seconds |
| 72°C | 150 seconds |
| 72°C | 60 seconds |

The next step was to overexpress the regulator UcN_ria1 in *U. cynodontis* NBRC *9727Δfuz7Δcyp3* like in *U. maydis* to enhance the titer of itaconic acid. For that plasmid pNATIV_ria1_UcN was used, which contain the native regulator ria1 including the native terminator and promoter. Further the plasmid contains a cbx resistance cassette for later selection. The plasmid was linearized with *SsPI* and transformed in *U. cynodontis* NBRC *9727Δfuz7Δcyp3* protoplasts and selected later on YEPS agar plates containing 10 g L⁻¹ yeast extract, 10g L⁻¹ peptone, 10 g L⁻¹ sucrose and 2 µg mL⁻¹ cbx. Transformants which could grow und cbx, were selected and used for later cultivation studies (*U. cynodontis* NBRC 9727Δ*fuz7*Δ*cyp3*↑*ria1*)*.* In the last step the mitochondrial transporter *mttA* from *A. terreus* was expressed in *U. cynodontis* NBRC 9727Δ*fuz7*Δ*cyp3*↑*ria1.* For that plasmid pETEF_hyg_A_ter_mttA was used, which contain the gene *mttA* which is controlled by the constitutive promoter pETEF and the terminator Tnos. The plasmid was linearized with *XmNI* and transformed in *U. cynodontis* NBRC 9727Δ*fuz7*Δ*cyp3*↑*ria1* protoplasts and selected later on YEPS agar plates containing 10 g L⁻¹ yeast extract, 10g L⁻¹ peptone, 10 g L⁻¹ sucrose and 200 µg mL⁻¹ hyg. Transformants which could grow on hyg, were selected and used for later cultivation studies (*U. cynodontis* NBRC 9727Δ*fuz7*Δ*cyp3*↑*ria1*↑*mttA*)*.*
Yield for itaconate and itaconate titer are represented in **Fig. 21****.** The cultivation was performed in production medium C and D as triplicates and had a total duration of 120 h. In summary, every single step by metabolic engineering leads to an increase of itaconic acid. As expected, deletion of *cyp3* abolished the production of 2-hydroyparaconate as a by-product. Deletion of *fuz7* alone leads to a significant increase for itaconic acid production. Further, overexpression of the regulator and the mitochondrial transporter resulted in a further increasing amount for itaconic acid. Further, overexpression strains that contain the *fuz7* deletion showed no filamentous growth for the used conditions.

### Example 18: Metabolic engineering to enhance itaconic acid production in U. maydis MB215.

The metabolism for itaconic acid production is well studied in *U. maydis* MB215 and different strategies of Geiser et al. 2016 (doi: 10.1111/1751-7915.12329) show how itaconic acid can be increased. In this study this knowledge was used for a new approach, where either CRISPR/Cas or FLP/FRT technology were applied for deletions.

2-hydroxyparaconate is a side product in the metabolism for itaconic acid and it is known that by deletion of the gene *cyp3* this product also is abolished. For deletion of *cyp3* CRISPR/Cas was applied. For that plasmid pHMS8_*Um*_cyp3 and pJET1.2_cyp3-F were used for transformation. Transformation of the plasmids was carried out via protoplasts accordance to Example 4. To verify deletion of *cyp3,* colony PCR was performed. For PCR, primer pair JB-9 and JB-64 were used, which resulted in a band of 1242 bp. Additionally, polymerase Q5 was used according to the manual (Q5® High-Fidelity DNA Polymerase / New England BioLabs, Frankfurt am Main). PCR conditions for the verification are shown in **Tab. 15.** Primer sequences are shown in **Tab. 18.**

**Tab. 15: PCR conditions for verification of deletion of cyp3.**

| | |
|---|---|
| 98°C | 30 seconds |
| 98°C | 10 seconds |
| 64°C | 30 seconds |
| 72°C | 1.2 min |
| 72°C | 2 min |

After successful deletion of *cyp3* the next step was to overexpress the regulator *ria1* in *U. maydis* MB215Δ*cyp3*. Geiser et al. showed that overexpression of the regulator increases significant the production of itaconic acid. To overexpress the regulator CRISPR/Cas was used to change the native promoter by the strong and constitutive promoter pETEF. As a plasmid pHMS8_*Um*_pOMA and pJET1.2_pETEF-F were used. Transformation of the plasmids was carried out via protoplasts accordance to **Example 4.** To verify the replacement, PCR was performed with HT-153 and HT-186, which resulted in a band of 1945 bp. Further sequencing was prepared to verify successful replacement. Addtionally, polymerase Q5 was used according to the manual (Q5® High-Fidelity DNA Polymerase / New England BioLabs, Frankfurt am Main). PCR conditions for the verification are shown in **Tab. 16.** Primer sequences are shown in **Tab. 18.** After successful promoter replacement in *U. maydis* MB215Δ*cyp3* deletion of *fuz7* was the next step in *U. maydis* MB215Δ*cyp3*↑*ria1* to prevent filamentous growth.

**Tab. 16: PCR conditions for promoter replacement.**

| | |
|---|---|
| 98°C | 30 seconds |
| 98°C | 10 seconds |
| 64°C | 30 seconds |
| 72°C | 1 min |
| 72°C | 2 min |

For deletion of *fuz7* in *U. maydis* the FLP/FRT mediated recombination system was adapted from Khrunyk et al. 2010. In the first step plasmid had to be designed which contained the deletion construct for *fuz7.* Afterwards, the deletion construct for *fuz7* was integrated in the genomic locus of *U. maydis* MB215 by homologous recombination, yielding a hygromycin resistant transformant. PCR was used to identify positive mutants, to allow identification of positive deletion, plus verification of the correct localization inside of the genome.
For the construction of the deletion construct the flanking regions were amplified from *U. maydis* MB215 genome using primer pair HT-204 and HT-205 for flanking region 1 and primer pair HT-208 and HT-209 for flanking region 2, with Q5 as polymerase according to the manual (Q5® High-Fidelity DNA Polymerase / New England BioLabs, Frankfurt am Main). Both resulted in a PCR fragment of 1030 bp. PCR conditions are shown in **Tab. 17.** Primer sequences are shown in **Tab. 18.**

**Tab. 17: PCR conditions for flanking regions 1 and 2 of Um_fuz7.**

| | |
|---|---|
| 98°C | 30 seconds |
| 98°C | 5 seconds |
| 64°C | 5 seconds |
| 72°C | 30 seconds |
| 72°C | 60 seconds |

**Tab. 18: Primer sequences for metabolic / morphologic engineering in U. maydis MB215.**

| | |
|---|---|
| HT-204 | ctcgagtttttcagcaagatCCGATCGCTGTTAGGACAC (SEQ ID NO: 69) |
| HT-205 | acttctggccCGTGAAACGTTGCAAAACAG (SEQ ID NO: 70) |
| HT-208 | acttctggccCCGACTGAGAGATTATGGTC (SEQ ID NO: 71) |
| HT-209 | aggagatcttctagaaagatAATCGGAACCGTGTACCTG (SEQ ID NO: 72) |
| HT-206 | acgtttcacgGGCCAGAAGTTCCTATTC (SEQ ID NO: 73) |
| HT-207 | tctcagtcggGGCCAGAAGTTCCTATAC (SEQ ID NO: 74) |
| HT-210 | TCGCTGTTAGGACACAACTG (SEQ ID NO: 75) |
| HT-211 | CCGTGTACCTGGCTGTGTAG (SEQ ID NO: 76) |
| HT-210a | TCGGTGTGCGGCGATTTCTG (SEQ ID NO: 77) |
| HT-4a | ACAGACGTCGCGGTGAGTTC (SEQ ID NO: 78) |
| JB-9 | CTTCAAGGCCACCACAAC (SEQ ID NO: 79) |
| JB-64 | TGCTGCCATGGTGCTACTCC (SEQ ID NO: 80) |

Further the hygromycin cassette including FRT-sites were amplified from pStorl_1rh_pTOa1522 using primer pair HT-206 and HT-207 with Q5 as polymerase according to the manual (Q5® High-Fidelity DNA Polymerase / New England BioLabs, Frankfurt am Main), which resulted in a band of 2808 bp. PCR conditions are shown in **Tab. 19.** Primer sequences are shown in **Tab. 18.**

**Tab.19: PCR conditions for amplification of the hygromycin cassette from pStorl_1rh_pTOa1522.**

| | |
|---|---|
| 98°C | 30 seconds |
| 98°C | 5 seconds |
| 60°C | 5 seconds |
| 72°C | 120 seconds |
| 72°C | 60 seconds |

Afterwards all PCR products were purified and assembled via Gibson cloning, together with pJET1.2 blunt as backbone (Gibson Asembly® Master Mix from New Wngland Biolabs (NEB) and the NEBuilder® Assembly Tool were used according to the manual. Afterwards the plasmid (**Fig. 22**) was sub-cloned into *E. coli.*
To verify that the deletion construct for the genes was perfectly assembled, PCR analyses was used using primer pair HT-210 and HT-211 with Q5 polymerase (Q5® High-Fidelity DNA Polymerase / New England BioLabs, Frankfurt am Main), which resulted in a band of 4776 bp. PCR conditions for the verification are shown in **Tab. 20.** Primer sequences are shown in **Tab. 18.**
After verification the deletion construct was amplified with primer pair HT-210 and HT-211 from pJET1.2FRT-KO-*fuz7* to obtain linearized DNA for transformation. As polymerase Q5 was used according to the manual (Q5® High-Fidelity DNA Polymerase / New England BioLabs, Frankfurt am Main). PCR conditions for the verification are shown in **Tab. 20.** Primer sequences are shown in **Tab. 18.**

**Tab. 20: PCR conditions for verification of the deletion constructs for Um_fuz7.**

| | |
|---|---|
| 98°C | 30 seconds |
| 98°C | 5 seconds |
| 64°C | 10 seconds |
| 72°C | 144 seconds |
| 72°C | 2 min |

The final linear DNA fragment was transformed into *U. maydis* MB215Δ*cyp3*+↑ria1 according to **Example 4.** Cells which are able to grow on hygromycin containing REG-agar plates were picked and further analyzed by colony PCR with primer pair HT-210a and HT-4a, which resulted in a band of 2048 bp. As negative control wildtype was used, which resulted in no band. Additionally, polymerase Q5 was used according to the manual (Q5® High-Fidelity DNA Polymerase / New England BioLabs, Frankfurt am Main). PCR conditions for the verification are shown in **Tab. 21.** Primer sequences are shown in **Tab. 18.**

**Tab. 21: PCR conditions for verification of deletion for fuz7 in U. maydis MB215Δcyp3↑ria1.**

| | |
|---|---|
| 98°C | 30 seconds |
| 98°C | 10 seconds |
| 62°C | 30 seconds |
| 72°C | 60 seconds |
| 72°C | 2 min |

In the last step the mitochondrial transporter (*mttA*) from *A. terreus* was expressed in *U. maydis* MB215Δ*cyp3*↑*ria1*Δ*fuz7,* because previous studies proved that it is more efficient for the transport of cis-aconitate. For expression plasmid Petef_cbx*_A_ter_mttA* was used, which contains a codon optimized version of *mttA* for Ustilago which is controlled by a constitutive promoter (Petef). Further, the plasmid contains a cbx resistance cassette for later selection. The plasmid was linearized with *SspI* and transformed in *U. maydis* MB215Δ*cyp3*↑*ria1*Δ*fuz7* protoplasts and selected later on YEPS agar plates containing 10 g L⁻¹ yeast extract, 10g L⁻¹ peptone, 10 g L⁻¹ sucrose and 2 µg mL⁻¹ cbx. Transformants which are able to grow on cbx, were selected and used for later cultivation studies (*U. maydis* MB215Δ*cyp3*↑*ria1*Δ*fuz7*↑*mttA*).
Afterwards all engineered strains and control strains were analyzed in cultivation studies. The first cultivation was performed in production medium J (see **Example 11**) as triplicates and had a total duration of 96 h. As depicted in **Fig. 23** the new designed strain *U. maydis* MB215 Δcyp3↑*ria1* reached the same end titer of approximately 19 g L⁻¹ in comparison to the published strain of Geiser et al. (*U. maydis* Δ*cyp3*P_{etef}*ria1*). Deletion of *fuz7* in *U. maydis* MB215 Δcyp3↑*ria1* resulted in an increase of itaconate up to 23.6 g L⁻¹ itaconate. Also expression of *mttA* resulted in an increase of itaconate up to 28.4 g L⁻¹. Like it is in *U. cynodontis,* deletion of *fuz7* stops the filamentous growth behavior in *U. maydis* and further stops cell wall growing in well plates (**Fig. 24**) and shaking flasks.
Second cultivation was performed in production medium I (see **Example 11**) as triplicates and had a total duration of 168h, where glycerol was the carbon source. The deletion of *fuz7* resulted in an increase of itaconic acid production by three-fold (**Fig. 25**). Again, *fuz7* deletion shows no filamentous growth behavior and no cell wall growing (**Fig. 26**).

## Claims

1. A method of producing a secreted, non-proteinaceous biomolecule, comprising
(a) culturing a fungal host cell belonging to the division of Basidiomycota in culture medium under conditions allowing the production of said biomolecule, wherein the fungal host cell is due to genetic engineering not capable to switch from budding growth to filamentous growth in its vegetative stage during said culturing; and
(b) obtaining the biomolecule produced by the fungal host cell from the supernatant of said culture medium.

2. The method of claim 1, wherein the biomolecule is an organic acid, a polyol, or a glycolipid.

3. The method of claim 2, wherein the organic acid is itaconic acid, malic acid, hydroxyparaconic acid, citramalic acid, aconitic acid or itatararic acid.

4. The method of claim 2, wherein the polyol is erythritol or mannitol.

5. The method of claim 2, wherein the glycolipid is ustilagic acid or mannosylerythritol lipid.

6. The method of any one of the preceding claims, wherein the fungal host cell belonging to the division of Basidiomycota is from the family of Ustilaginaceae, the family of Dacrymycetaceae, or from the family of Moniliellaceae.

7. The method of claim 6, wherein the fungal host cell from the family of Ustilaginaceae is from the genus *Ustilago, Pseudozyma, or Sporisorium.*

8. The method of claim 7, wherein the fungal host cell from the genus *Ustilago* is *Ustilago maydis, Ustilago cynodontis, Ustilago trichophora, Ustilago vetiveriae* or *Ustilago xerochloae.*

9. The method of claim 6, wherein the fungal host cell from the family of Dacrymycetaceae is from the genus *Dacryopinax, Dacrymyces, Ditiola, Guepiniopsis* or *Femsjonia.*

10. The method of claim 9, wherein the fungal host cell from the genus
a) *Dacryopinax* is *Dacryopinax spathularia* or *Dacryopinax primogenitus*
b) *Dacrymyces is Dacrymyces stillatus* or *Dacrymyces chrysocomus;*
c) *Guepiniopsis* is *Guepiniopsis buccina;*
d) *Femsjonia* is *Femsjonia luteo-alba.*

11. The method of any one of the preceding claims, wherein the fungal host cell which is no longer capable of switching from budding growth to filamentous growth in its vegetative stage during said culturing is genetically engineered by knocking-out one or more polynucleotide sequence(s) encoding a polypeptide being involved in a signal transduction pathway which regulates the switching from budding growth to filamentous growth of said fungus.

12. The method of claim 11, wherein the one or more polynucleotide sequence(s) encoding a polypeptide is
a) a polynucleotide sequence encoding a polypeptide having an amino acid sequence having at least 25% sequence identity to the amino acid sequence of SEQ ID NO. 1 (fuz7 von *U. maydis),* said polynucleotide sequence encoding a polypeptide or fragment thereof having pheromone-responsive MAP kinase kinase activity,
b) a polynucleotide sequence encoding a polypeptide having an amino acid sequence having at least 25% sequence identity to the amino acid sequence of SEQ ID NO. 2 (ras2 von *U. maydis),* said polynucleotide sequence encoding a polypeptide or fragment thereof having pheromone-responsive MAP kinase pathway-interacting activity,
c) a polynucleotide sequence encoding a polypeptide having an amino acid sequence having at least 25% sequence identity to the amino acid sequence of SEQ ID NO. 3 (ubc3 von *U. maydis),* said polynucleotide sequence encoding a polypeptide or fragment thereof having pheromone-responsive MAP kinase activity,
d) a polynucleotide sequence encoding a polypeptide having an amino acid sequence having at least 25% sequence identity to the amino acid sequence of SEQ ID NO. 4 (ubc1 von *U. maydis),* said polynucleotide sequence encoding a polypeptide or fragment thereof having cAMP-dependent protein kinase regulator activity,
e) a polynucleotide sequence encoding a polypeptide having an amino acid sequence having at least 25% sequence identity to the amino acid sequence of SEQ ID NO. 5 (ubc2 von *U. maydis),* said polynucleotide sequence encoding a polypeptide or fragment thereof having pheromone-responsive MAP kinase pathway-interacting activity,
f) a polynucleotide sequence encoding a polypeptide having an amino acid sequence having at least 25% sequence identity to the amino acid sequence of SEQ ID NO. 6 (ubc4 von *U. maydis),* said polynucleotide sequence encoding a polypeptide or fragment thereof having pheromone-responsive MAPKK kinase activity, or
g) a polynucleotide sequence encoding a polypeptide having an amino acid sequence having at least 25% sequence identity to the amino acid sequence of SEQ ID NO. 7 (prf1 von *U. maydis),* said polynucleotide sequence encoding a polypeptide or fragment thereof having pheromone-responsive transcription factor activity.

13. The method of claim 12, wherein the one or more polynucleotide sequence(s) encoding a polypeptide is
a) a polynucleotide sequence encoding a polypeptide having an amino acid sequence having at least 25% sequence identity to the amino acid sequence of SEQ ID NO. 1 (fuz7 von *U. maydis),* said polynucleotide sequence encoding a polypeptide or fragment thereof having pheromone-responsive MAP kinase kinase activity,
b) a polynucleotide sequence encoding a polypeptide having an amino acid sequence having at least 25% sequence identity to the amino acid sequence of SEQ ID NO. 2 (ras2 von *U. maydis),* said polynucleotide sequence encoding a polypeptide or fragment thereof having pheromone-responsive MAP kinase pathway-interacting activity, or
c) a polynucleotide sequence encoding a polypeptide having an amino acid sequence having at least 25% sequence identity to the amino acid sequence of SEQ ID NO. 3 (ubc3 von *U. maydis),* said polynucleotide sequence encoding a polypeptide or fragment thereof having pheromone-responsive MAP kinase activity.

14. The method of any one of the preceding claims, wherein the capability of switching from budding growth to filamentous growth is determined by the influence of sunflower oil, low pH, high glucose concentration or glycerol concentration during culturing of the fungal host cell.

15. Use of a fungal host cell belonging to the division of Basidiomycota for the production of a biomolecule, wherein the fungal host cell is due to genetic engineering not capable of switching from budding growth to filamentous growth in its vegetative stage.


SEQUENCE LISTING

<110>  RWTH Aachen

<120>  MORPHOLOGIC ENGINEERING IN BASIDIOMYCOTA

<130>  RWT16280EP

<160>  80

<170>  PatentIn version 3.5

<210>  1
<211>  435
<212>  PRT
<213>  Ustilago maydis

<400>  1

Met Leu Ser Ser Gly Ala Gly Ser Ser Ile Arg Lys Lys Arg Asn Phe
1               5                   10                  15

Lys Gly Leu Gln Leu Ala Glu Ser Pro Leu Ala Ser Pro Val Asp Ala
            20                  25                  30

Ser Ala Thr Thr Pro Ser His Lys Pro Gly Glu Gly Ser Ala Ala Ser
        35                  40                  45

Asn Ala Ser Thr Ile Gly Lys Ser Ser Ala Val Thr Pro Gly Gly Ser
    50                  55                  60

Leu Ala Leu Pro Val Lys Asn Gly Leu Asp Thr Glu Pro Asn Ser Gly
65                  70                  75                  80

Ala Asn Tyr His Asn Lys Leu Thr Gln Gln Leu Ala Asn Leu Glu Leu
                85                  90                  95

Gly Val Glu Tyr Lys Leu Asp Leu Lys Asn Glu Asp Leu Lys Thr Leu
            100                 105                 110

Ser Glu Leu Gly Ala Gly Asn Gly Gly Thr Val Thr Lys Val Leu His
        115                 120                 125

Glu Lys Ser Gly Thr Val Met Ala Lys Lys Val Val Phe Ile Asp Ala
    130                 135                 140

Lys Pro Ser Val Arg Lys Gln Ile Leu Arg Glu Leu Gln Ile Leu His
145                 150                 155                 160

Glu Cys Asn Ser Pro Tyr Ile Val Ser Phe Tyr Gly Ala Tyr Leu Asn
                165                 170                 175

Glu Pro His Ile Cys Met Cys Met Glu Phe Met Gln Lys Asp Ser Leu
            180                 185                 190

Asp Gly Ile Tyr Lys Lys Tyr Gly Pro Ile Ser Pro Glu Ile Cys Gly
        195                 200                 205

Lys Ile Ala Val Ala Val Ser His Gly Leu Thr Tyr Leu Tyr Asp Val
    210                 215                 220

His Arg Ile Ile His Arg Asp Val Lys Pro Ser Asn Ile Leu Val Asn
225                 230                 235                 240

Gly Ala Gly Gln Ile Lys Ile Cys Asp Phe Gly Val Ser Gly Glu Leu
                245                 250                 255

Ile Asn Ser Ile Ala Asp Thr Phe Val Gly Thr Ser Thr Tyr Met Ser
            260                 265                 270

Pro Glu Arg Ile Gln Gly Asp Gln Tyr Ser Val Lys Ser Asp Val Trp
        275                 280                 285

Ser Leu Gly Val Ser Ile Ile Glu Leu Ala Leu Gly Arg Phe Pro Phe
    290                 295                 300

Ala Glu Asn Glu Glu Asp Asp Asp Ser Asp Ala Asp Asn Asn Tyr Thr
305                 310                 315                 320

Asn Glu Asp Leu Ala Gly Thr Leu Ser Pro Thr Lys Pro Ala Pro Met
                325                 330                 335

Ile Ser Leu Gly Gln Asn Glu Lys Gln Arg Arg Arg Lys Ser Lys Pro
            340                 345                 350

Ala Gly Val Ser Leu Glu Gly Ser Ser His Gln Met Ser Ile Leu Asp
        355                 360                 365

Leu Leu Gln His Ile Val Asn Glu Pro Pro Pro Lys Leu Pro Glu Gly
    370                 375                 380

Arg Phe Pro Lys His Met Glu Glu Phe Val Asn Leu Cys Leu Leu Lys
385                 390                 395                 400

Asp Pro Ala Lys Arg Pro Thr Pro Lys Asp Leu Thr Lys His Gln Tyr
                405                 410                 415

Val Ile Asp Ala Asp Ala Ala Lys Val Asp Leu Gln Ala Trp Ala Asp
            420                 425                 430

Gly Met Lys
        435

<210>  2
<211>  192
<212>  PRT
<213>  Ustilago maydis

<400>  2

Met Ser Gly Lys Met Met Ile Tyr Lys Leu Val Val Leu Gly Asp Gly
1               5                   10                  15

Gly Val Gly Lys Thr Ala Leu Thr Ile Gln Leu Cys Leu Asn His Phe
            20                  25                  30

Val Glu Thr Tyr Asp Pro Thr Ile Glu Asp Ser Tyr Arg Lys Gln Thr
        35                  40                  45

Val Ile Asp Asp Gln Pro Cys Met Leu Glu Val Leu Asp Thr Ala Gly
    50                  55                  60

Gln Glu Glu Tyr Thr Ala Leu Arg Asp Gln Trp Ile Arg Glu Gly Glu
65                  70                  75                  80

Gly Phe Leu Leu Val Tyr Ser Ile Ser Ala Arg Ala Thr Phe Glu Arg
                85                  90                  95

Val Glu Arg Phe Arg Ser Gln Ile Ser Arg Val Lys Asp Gln Glu Pro
            100                 105                 110

His Thr Val Pro Ile Met Leu Val Gly Asn Lys Cys Asp Lys Val Asn
        115                 120                 125

Glu Arg Glu Val Ser Arg Glu Glu Gly Gln Ala Leu Ala His Arg Leu
    130                 135                 140

Gly Cys Lys Phe Ile Glu Ser Ser Ala Lys Thr Cys Val Asn Val Glu
145                 150                 155                 160

Arg Ala Tyr Tyr Thr Val Val Arg Met Ile Arg Glu Gln Arg Glu Gly
                165                 170                 175

Thr Val Thr His Lys Lys Glu Lys Lys Lys Ser Lys Cys Asn Ile Leu
            180                 185                 190

<210>  3
<211>  354
<212>  PRT

<213>  Ustilago maydis

<400>  3

Met Ser His Ala His Gly Gln Gln Pro Asn His Ser Thr Thr Phe Lys
1               5                   10                  15

Val Gly Glu Thr Tyr Lys Val Val Asp Val Val Gly Glu Gly Ala Tyr
            20                  25                  30

Gly Val Val Cys Ser Ala Ile His Val Pro Ser Ser Ser Arg Val Ala
        35                  40                  45

Ile Lys Lys Ile Thr Pro Phe Asp His Ser Met Phe Cys Leu Arg Thr
    50                  55                  60

Leu Arg Glu Ile Lys Leu Leu Arg His Phe Asn His Glu Asn Ile Ile
65                  70                  75                  80

Ser Ile Leu Asp Ile Val Lys Pro Asp Asp Tyr Asp Ser Phe Ser Glu
                85                  90                  95

Val Tyr Leu Ile Gln Glu Leu Met Glu Thr Asp Met His Arg Val Ile
            100                 105                 110

Arg Thr Gln Glu Leu Ser Asp Asp His Cys Gln Tyr Phe Ile Tyr Gln
        115                 120                 125

Thr Leu Arg Gly Leu Lys Ala Leu His Ser Ala Gln Val Leu His Arg
    130                 135                 140

Asp Leu Lys Pro Ser Asn Leu Leu Leu Asn Ala Asn Cys Asp Leu Lys
145                 150                 155                 160

Ile Cys Asp Phe Gly Leu Ala Arg Ser Ala Asn Gln Pro Glu Ala Glu
                165                 170                 175

Gly Thr Gly Phe Met Thr Glu Tyr Val Ala Thr Arg Trp Tyr Arg Ala
            180                 185                 190

Pro Glu Ile Met Leu Thr Phe Lys Glu Tyr Thr Lys Ala Ile Asp Val
        195                 200                 205

Trp Ser Val Gly Cys Ile Leu Ala Glu Met Leu Ala Gly Lys Pro Leu
    210                 215                 220

Phe Pro Gly Arg Asp Tyr His His Gln Leu Ser Leu Thr Leu Glu Ile
225                 230                 235                 240

Leu Gly Thr Pro Ser Leu Asp Asp Phe Tyr Ala Ile Thr Ser Thr Arg
                245                 250                 255

Ser Arg Asp Tyr Ile Arg Ala Leu Pro Phe Arg Lys Arg Arg Asn Leu
            260                 265                 270

Ser Leu Met Phe Pro Asn Ala Asn Pro Leu Ala Val Asp Leu Met Glu
        275                 280                 285

Lys Cys Leu Thr Phe Ser Pro Arg Lys Arg Ile Thr Val Glu Glu Ala
    290                 295                 300

Leu Ala His Pro Tyr Leu Glu Pro Tyr His Asp Pro Glu Asp Glu Pro
305                 310                 315                 320

Thr Ala Glu Pro Leu Asp Pro Ser Phe Phe Asp Phe Asp Tyr Cys Lys
                325                 330                 335

Glu Gln Leu Ser Arg Ser Glu Leu Lys Arg Leu Ile Tyr Asn Glu Ile
            340                 345                 350

Met Arg

<210>  4
<211>  227
<212>  PRT
<213>  Ustilago maydis

<400>  4

Met Gly Asp Ala Arg Leu Arg Arg Ile Asn Lys Glu Ile Asn Asp Cys
1               5                   10                  15

Lys Asn Asp Lys Val Ser Gly Ile Gln Val Ser Leu Ile Asp Glu Ser
            20                  25                  30

Pro Phe His Leu Val Gly Thr Phe Pro Gly Pro Glu Asn Ser Pro Tyr
        35                  40                  45

Glu Lys Gly Thr Phe Asp Val Asp Ile Val Val Pro Glu Gly Tyr Pro
    50                  55                  60

Phe Gln Pro Ile Lys Met Lys Phe Ile Thr Arg Val Tyr His Pro Asn
65                  70                  75                  80

Ile Ser Ser Gln Ser Gly Ala Ile Cys Leu Asp Ile Leu Lys Asp Gln
                85                  90                  95

Trp Ser Pro Val Leu Thr Leu Lys Ser Thr Leu Met Ser Leu Arg Ser
        100                 105                 110

Leu Leu Cys Ser Pro Glu Pro Asn Asp Pro Gln Asp Ala Glu Val Ala
    115                 120                 125

Lys His Tyr Leu Arg Asp Lys Asn Asp Phe Glu Lys Thr Ala Arg Tyr
    130                 135                 140

Trp Thr Glu Ile Tyr Ala Asn Gly Ser Glu Ser Ala Gln Pro Pro Ala
145                 150                 155                 160

Asn Ala Gln Asn Thr Val Gly Lys Thr Gly Asp Arg Ala Asp Pro Ile
                165                 170                 175

Leu Leu Ala Gly Leu Lys Arg Glu His Val Asp Gln Phe Val Asn Met
        180                 185                 190

Gly Phe Glu Thr Asn Lys Val Ile Glu Ala Leu Lys Lys Leu Asn Tyr
    195                 200                 205

Arg Gly Ala Asn Val Arg Asn Ile Thr Asp Asp Thr Val Leu Asn Glu
    210                 215                 220

Leu Leu Lys
225

<210>  5
<211>  829
<212>  PRT
<213>  Ustilago maydis

<400>  5

Met Ser Ser Gln Ser Pro Lys Ser Ser Pro Thr Val His Val Asn Lys
1               5                   10                  15

Trp Ser Glu Gln Gln Val Val Asp Trp Leu Ser Ser Val Gly Leu Ser
            20                  25                  30

Lys Tyr Ala Arg Asp Phe Lys Ser Asn Gly Ile Thr Gly Asp Val Leu
        35                  40                  45

Val Leu Leu Asp Asp Glu Ala Leu Arg Asp Ile Gly Val Val Thr Ile
    50                  55                  60

Gly Gln Arg Leu Ala Leu Leu Ala Ala Ile Tyr Arg Leu Lys Gln Gln
65                  70                  75                  80

Phe Asp Ile Pro Ile Gln Glu Gly Asp Trp Ile Pro Lys Ser Ile Glu
                85                  90                  95

Ala Gln Glu His Ala Thr Asn Thr Leu Ser Thr Ala His Met Ser Thr
            100                 105                 110

Ala Leu Arg Gln Arg Asp Asp Arg Ile Arg Leu Leu Glu Ser Gln Ile
        115                 120                 125

Leu Arg Leu Ala Asp Tyr Leu Val Arg Phe Gln Gln Asp Met Ala Ser
    130                 135                 140

Val Ala Arg His Val Gly Val Lys Ala His Ser Ile Asp Thr Pro Ile
145                 150                 155                 160

Thr Leu Val Ser Asn Ser Leu Ser Ala His Arg Ser Ser Glu Ser Ser
                165                 170                 175

Ala Thr Thr Ser Ala Phe Gly Ser Asn Ser Asp Leu Ala Ser Ser Ala
            180                 185                 190

Ala Ser Ser Leu Pro Met Glu Ser Pro Thr Ser Arg Asn Phe Ala Gly
        195                 200                 205

Leu Val Ser Pro Thr Arg Thr Gly Phe Thr Tyr Gly Gly Ser Gln Ala
    210                 215                 220

Met Ser Gly Phe Lys Pro Thr Thr Val Lys Ser Ser Arg Ser Asn Ser
225                 230                 235                 240

Ile Gly Gly Ala Ala Pro Met Thr Pro Thr Thr Ala Ala His Met Tyr
                245                 250                 255

Ala Ala Ser Ala Ser Asn Thr Val Pro Thr Gly Asp Val Ser Pro Thr
            260                 265                 270

Gln Ile Thr Pro Ser Val Thr Pro Thr Ser Leu Gly Ser Gln Pro Ala
        275                 280                 285

Phe Pro Gln Gln Gln Pro Pro Ser Ser Ala Gly Ser Thr Ser Met Val
    290                 295                 300

Ser Pro Thr Arg Arg Leu Ala His Gln Asn Ser Leu Ala His Pro Asp
305                 310                 315                 320

Ala Ala Gly Ser Ala Arg Pro Arg Ala Gly Ser Val Gly Ala Ser Ala
                325                 330                 335

Ala Leu Ser Ser Thr Ser Gly Ser Ser Leu Phe Ser Asn Thr Gly Ser
            340                 345                 350

Ser Gly Ser Ile Asn Ala Thr Thr Val Ser Ala Ala Thr Glu Arg Asp
        355                 360                 365

Asp Arg Ile Lys Asp Ala Ala Ala Thr Thr Val Ser Glu Pro Lys Pro
    370                 375                 380

Lys Phe Ser Thr Val Ala Ala Ser Gly Ser Ser Leu Ser Ala Ser Pro
385                 390                 395                 400

Ala Ala Ser Ala Ala Ala Ala Thr Thr Ala Ser Ser Asp Ser Asn Pro
                405                 410                 415

Tyr Lys Ser Phe Arg Val Thr Leu Asp Asp Pro Cys Tyr Lys Val Leu
            420                 425                 430

Pro Ala Ala Leu Lys Lys Tyr Lys Ile Asn Asp Asp Trp Arg Lys Tyr
        435                 440                 445

Ala Leu Phe Ile Cys Tyr Gly Lys Thr Glu Arg Cys Leu Ser Tyr Asp
    450                 455                 460

Glu Lys Pro Leu Leu Leu Phe Gln Lys Leu Lys Glu Asn Glu Gln Ser
465                 470                 475                 480

Pro Val Phe Met Leu Arg His Ile Arg Asp Val Lys Ser Pro Ile Ala
                485                 490                 495

Ile Ala Glu Ala Lys Ala Ala Ser Lys Leu Gln Glu Arg Glu Thr Asp
            500                 505                 510

Gly Gly Ser Ser Ala Thr Lys Lys Arg Ser Ala Pro Arg Ala Asp Arg
        515                 520                 525

Arg Arg Val Ile Ala Gly Thr Pro Pro Pro Gly Ala Leu Val Thr Asn
    530                 535                 540

Ala Ser Asn Pro Val Glu Val Gly Pro Ala Ala Asp Leu Pro Glu Arg
545                 550                 555                 560

Asn Lys Val Ala Arg Thr Tyr Ala Val Ala Ile Tyr Pro Tyr Met Pro
                565                 570                 575

Glu Arg Asp Asp Glu Phe Asp Val Asn Val Gly Asp Thr Phe Val Val

            580                 585                 590

Ile Asn Lys Ala Lys Gly Trp Trp Ile Val Gln Arg Asp Ala Lys Gln
        595                 600                 605

Asp Gly Ala Gly Asp Val Val Tyr Ser Val Pro Ala Ser Glu Ala Ser
    610                 615                 620

Ser Ala Asp Asp Asp Gly Val Thr Tyr Ser Asn Tyr Arg Ala Glu His
625                 630                 635                 640

Ala Ser Gly Trp Val Pro Ala Gly Cys Leu Leu Glu Thr Ser Arg Pro
                645                 650                 655

Leu Gly Ser Ile Val Glu Ala Glu Ala Ala Ser Met Ser His Val Arg
            660                 665                 670

Ser Gly Ala Ser Thr Ile Ser Asn Pro Ser Thr Pro Thr Ala Ser Ser
        675                 680                 685

Ser Gly Ala Lys Leu Asp Ala Lys Arg Ala Ala Ile Pro Pro Ala Leu
    690                 695                 700

Ile Thr Ser Thr Ser Thr Pro Gly Ile Met Leu Met Asp Tyr Gln Ser
705                 710                 715                 720

Gly Glu Gly Asp Leu Asp Leu Arg Lys Asp Glu Arg Leu Arg Val Phe
                725                 730                 735

Lys Arg Tyr Asn His Trp Ser Tyr Cys Val Gln Glu Arg Asp Ala His
            740                 745                 750

Ala Arg Gly Trp Val Pro Ser Trp Tyr Ile Gly Lys Leu Ser Ser Ala
        755                 760                 765

Ala Ser Gly Ser Leu Gly Lys Ser Ala Ser Thr Thr Ala Leu Ser Ala
    770                 775                 780

Thr Pro Val Pro Asp Ser Arg Leu Met His Ala Ala Glu Ser Val Thr
785                 790                 795                 800

Ala Val Ala Gly Ser Pro Ser Tyr Asp Ser Ser Leu Thr His Asp His
                805                 810                 815

Pro Ser Ala His Ser Ala Ser Ala Pro Met Ala Thr Val
            820                 825

<210>  6
<211>  147
<212>  PRT
<213>  Ustilago maydis

<400>  6

Met Ala Thr Lys Arg Ile Asn Lys Glu Leu Val Asp Leu Gly Arg Asp
1               5                   10                  15

Pro Pro Ser Ser Cys Ser Ala Gly Pro Thr Gly Asp Asn Met Phe Gln
            20                  25                  30

Trp Gln Ala Thr Ile Met Gly Pro Gly Asp Ser Pro Tyr Ala Gly Gly
        35                  40                  45

Val Phe Phe Leu Ser Ile Thr Phe Pro Thr Asp Tyr Pro Phe Lys Pro
    50                  55                  60

Pro Lys Val Ser Phe Ser Thr Lys Ile Tyr His Pro Asn Ile Asn Ser
65                  70                  75                  80

Asn Gly Ser Ile Cys Leu Asp Ile Leu Arg Asp Gln Trp Ser Pro Ala
                85                  90                  95

Leu Thr Ile Ser Lys Val Leu Leu Ser Ile Cys Ser Met Leu Thr Asp
            100                 105                 110

Pro Asn Pro Asp Asp Pro Leu Val Pro Glu Ile Ala Asn Leu Tyr Lys
        115                 120                 125

Thr Asp Arg Gln Arg Tyr Glu Ser Thr Ala Arg Glu Trp Thr Arg Lys
    130                 135                 140

Tyr Ala Met
145

<210>  7
<211>  840
<212>  PRT
<213>  Ustilago maydis

<400>  7

Met Arg Asp Pro Gly Cys Ala Asp Ala Ile Ala Thr Arg Ser Gly Pro
1               5                   10                  15

Arg Asn Gln Ile Met Val Asp Leu Leu Arg Gly Trp Ser Thr Glu Ala
            20                  25                  30

Glu Ser Gly Ser Ser Ser Ser Thr Ala Leu Ser Gly Leu Asp Gly Ser

        35                  40                  45

Pro Ile Lys Arg Ser Ala Ser Gln Glu Ala Ser Ile Leu Ser Asn Gln
    50                  55                  60

Glu Thr Val Pro Leu Phe Gln Thr Ile Ser Leu Pro Asn Lys Asp Val
65                  70                  75                  80

Ser Leu Ala Val Ser Leu Tyr Thr Gly Cys Lys Ser Glu Gln Glu Met
                85                  90                  95

Asp Gln Glu Glu Leu Glu Arg Ile Lys Arg Leu Pro Pro Ser Lys Arg
            100                 105                 110

Pro Gly Val Ser His Ala Lys Arg Thr Pro Pro Asn His Ile Lys Arg
        115                 120                 125

Pro Arg Asn Ala Tyr Ile Ile Phe Arg Ser His Ile Val Ser Gln Lys
    130                 135                 140

Leu Ile Pro Lys Glu Val Glu Asn Asp His Arg Asn Ile Ser Arg Ile
145                 150                 155                 160

Ile Ala His Met Trp Lys Ser Leu Glu Pro Gln Glu Arg Ala Gln Tyr
                165                 170                 175

Glu Gln Ile Ala Lys Gln Glu Lys Glu Arg His Lys Gln Leu Phe Pro
            180                 185                 190

Glu Tyr Arg Tyr Arg Pro Thr Thr Arg Arg Thr Gly Val Ser Lys Arg
        195                 200                 205

Asn Val Lys Lys Leu Glu Asn Gly Glu Glu Glu Cys Gln Glu Ile Ala
    210                 215                 220

Asp Ile Ile Leu Lys Ala Gln Gly Lys Glu Gly Val Ile Val Arg Gly
225                 230                 235                 240

Gly Pro Ser Lys Ala Ile Lys Arg Ala Arg Lys Ala Thr Arg Ala Ser
                245                 250                 255

Val Ala Ala Arg Asn Cys Gly Lys Pro Ser Ser Pro Arg Ala Lys Arg
            260                 265                 270

Ala Arg Val Asp Lys Ser Lys Pro Asp Glu Pro Val Pro Pro Ser Gln
        275                 280                 285

Ala Ala Asp Glu Gly Leu Glu Thr Ile Phe Leu His Pro Ser Pro Ser
    290                 295                 300

Gln Gln Ser Pro Thr Leu Ser Pro Ser Tyr Ser Pro Glu Ala Glu Ser
305                 310                 315                 320

Val Pro Thr Pro Glu Pro Arg Pro Glu Ser Ser Arg Met Thr Ser Arg
                325                 330                 335

Thr Pro Ser Ile Asp Arg Gly Gln Pro Leu Pro Ser Ser Gly Ser Ala
            340                 345                 350

Leu Met Leu Asp Ile Ser Glu Met Phe Gly Arg Arg Ser Ser Ser Val
        355                 360                 365

Pro Val Met His Pro Cys Ser Pro Thr Leu Ala Phe Ile Pro Pro Thr
    370                 375                 380

Gln Glu Asn Thr Asp Ala Glu Thr Leu Gly Thr His Gln Asp Glu Leu
385                 390                 395                 400

Gly Thr His Val Ala Asp Ala Gly Asp Gly Tyr Thr Asn His Phe Ser
                405                 410                 415

Met Pro Ala Pro Arg Trp Lys Gly Arg Lys Thr Ile Pro Ala Pro Ile
            420                 425                 430

Ala Asn Thr Trp Gln Phe Cys Ser Tyr Asp Asp Gln Ser Leu Pro Ser
        435                 440                 445

Pro Gln Ser Phe Asp Thr Phe Ala Ser Ala Ser Lys Met Pro Ala Ala
    450                 455                 460

Arg Pro Arg Thr Ala Trp Pro Ser Thr Pro Ser Ser Ser Thr Phe Arg
465                 470                 475                 480

Gly Phe Phe His Pro Trp Ala Val Glu His Asn Asn Asp Ser Met Leu
                485                 490                 495

Ile Ser Pro Met Thr Ala Ser Phe Gln Asp Leu Arg Arg Arg Ser Ser
            500                 505                 510

Leu Ala Arg Ser Gly Leu Ser Ala Thr Arg Arg Pro Gly Ser Phe Gly
        515                 520                 525

Val Asp Val Gln Pro Asn Ser Ser Gly Arg Leu Glu Gly Asp Val Gly
    530                 535                 540

Thr Gln Ala Leu Leu Gln Pro Ser Ser Gly Ala Arg Leu Pro Asp Gly
545                 550                 555                 560

Gln Leu Phe Gly Glu Ala His Pro Ala Ala Ala Val His Leu Asn Ser
                565                 570                 575

Glu Ile Ala Ser Ala Asp Tyr Asp Glu Thr Pro Asn Phe Ala Phe Asp
            580                 585                 590

Pro Ala Leu Glu Gly Glu Gly Arg Val Pro Ala Ala Pro Asp Ser Val
        595                 600                 605

Ala Glu Gln Pro Cys His Ser Asp Arg Ser Leu Ala Ala Gln Val Met
    610                 615                 620

Ser Ala Ser Pro Arg Ser Gln Ala Gln Ser Pro Arg Pro Ser Phe Ser
625                 630                 635                 640

Gly Ser Thr Leu Ala Ala Ala Ala Arg Asp Trp Ala Ser Met Lys Arg
                645                 650                 655

Arg Arg Ser Lys Met Ala Gln Thr Pro Ile Val Asn Ala Val Ser Glu
            660                 665                 670

Ser Leu Pro Ala Pro Ile Asp Ser Ser Asp Ser Ile Glu Ala Ser Met
        675                 680                 685

Leu Arg Glu Thr Asp Lys Pro Gly Arg Leu Tyr Gly Ser Ser Leu Glu
    690                 695                 700

Glu Ser Val Glu Arg Ala Val Met Leu Ala Leu Ser Lys Asp His Gly
705                 710                 715                 720

Gln Asp Ser Asn Leu Gly Glu Arg Asn Ser Asn Ile Val Gln Gln Ile
                725                 730                 735

Leu Ser Trp Leu Ser Thr Glu Leu Ala Leu Gln Ser Gln Asn Gln Gln
            740                 745                 750

Glu His Gln Gln Phe Thr Pro Ser Ile Cys His Ala Asp Lys Ala Pro
        755                 760                 765

Leu Ser Phe Val Glu Ser Arg Pro Arg Ala Cys Ser Arg Ala Ser Arg
    770                 775                 780

Gly Val Ala Arg Pro Asn Gln Tyr Tyr Ala Ala Asn Ala Ala Ile Glu
785                 790                 795                 800

Gly Gly Ser Ser Arg Leu Gly Lys Pro Leu Pro Ser Pro Leu Gln Leu
                805                 810                 815

Val Thr Ser Asn Tyr Glu Ile Gly Thr Ser Thr Arg His Ser His Ser
            820                 825                 830

Tyr Ser Ser Pro Gln His Cys Ile
        835                 840

<210>  8
<211>  435
<212>  PRT
<213>  Ustilago cynodontis

<400>  8
   Met Leu Ser Ser Ser Ala Gly Ser Ser Ile Arg Lys Lys Arg Asn Phe
   1               5                   10                  15
   Lys Gly Leu Gln Leu Ala Glu Ser Pro Leu Ser Ser Pro Ala Ala Ala
               20                  25                  30
   Thr Thr Pro Ser Gln Asn Thr Gly Glu Gly Ser Ala Asn Ser Asn Ala
           35                  40                  45
   Ser Thr Ile Gly Lys Ser Ser Thr Val Thr Pro Gly Gly Ser Leu Ser
       50                  55                  60
   Leu Thr Ala Lys Asn Gly Leu Asp Thr Glu Pro Asn Ser Gly Ala Asn
   65                  70                  75                  80
   Tyr His Asn Lys Leu Thr Gln Gln Leu Ala Asn Leu Glu Leu Gly Val
                   85                  90                  95
   Glu Tyr Lys Leu Asp Leu Lys Asn Glu Asp Leu Lys Thr Leu Ser Glu
               100                 105                 110
   Leu Gly Ala Gly Asn Gly Gly Thr Val Thr Lys Val Leu His Glu Lys
           115                 120                 125
   Ser Gly Thr Val Met Ala Lys Lys Val Val Phe Ile Asp Ala Lys Pro
       130                 135                 140
   Ser Val Arg Lys Gln Ile Leu Arg Glu Leu Gln Ile Leu His Glu Cys
   145                 150                 155                 160
   Asn Ser Pro Tyr Ile Val Ser Phe Tyr Gly Ala Tyr Leu Ser Glu Pro
                   165                 170                 175
   His Ile Cys Met Cys Met Glu Phe Met Glu Lys Asp Ser Leu Asp Gly
   180 185 190
   Ile Tyr Lys Lys Tyr Gly Pro Ile Ala Pro Glu Ile Cys Gly Lys Ile
   195 200 205
   Ala Val Val Val Ala His Gly Leu Thr Tyr Leu Tyr Asp Val His Arg
   210 215 220
   Ile Ile His Arg Asp Val Lys Pro Ser Asn Ile Leu Val Asn Gly Ala
   225 230 235 240
   Gly Gln Ile Lys Ile Cys Asp Phe Gly Val Ser Gly Glu Leu Ile Asn
   245 250 255
   Ser Ile Ala Asp Thr Phe Val Gly Thr Ser Thr Tyr Met Ser Pro Glu
   260 265 270
   Arg Ile Gln Gly Asp Gln Tyr Ser Val Lys Ser Asp Val Trp Ser Leu
   275 280 285
   Gly Val Ser Ile Ile Glu Leu Ala Leu Gly Arg Phe Pro Phe Ala Glu
   290 295 300
   Asn Glu Asp Asp Phe Asp Ser Asp Asp Asp Asp Ala Ser Asp Leu Leu
   305 310 315 320
   Gly Asp Asp Leu Ser Gly Thr Leu Ser Pro Thr Lys Pro Ala Pro Lys
   325 330 335
   Leu His Val Ala Asn Lys Glu Lys Gln Asn Arg Arg Lys Ser Lys Ala
   340 345 350
   Ala Gly Val Ser Leu Glu Gly Ser Ser His Gln Met Ser Ile Leu Asp
   355 360 365
   Leu Leu Gln His Ile Val Asn Glu Pro Pro Pro Lys Leu Pro Glu Gly
   370 375 380
   Arg Phe Pro Lys Leu Met Glu Glu Phe Val Asn Leu Cys Leu Val Lys
   385 390 395 400
   Asp Pro Ala Lys Arg Pro Thr Pro Lys Asp Leu Thr Lys His Arg Tyr
   405 410 415
   Val Ile Glu Ala Asp Ala Ala Lys Val Asp Leu Gln Gly Trp Val Asp
   420                                     425                                     430
   Ala Met Lys
           435

<210>  9
<211>  192
<212>  PRT
<213>  Ustilago cynodontis

<400>  9

Met Ser Gly Lys Met Met Ile Tyr Lys Leu Val Val Leu Gly Asp Gly
1               5                   10                  15

Gly Val Gly Lys Thr Ala Leu Thr Ile Gln Leu Cys Leu Asn His Phe
            20                  25                  30

Val Glu Thr Tyr Asp Pro Thr Ile Glu Asp Ser Tyr Arg Lys Gln Thr
        35                  40                  45

Val Ile Asp Asp Gln Pro Cys Met Leu Glu Val Leu Asp Thr Ala Gly
    50                  55                  60

Gln Glu Glu Tyr Thr Ala Leu Arg Asp Gln Trp Ile Arg Glu Gly Glu
65                  70                  75                  80

Gly Phe Leu Leu Val Tyr Ser Ile Ser Ala Arg Ala Thr Phe Glu Arg
                85                  90                  95

Val Glu Arg Phe Arg Ser Gln Ile Ser Arg Val Lys Asp Gln Glu Pro
            100                 105                 110

His Thr Val Pro Ile Met Leu Val Gly Asn Lys Cys Asp Lys Val Asn
        115                 120                 125

Glu Arg Glu Val Ser Arg Glu Glu Gly Gln Ala Leu Ala His Arg Leu
    130                 135                 140

Gly Cys Lys Phe Ile Glu Ser Ser Ala Lys Thr Cys Val Asn Val Glu
145                 150                 155                 160

Arg Ala Tyr Tyr Thr Val Val Arg Met Ile Arg Glu Gln Arg Glu Gly
                165                 170                 175

Thr Thr Thr His Lys Lys Glu Lys Lys Lys Ser Arg Cys Asn Ile Leu
            180                 185                 190

<210>  10

<211>  352
<212>  PRT
<213>  Ustilago cynodontis

<400>  10

Met Ala His Gly Gln Gln Ser Asn Gln Ser Ala Ser Phe Asn Val Gly
1               5                   10                  15

Glu Thr Tyr Lys Ile Val Asp Val Val Gly Glu Gly Ala Tyr Gly Val
            20                  25                  30

Val Cys Ser Ala Ile His Val Pro Ser Ser Ser Arg Val Ala Ile Lys
        35                  40                  45

Lys Ile Thr Pro Phe Asp His Ser Met Phe Cys Leu Arg Thr Leu Arg
    50                  55                  60

Glu Ile Lys Leu Leu Arg His Phe Asn His Glu Asn Ile Ile Ser Ile
65                  70                  75                  80

Leu Asp Ile Val Lys Pro Thr Asp Tyr Asp Ser Phe Ser Glu Val Tyr
                85                  90                  95

Leu Ile Gln Glu Leu Met Glu Thr Asp Met His Arg Val Ile Arg Thr
            100                 105                 110

Gln Glu Leu Ser Asp Asp His Cys Gln Tyr Phe Ile Tyr Gln Thr Leu
        115                 120                 125

Arg Gly Leu Lys Ala Leu His Ser Ala Gln Val Leu His Arg Asp Leu
    130                 135                 140

Lys Pro Ser Asn Leu Leu Leu Asn Ala Asn Cys Asp Leu Lys Ile Cys
145                 150                 155                 160

Asp Phe Gly Leu Ala Arg Ser Ala Asn Gln Pro Glu Ala Glu Gly Thr
                165                 170                 175

Gly Phe Met Thr Glu Tyr Val Ala Thr Arg Trp Tyr Arg Ala Pro Glu
            180                 185                 190

Ile Met Leu Thr Phe Lys Glu Tyr Thr Lys Ala Ile Asp Val Trp Ser
        195                 200                 205

Val Gly Cys Ile Leu Ala Glu Met Leu Ser Gly Lys Pro Leu Phe Pro
    210                 215                 220

Gly Arg Asp Tyr His His Gln Leu Ser Leu Thr Leu Glu Ile Leu Gly

225                 230                 235                 240

Thr Pro Ser Leu Asp Asp Phe Tyr Ala Ile Thr Ser Thr Arg Ser Arg
                245                 250                 255

Asp Tyr Ile Arg Ala Leu Pro Phe Arg Lys Lys Arg Asn Phe Ser Gln
            260                 265                 270

Met Phe Pro Asn Ala Asn Pro Leu Ala Val Asp Leu Met Glu Arg Cys
        275                 280                 285

Leu Thr Phe Ser Pro Arg Lys Arg Ile Thr Val Glu Glu Ala Leu Ala
    290                 295                 300

His Pro Tyr Leu Glu Pro Tyr His Asp Pro Glu Asp Glu Pro Thr Ala
305                 310                 315                 320

Glu Pro Leu Asp Pro Ser Phe Phe Asp Phe Asp Tyr Cys Lys Glu Gln
                325                 330                 335

Leu Ser Arg Ser Glu Leu Lys Arg Leu Ile Tyr Asn Glu Ile Met Arg
            340                 345                 350

<210>  11
<211>  208
<212>  PRT
<213>  Ustilago cynodontis

<400>  11

Met Gly Asp Ala Arg Leu Arg Arg Ile Asn Lys Glu Ile Asn Asp Cys
1               5                   10                  15

Lys Asn Asp Lys Val Ser Gly Ile Gln Val Glu Leu Ile Asp Glu Ser
            20                  25                  30

Pro Phe His Leu Val Gly Thr Phe Pro Gly Pro Glu Asn Ser Pro Tyr
        35                  40                  45

Glu Lys Gly Thr Phe Asp Val Pro His His Glu Gln Ser Cys Phe Ala
    50                  55                  60

Lys Pro Thr Ser Gln Asp Ile Val Val Pro Glu Gly Tyr Pro Phe Gln
65                  70                  75                  80

Pro Ile Lys Met Lys Phe Ile Thr Arg Val Tyr His Pro Asn Ile Ser
                85                  90                  95

Ser Gln Ser Gly Ala Ile Cys Leu Asp Ile Leu Lys Asp Gln Trp Ser

100                 105                 110

Pro Val Leu Thr Leu Lys Ser Thr Leu Met Ser Leu Arg Ser Leu Leu
        115                 120                 125

Cys Ser Pro Glu Pro Asn Asp Pro Gln Asp Ala Glu Val Ala Lys His
    130                 135                 140

Tyr Leu Arg Asp Lys Asp Asp Phe Glu Lys Thr Ala Arg Tyr Trp Thr
145                 150                 155                 160

Glu Ile Tyr Ala Asn Gly Ser Glu Ser Ala Gln Pro Pro Ser Lys Gly
                165                 170                 175

Gln Ile Asp Asp Lys Ala Asp Pro Ile Leu Leu Ala Gly Leu Lys Lys
            180                 185                 190

Glu His Val Asp Gln Phe Val Asn Met Val Ser Val Ser Gln Val Leu
        195                 200                 205

<210>  12
<211>  784
<212>  PRT
<213>  Ustilago cynodontis

<400>  12

Met Thr Thr His Ser Pro Lys Ser Ser Pro Lys Ile His Val Asn Lys
1               5                   10                  15

Trp Thr Glu Gln Gln Val Val Asp Trp Leu Ser Thr Val Gly Leu Ser
            20                  25                  30

Lys Tyr Ala Arg Asp Phe Lys Ser Asn Gly Ile Thr Gly Asp Val Leu
        35                  40                  45

Val Leu Leu Asp Asp Glu Ala Leu Lys Asp Ile Gly Val Val Thr Ile
    50                  55                  60

Gly Gln Arg Leu Ala Leu Leu Ser Ala Ile His Arg Leu Lys Gln Gln
65                  70                  75                  80

Phe Gly Ile Pro Ile Gln Asp Gly Asp Trp Val Pro Lys Ala Val Glu
                85                  90                  95

Ala Gln Glu Gln Ala Ser Asn Ala Leu Thr Thr Ala His Met Ala Asp
            100                 105                 110

Ala Leu Arg Gln Arg Asp Asp Arg Ile Arg Leu Leu Glu Ser Gln Ile

115                 120                 125

Leu Arg Leu Ala Asp Tyr Leu Ala Arg Phe Gln Gln Asp Met Ala Ser
    130                 135                 140

Val Ala Arg His Val Gly Val Lys Ala His Ser Ile Asp Thr Pro Ile
145                 150                 155                 160

Thr Leu Val Ser His Ser Phe His Ser Tyr Asn Ser Ser Glu Ser Ser
                165                 170                 175

Ile Pro Ser Met Ser Thr Tyr Gly Ser Asn Pro Asp Ile Ala Ser Thr
            180                 185                 190

Ala Thr Ser Gly Leu Pro Leu Glu Ser Pro Thr Tyr Arg Asn Val Ala
        195                 200                 205

Gly Leu Val Ser Pro Thr Arg Ser Ala Gly Phe Thr Tyr Pro Ala Ser
    210                 215                 220

His Ala Met Ser Gly Phe Lys Pro Ala Thr Val Thr Ser Ala Arg Ser
225                 230                 235                 240

Asp Ser Ile Gly Gly Asn Ser Pro Met Thr Pro Thr Thr Ala Ala Gln
                245                 250                 255

Ser Asp Ala Ala Gly Ser Ser His Ala Ala Gly Pro Thr Asp Leu Ser
            260                 265                 270

Pro Thr Gln Ser Thr Pro Ser Ala Thr Pro Thr Ser Leu Gly Ser Gln
        275                 280                 285

Pro Gly Phe Pro His Gln Leu Pro Gln Gln Gln Gln Gln Gln Gln Gln
    290                 295                 300

Pro Gln Pro Ser Ser Ala Gly Ser Gly Ser Met Val Ser Pro Thr Arg
305                 310                 315                 320

Arg Leu Ala His Gln Asn Ala Leu Ser Gln Pro Asp Thr Ser Gly Pro
                325                 330                 335

Ser Arg Ala Arg Ala Gly Ser Leu Gly Thr Ser Thr Ala Phe Ser Ala
            340                 345                 350

Asn Gly Ser Ser Pro Phe Ala Ser Gly Ser Ala Ser Thr Ser Asn Glu
        355                 360                 365

Ala Thr Ser Ser Ala Asn Ala Gly Asn Ile Ala Pro Arg Ser Ala Thr
    370                 375                 380

Asp Arg Glu Glu Arg Lys Asp Thr Ser Thr Ala Ser Ser Ser Glu Pro
385                 390                 395                 400

Asn Ala Lys Thr Val Ala Ala Thr Ala Ser Ser Thr Ser Ser Thr Thr
                405                 410                 415

Ser Thr Ser Ala Ser Ala Asn Ser Val Ser Ser Asp Ser Asn Pro Tyr
            420                 425                 430

Lys Ser Phe Arg Val Thr Leu Asp Asp Pro Cys Tyr Lys Val Leu Pro
        435                 440                 445

Ala Ala Leu Lys Lys Tyr Lys Ile Asn Asp Asp Trp Arg Lys Tyr Ala
    450                 455                 460

Leu Phe Ile Cys Tyr Gly Lys Thr Glu Arg Cys Leu Ser Tyr Asp Glu
465                 470                 475                 480

Lys Pro Leu Leu Leu Phe Gln Lys Leu Lys Glu Asn Glu Gln Ser Pro
                485                 490                 495

Val Phe Met Leu Arg His Ile Arg Asp Val Lys Ser Pro Ile Ala Ile
            500                 505                 510

Ala Glu Ala Lys Ala Ala Ser Arg Leu Gln Glu Arg Glu Gly Asp Gly
        515                 520                 525

Asn Ala Pro Ala Lys Lys Arg Ala Ala Pro Arg Ala Asp Arg Arg Arg
    530                 535                 540

Val Ile Ala Gly Thr Pro Pro Pro Gly Ala Leu Val Thr Asn Thr Ser
545                 550                 555                 560

Asn Pro Val Glu Val Gly Pro Ala Ala Asp Leu Pro Asp Arg Asn Lys
                565                 570                 575

Ile Thr Lys Thr Tyr Ala Val Ala Ile Tyr Pro Tyr Met Pro Glu Arg
            580                 585                 590

Asp Asp Glu Phe Asp Val Asn Val Gly Asp Thr Phe Val Val Ile Asn
        595                 600                 605

Lys Ala Lys Gly Trp Trp Ile Val Gln Arg Asp Ala Lys Gly Asp Gly
    610                 615                 620

Ala Gly Asp Val Val Tyr Ser Val Pro Ala Ser Glu Gly Asn Pro Ala
625                 630                 635                 640

Asp Asp Asp Ser Ile Thr Tyr Ser Asn Tyr Arg Ala Glu His Ala Ser
                645                 650                 655

Gly Trp Val Pro Ala Gly Cys Leu Leu Glu Thr Ser Arg Pro Leu Gly
            660                 665                 670

Ser Ile Val Asp Ala Glu Val Val Ser Ser Arg Met Arg Ser Gly Pro
        675                 680                 685

Ser Thr Ile Ser Asn Pro Ser Thr Pro Thr Phe Ser Thr Ile Ser Gly
    690                 695                 700

Ser Ser Gly Ser Thr Ser Gly Gly Lys Met Asp Ala Lys Arg Ala Ser
705                 710                 715                 720

Ile Pro Pro Ala Leu Ile Thr Ser Thr Ser Thr Pro Gly Ile Met Leu
                725                 730                 735

Met Asp Tyr Gln Ser Ala Glu Gly Asp Leu Asp Leu Arg Lys Asp Glu
            740                 745                 750

Arg Leu Arg Val Phe Lys Arg Tyr Asn His Trp Ser Tyr Cys Val Gln
        755                 760                 765

Glu Arg Asp Gly His Ala Arg Gly Trp Val Pro Ser Trp Tyr Ile Gly
    770                 775                 780

<210>  13
<211>  144
<212>  PRT
<213>  Ustilago cynodontis

<400>  13

Met Ala Met Lys Arg Ile Asn Lys Glu Leu Ala Asp Leu Gly Arg Asp
1               5                   10                  15

Pro Pro Ser Ser Cys Ser Ala Gly Pro Thr Gly Asp Asn Met Phe Gln
            20                  25                  30

Trp Gln Ala Thr Ile Met Gly Pro Gly Asp Ser Pro Tyr Ala Gly Gly
        35                  40                  45

Val Phe Phe Leu Ser Ile Thr Phe Pro Thr Asp Tyr Pro Phe Lys Pro
    50                  55                  60

Pro Lys Val Ser Phe Ser Thr Lys Ile Tyr His Pro Asn Ile Asn Ser
65                  70                  75                  80

Asn Gly Ser Ile Cys Leu Asp Ile Leu Arg Asp Gln Trp Ser Pro Ala
                85                  90                  95

Leu Thr Ile Ser Lys Val Leu Leu Ser Ile Cys Ser Met Leu Thr Asp
            100                 105                 110

Pro Asn Pro Asp Asp Pro Leu Val Pro Glu Ile Ala Asn Leu Tyr Lys
        115                 120                 125

Thr Asp Arg Gln Arg Tyr Glu Ser Thr Ala Arg Glu Trp Thr Arg Lys
    130                 135                 140

<210>  14
<211>  831
<212>  PRT
<213>  Ustilago cynodontis

<400>  14
   Met Pro Pro Thr Thr Thr Arg Leu Gln Thr Arg Gln Ala Gly Leu Ser
   1               5                   10                  15
   Ala Gln Glu Ala Ala Ser Ser Asp Ser Gly Arg Arg His His Ile Ser
               20                  25                  30
   Val Asn Leu Phe His Gly Trp Ser Thr Glu Thr Pro Ala Ser Gly Ser
           35                  40                  45
   Ser Thr Ser Arg Gln Glu Ser Ile Met Val Lys Arg Ser Leu Ser Gln
       50                  55                  60
   Glu His Leu Asp Met Val Met Pro Ala Ser Gln Ser Ala Pro Ile Phe
   65                  70                  75                  80
   Gln Lys Ile Ser Leu Ala Lys Gly Asp Pro Gly Ser Ser Ser Leu Ser
                   85                  90                  95
   Gln Asp Asp Glu Glu Glu Ala Glu Arg Ile Lys His Leu Pro Pro Ser
               100                 105                 110
   Lys Arg Pro Gly Val Ser His Ala Lys Lys Thr Pro Pro Asn His Ile
           115                 120                 125
   Lys Arg Pro Arg Asn Ala Tyr Ile Ile Phe Arg Ser His Thr Val Ser
       130                 135                 140
   Gln Lys Leu Ile Pro Lys Glu Val Glu Asn Asp His Arg Asn Ile Ser
   145                 150                 155                 160
   Arg Ile Ile Ala His Met Trp Lys Ser Leu Asp Pro Glu Asp Arg Ala
                   165                 170                 175
   Tyr Tyr Glu Glu Ile Ala Arg Glu Glu Lys Glu Arg His Lys Arg Leu
               180                 185                 190
   Phe Pro Asn Tyr Arg Tyr Gln Pro Thr Thr Arg Arg Gly Asp Val Lys
           195                 200                 205
   Gln Arg Asn Val Lys Lys Leu Glu Asn Gly Glu Glu Glu Cys Gln Glu
       210                 215                 220
   Ile Ala Asp Ile Ile Leu Lys Ala Gln Gly Lys Glu Gly Val Val Val
   225                 230                 235                 240
   Arg Pro Glu Pro Ser Lys Pro Met Lys Arg Ala Arg Glu Ala Thr Arg
                   245                 250                 255
   Pro Arg Ser Lys Arg Tyr Ser Thr Ala Thr Pro Val Arg Lys Arg Val
               260                 265                 270
   Lys Thr Ala Lys Thr Pro Glu Ser Asp Ala Gln Ser Ser Val Gln Thr
           275                 280                 285
   Leu Gln Thr Val Phe Leu His Pro Ser Pro Ser Thr Ser Pro Thr Thr
       290                 295                 300
   Ala Pro Ser Ser Gly Gly Val Gly Ala Val Ser Thr Pro Ser Pro Gln
   305                 310                 315                 320
   Ser Phe Arg Leu Gly Ser Arg Thr Pro Ser Ile Asp Asn Ser Met Pro
                   325                 330                 335
   Gln Ala Pro Ser Ala Glu Pro Leu Pro Ile Thr Glu Asp Val Phe Gly
               340                 345                 350
   Arg Arg Ala Ser Ser Val Pro Val Leu Arg Pro Cys Ser Pro Pro Pro
           355                 360                 365
   Ala Phe Val Ser Glu Ser Gln Pro Ser Pro Val Leu Glu Ser Arg Asp
       370                 375                 380
   Leu Val Ser Ser Ala Glu Ala Ser Gly Arg Glu Arg Ala Met Gln Asp
   385                 390                 395                 400
   Gly Ile Arg Ser Ser Ser Val Gln Ser Arg Val Gly Phe Ala Asp Glu
                   405                 410                 415
   Gln Gly Met Pro Ala Pro Ser Trp Lys Gly Arg Lys Ala Leu Pro Pro
               420                 425                 430
   Pro Ile Pro Asn Thr Trp Gln His Cys Ser Phe Asp Asp Gln Ser Leu
           435                 440                 445
   Pro Ser Pro Arg Ser Phe Asp Ala Met Gly Ala Ala Gly Lys Pro Ala
       450                 455                 460
   Met Ala Arg Pro Arg Thr Ala Tyr Pro Ser Thr Pro Ser Ser Gly Ser
   465                 470                 475                 480
   Phe Arg Gly Phe Phe His Pro Trp Ala Phe Asp Asp Gly Thr Glu Ser
                   485                 490                 495
   Met Leu Ile Ser Pro Met Thr Ala Ser Phe Gln Asp Thr Arg Arg Arg
               500                 505                 510
   Ser Ser Leu Val Arg Ser Gly Leu Val Ala Gly Arg Arg Pro Gly Ser
           515                 520                 525
   Leu Gly Leu Asp Ala His Asn Asn Leu Gly Val Arg Pro Asp Pro Val
       530                 535                 540
   Asp Gly Ser His Ala Phe Val Ser Gly Pro Asp Ser His Ile Ser Asp
   545                 550                 555                 560
   Val Gln Leu Phe Asp Glu Ala Thr Arg Ala Ala Glu Ile Ser Leu Gly
                   565                 570                 575
   Ile Glu Ser Ser His Gly Glu His Val Asp Val Ser Thr Phe Ala Phe
               580                 585                 590
   Asp Pro Ala Leu Glu Gly Glu Gly Gly Gly Gln Ala Gln Leu Ser Ser
           595                 600                 605
   Phe His Gly Gln Val Ala Leu Arg Glu His Gln Ala Leu Pro Ser Pro
       610                 615                 620
   Val Ala Ser Pro Arg Leu Met Ala Asp Ser Pro Arg Pro Ser Phe Ser
   625                 630                 635                 640
   Gly Ser Thr Leu Ala Ala Ala Ala Arg Asp Trp Ala Ser Met Lys Lys
   645 650 655
   Arg Arg Ser His His Glu His Arg Ser Val Leu Asp Pro Val Gln Asp
   660 665 670
   Ser Ile Ser Val Pro Thr Glu Gly Ile Met Ala Pro Ala Glu Ala Arg
   675 680 685
   Val Gly Ala Ala Thr Asn Ser Ser Gln Gln Arg Ser Ala Leu Glu Glu
   690 695 700
   Ser Ile Glu Arg Ala Val Leu Leu Ala Leu Gly Arg Asp Ser Gly Asp
   705 710 715 720
   Gly Ala Asp Ser Gly Asp Arg Asn Ser Gln Ile Val Gln Gln Ile Leu
   725 730 735
   Ser Ser Leu Asn Ala Glu Leu Ser Leu Gln Gln Gly Ser Ala His Gly
   740 745 750
   Ile Ala Thr Ala Gly Lys Ala Ser Ser Pro Ile Leu Glu Pro Gly Arg
   755 760 765
   Arg Ser Ser Leu Arg Gly Ser Val Val Gly Pro Pro Tyr Arg Ser Ala
   770 775 780
   Pro Tyr Phe Ser Gly Leu Ser Ser Ile Asp His Gln Arg Asn Leu Lys
   785 790 795 800
   Pro Leu Pro Ser Pro Leu Gln Leu Val Met Ser Asn His Glu Ile Ser
   805 810 815
   Ser Glu Ala Tyr Ser His Ser Tyr Pro Thr Ser Gln His Cys Ile
   820 825 830

<210>  15
<211>  1308
<212>  DNA
<213>  Ustilago maydis

<400>  15
atgctttcgt ccggtgcggg atcttccata cgcaagaagc gcaacttcaa ggggctacaa      60

ctggccgagt cacctctcgc cagtcccgtc gacgcttcgg ctacaacacc atcacacaaa     120

ccaggcgaag gctccgccgc ctcaaatgca tccaccatcg gcaaatcctc tgcagtgacg     180

ccgggcggct cccttgcact cccggtcaag aatggtctgg atacagaacc caactcggga     240

gccaattacc acaacaagtt gacgcagcaa ctggccaacc tcgaactggg cgtcgaatac     300

aagcttgact tgaagaacga ggacctcaag acgctctcgg agctcggcgc cggtaatggc 360

ggaactgtca ccaaggtgct gcacgagaag agcggaaccg tgatggccaa aaaggtggtc 420

ttcatcgacg ccaaacctag cgtacgcaag cagatcctac gagagcttca gatcctgcac 480

gagtgcaatt cgccctacat tgtctcgttc tacggcgcct atctcaacga accgcacatc 540

tgcatgtgca tggagtttat gcagaaggat tcgctcgatg gcatctacaa aaagtacggg 600

ccaatctcac cggaaatttg tggcaagatt gccgtggcag tctcgcacgg cctcacctac 660

ctctacgacg tgcatcgaat catccaccgt gacgtcaagc ctagcaacat tctagtcaac 720

ggtgccggtc aaatcaaaat ctgtgacttt ggcgtaagcg gagagctcat caacagcatt 780

gcagacacgt ttgttggtac aagtacctac atgtcgcctg agcgaattca gggagatcag 840

tacagtgtca agtcggatgt atggagcttg ggcgtgtcga tcatcgagct ggcgctgggt 900

cgcttccctt ttgcagagaa cgaagaggac gacgactcgg acgcggataa caactatacg 960

aacgaagatc tggcggggac actttcaccg accaaaccgg caccaatgat ttcgctcggc 1020

caaaacgaaa aacagagacg cagaaaatca aaaccggccg gcgtaagcct cgagggctcg 1080

agccatcaga tgtcaattct cgacctgctg caacacattg tcaacgaacc acctcccaag 1140

ctgccagagg gcagatttcc caagcacatg gaagagtttg tcaacctttg tctgctcaaa 1200

gacccggcga aacggcccac acccaaggac ctcaccaagc accagtacgt gatcgatgcc 1260

gatgccgcca aggtggactt gcaagcatgg gccgatggga tgaagtaa 1308

<210> 16
<211> 579
<212> DNA
<213> Ustilago maydis

<400> 16
atgagtggca aaatgatgat ctacaagctt gtggtgctgg gagatggagg tgtaggaaag 60

acggcgttga ccatccagct ctgtctcaac cactttgtcg agacgtacga cccgaccatc 120

gaagactcgt atcgcaaaca gaccgtgatc gacgaccaac catgcatgct cgaagtactt 180

gacacggctg gtcaggagga gtacactgcg cttagagacc aatggatccg cgaaggagaa 240

ggttttctgc tcgtctattc gatctcggcg agggcgacgt ttgaacgtgt agagcgattc 300

cgcagccaaa tctcgcgcgt caaggaccaa gagccacata cagtgcccat catgctggtt 360

gggaataaat gcgacaaggt gaatgagagg gaggtatcaa gagaggaggg gcaagcgctt 420

gcgcatcgac taggctgcaa gtttatcgaa tcgagcgcaa agacgtgtgt caacgtcgaa 480

cgtgcttact acaccgtggt tcgcatgatc cgcgaacagc gcgaaggtac ggtaacgcac 540

aaaaaggaga agaagaagtc aaaatgcaat atcctttga 579

<210> 17

<211>  1065
<212>  DNA
<213>  Ustilago maydis

<400>  17
atgtcacatg cccacggaca gcagcccaac cactcgacca ccttcaaagt aggcgagacg      60

tacaaggtcg tcgatgtggt cggcgaggga gcttacggtg ttgtgtgctc ggccatccac     120

gttccctcga gctctcgtgt cgccattaag aagatcaccc cattcgatca ctccatgttc     180

tgtcttcgaa ccctacgcga gatcaagctg ctgcgtcact tcaaccacga aaacatcatt     240

tcaatcctcg acattgtcaa gcccgatgat tacgacagct tttccgaagt ttacctcatc     300

caagaactta tggagaccga catgcatcgc gtcattcgca cccaggagct ctccgatgac     360

cactgtcagt actttattta tcaaacactg cgtggcttga aggcgctcca ctcggcgcaa     420

gtgctgcatc gcgatttgaa accctcaaac ctcttactca acgctaactg cgatctcaag     480

atctgcgatt tcggtctagc ccgttccgca aaccaacccg aagccgaagg gacgggtttc     540

atgaccgaat acgttgccac acgatggtat cgcgcaccgg aaatcatgtt gacgttcaaa     600

gagtatacaa aggccatcga tgtttggtcc gtgggctgca ttctggccga gatgcttgcc     660

ggaaaaccgc tcttccctgg tagagactac caccaccagc tttcgctcac cttggagatc     720

ctcggaacgc cttcgttgga cgacttctac gcaatcactt ctactcgctc ccgagattac     780

atcagggcac tacctttccg aaaacgacgc aacctttcgc tcatgtttcc caatgccaat     840

cctctggccg tcgatctgat ggaaaagtgt ctcaccttta gccccaggaa gcgcatcacc     900

gtggaagagg cgttagcaca tccatatttg gaaccctatc atgatcccga agacgaaccg     960

acagctgaac cgctcgatcc tagcttcttc gactttgact actgcaaaga gcaactctca    1020

aggtccgaac tcaagaggtt gatttataac gagatcatgc gttga                    1065


<210>  18
<211>  1104
<212>  DNA
<213>  Ustilago maydis

<400>  18
atgggagacg cacggctaag acgtatcaac aaggagatca acgactgcaa aaatgacaag      60

gtttcgggca ttcaagtctc attgatagat ggtaggcata gcttcaaagc cgctcattcc     120

agctggacgt cgctgatcac ttctttttgc cgccttcttc tgctctggcc gcacgtcaga     180

atcgcccttc catctggttg gaacttttcc cgggccagag aactctccat acgagaaggg     240

tactttcgat gtagtatgtt tcaacctgcc gtagctgccg aagctcggtg gcgcaaatgc     300

gatccagtaa tgctggcaga ttgtgccgac tgttggtcat cacgactaaa ccaatgctgc     360

gctgtacatt gccttgatga tttttgcagg acattgtcgt acccgaaggt tacccattcc     420

agcccatcaa gatgaagttt atcacgcgcg tatatcaccc caacatctct tcgcagtctg     480

gcgctatttg cctcgacatt cttaaagatc agtggtcgcc cgtgcttacg ctcaagtcta 540

cgcttatgtc gctgcgctct ctgctatgct cgcccgagcc aaacgaccca caagatgctg 600

aagtagccaa gcactacctt cgagacaaaa atgactttga aaagaccgcc agatattgga 660

ccgagatcta cgcaaatggc tccgaatcag cgcagccacc cgcaaatgcc cagaatacgg 720

tgggaaagac aggtgacagg gccgatccaa tcctgctggc aggattgaag agggagcatg 780

tcgaccagtt tgtcaacatg gtgagttgcc cagtctcaca agatagtcac aagatcgtct 840

caactcggaa atcattctag actgacatgt cgattcgtcc acatacatga tcattctcac 900

cagggcttcg aaaccaacaa ggtgatcgag gcgttgaaaa agcttaacta ccgaggtgca 960

aagtaagttg catcaccaac agttctcaaa gacgtcttga ttcgtatcgc aactctgtgg 1020

cgtcagctga cctgttcgct tggtttctcc cacagcgtcc gcaacatcac cgacgacaca 1080

gtcctcaacg agctcctcaa ataa 1104

<210> 19
<211> 2490
<212> DNA
<213> Ustilago maydis

<400> 19
atgtcctctc aaagcccaaa gtcgtcaccg acagttcatg tcaacaagtg gtccgagcag 60

caggtcgtag actggctctc ctccgtcggt ctttccaagt atgcaagaga cttcaagtca 120

aacggcatca ctggcgatgt gcttgttctg ctcgacgacg aagctctcag ggatatcggc 180

gtcgtcacca taggtcagcg cctcgcgctc cttgctgcca tctaccgtct caagcagcag 240

tttgacatcc ccatccaaga aggcgactgg attcccaagt caatcgaggc gcaagaacat 300

gccaccaaca cgctcagcac agctcacatg tctactgcgc tacgtcaacg cgacgatcgc 360

attcgtctct tggaatcgca gatcctgcga ctcgcagact atctcgttcg cttccagcag 420

gacatggcgt ccgtagcaag gcacgtcgga gtcaaggcgc attccattga cacgcccatc 480

acccttgttt ccaactcgct ctccgcgcat cgttccagtg agagcagcgc caccacgtcc 540

gccttcggct caaattccga tctggcttcc tcagccgcat ccagcttgcc catggaatcg 600

cccacttcgc gcaactttgc tggtcttgtc tctcccactc gaaccggctt cacatatggc 660

ggctcacaag ccatgagcgg cttcaaacca accaccgtca agtcgtccag gtctaatagt 720

atcggtggtg ccgcgcccat gacacccact actgccgcac acatgtatgc ggccagcgcg 780

tccaatacag tgcccacagg agatgtttcg ccaacgcaga ttactccctc ggtcactccc 840

acatcactcg gatctcagcc tgctttccct cagcagcaac cgccatccag tgccggttcc 900

acctcgatgg tgagtccgac ccgtcggctt gcgcatcaga actcgcttgc acatccagac 960

gccgctggct ctgctcgacc gcgtgcaggc tccgtgggtg catccgctgc tctgtcgtct 1020

accagtggct catctctctt ttccaatact ggaagcagcg gctccatcaa cgccaccact 1080

gtcagcgcag caaccgaacg cgacgatcgc atcaaagacg ccgcagcgac gaccgtgtcg 1140

gaaccgaagc ccaaattttc gacggtcgct gcctcgggct cttcgttgtc ggcatcgcca 1200

gccgcttctg cagcggctgc taccacggca tcctccgaca gcaacccgta caagtcgttt 1260

cgcgtcactc tcgacgaccc atgctataag gtgctcccag cagcgctcaa aaagtacaag 1320

atcaacgacg actggcgcaa gtacgcgctc tttatctgct acggcaagac ggagcggtgt 1380

ctcagctacg acgaaaaacc tctgcttctg tttcagaagc tcaaggaaaa cgaacagagc 1440

cctgtcttta tgcttcgtca catccgcgac gtaaaatcgc ccattgcgat cgccgaggca 1500

aaggcagcct cgaagcttca ggaacgcgaa accgacggtg gttcgtccgc taccaagaag 1560

cgaagcgcgc cgcgtgccga ccgacgtcgt gtgattgcag gaacaccgcc gccgggtgcg 1620

ctcgtcacca acgcttcgaa cccggtggaa gttggaccgg ctgccgactt gcccgaacgc 1680

aacaaggtgg cgagaacata cgcggtggcc atctacccat acatgcccga gcgcgatgac 1740

gagtttgacg tgaatgtggg cgatacgttt gtcgtgatca acaaggccaa agggtggtgg 1800

atcgtgcaac gagacgcaaa gcaagacggt gcaggggacg tggtgtatag cgtcccggcg 1860

agcgaagcga gttcggcgga tgatgatgga gtcacctact cgaactaccg agccgagcat 1920

gcgtctggat gggtgcctgc cgggtgtctg ctcgagacaa gtcgacctct ggggagcatt 1980

gtggaagccg aggcggcgtc gatgtcgcat gtgcggtcgg gtgcaagcac catctcgaac 2040

ccgagcacgc ccacggcatc gagcagcgga gccaagctgg atgccaagcg tgcggcgatt 2100

ccgccggcgc tgattacgag caccagcacg ccggggatca tgctcatgga ttaccagagt 2160

ggcgagggcg atctggatct gcgcaaggac gagcgtctgc gcgtattcaa gcggtacaac 2220

cactggtcat attgcgtaca ggagcgagat gcgcacgcca ggggctgggt ccccagctgg 2280

tacattggca agctttcctc ggccgccagc ggatcgttgg gcaagtcggc gtcgaccact 2340

gctttgtctg cgacgcccgt cccggattcg cgcttgatgc atgcggcaga gtccgtcaca 2400

gccgtggccg gctcgccgtc gtacgacagc agtttgacac acgaccatcc gtccgcgcat 2460

tcagcttccg cgccgatggc taccgtctga 2490

<210> 20
<211> 444
<212> DNA
<213> Ustilago maydis

<400> 20
atggctacga agagaatcaa caaggagctc gtcgacctcg gtcgcgatcc tccttcgtcc 60

tgcagcgctg gccccaccgg tgacaacatg ttccaatggc aagcaacgat catgggaccc 120

ggcgactctc cttacgccgg cggtgtcttt ttcctctcaa tcactttccc cactgactac 180

cccttcaagc ctcccaaggt ttctttttcc accaagatct accatccgaa catcaacagc 240

aacggcagca tctgtctcga cattctcaga gaccagtgga gtccagcgct gaccatctcc 300

aaggtgttgc tctcgatctg ttcgatgctc actgacccaa atcctgatga tccgcttgtt 360

cccgagatcg ccaacttgta caagacggac cgtcagcgtt acgagagtac tgcccgtgaa 420

tggaccagaa agtacgccat gtaa 444

<210> 21
<211> 2523
<212> DNA
<213> Ustilago maydis

<400> 21
atgcgagatc caggctgcgc cgacgccatc gctacgcgat cagggcctcg aaatcagatc 60

atggtcgacc tcctccgggg atggtctacc gaagcggaat ccggcagcag ttcatccaca 120

gcactcagtg gactcgacgg gagcccaatc aagcgatcag cctctcaaga ggcatccatc 180

ttgagcaacc aagaaacagt gcccctgttt caaaccatct cgcttccaaa caaggatgta 240

tcgctggcag tctcgctcta cacaggctgc aaatccgaac aggagatgga ccaggaagag 300

ctcgaaagaa tcaagcgtct acctccatcc aaacgacctg gtgtctcgca cgccaaaagg 360

acgccgccca accatatcaa gcgtcctcgc aatgcctaca tcatctttcg atcccacatt 420

gtctcgcaaa aacttatacc caaggaggtc gaaaacgatc atcgcaatat cagccgcatc 480

attgcccaca tgtggaagag cttggaaccc caagaacgtg cccaatacga gcaaatcgcc 540

aagcaagaga aagaaaggca caagcaactg tttcccgagt accgatatcg acccaccacg 600

cgtcgcaccg gcgtcagcaa gcgaaatgtc aaaaaactcg agaatggcga agaggagtgc 660

caagagatcg ccgacatcat cctcaaggca caaggcaaag agggtgtaat tgttcgcggc 720

ggaccctcca aagccatcaa aagggcacgc aaggcgacgc gcgcttccgt ggcagctcgc 780

aattgtggca agccgtcatc gccacgtgca aagcgagcca gggtggacaa atcaaagccc 840

gacgagccag tccctccttc tcaagcagct gatgaggggc tcgagacgat attcttgcat 900

ccgtcacctt cgcagcagtc gccgacattg agcccaagct acagccctga ggctgaatcg 960

gtgccaacgc ccgagccgcg tcccgaatcc tctcgaatga catcgaggac accttctatc 1020

gatcgtggac agcctttgcc atcgtctggt tcggctctga tgctggacat ttcggaaatg 1080

tttggacgcc ggtcctcttc tgtaccagtc atgcaccctt gctctccaac gctagccttc 1140

atcccaccta cccaagagaa cacagacgct gagaccttag gaacgcatca agatgagctt 1200

ggcacccatg tagcagacgc aggtgatggc tacaccaatc actttagtat gccagcacca 1260

aggtggaaag gtcgcaagac gattcctgct cccattgcaa acacgtggca attctgctcg 1320

tacgacgacc agtcactacc ttcgccgcaa tcctttgaca ccttcgcatc ggcaagcaag 1380

atgcccgccg ctcgaccgcg caccgcatgg ccttcgacgc caagctcgag cactttccgc 1440

ggatttttcc atccgtgggc cgtcgaacac aacaacgact cgatgctcat ctcgcccatg 1500

acagcgtcat ttcaagatct gaggagacgc agctcccttg cacgatctgg tctaagtgcc 1560

actcgaaggc cgggctcgtt cggggtagat gttcaaccca actctagcgg tcgccttgag 1620

ggtgacgtcg gaacccaagc tcttttgcaa ccctcgtccg gggctcgtct gccggacgga 1680

cagctttttg gcgaggcaca tccagccgcc gccgtccact tgaactcgga gattgcttcg 1740

gctgactatg atgaaactcc caattttgcc ttcgatccgg ctctggaggg cgaaggtcgc 1800

gtgccagctg cgcccgactc tgtcgctgaa cagccgtgcc atagcgaccg cagtctggca 1860

gctcaagtga tgtctgcttc tcctcgctcc caggcccaga gtccacgccc ttcgttttcg 1920

ggtagcacgc tggccgctgc cgctcgtgac tgggctagca tgaaaaggcg tcggagcaaa 1980

atggcacaga cgcccatcgt caatgccgtt agtgagtctt tgcccgcacc gattgactcc 2040

agtgactcga tcgaagcctc gatgcttcga gaaacagaca aaccggggcg gctctacgga 2100

tccagcctcg aagagtcggt agaacgagct gtgatgctgg ctctctccaa ggatcacggt 2160

caagattcca atctgggcga gcgcaactcc aacatcgtcc aacagatcct gtcttggctc 2220

agtacagagc ttgcactcca gtcacaaaat cagcaggagc atcaacagtt cacgccctcg 2280

atctgccacg ccgacaaagc ccctctgagc tttgtcgagt ctcgcccaag ggcgtgctct 2340

cgtgcgtctc gtggtgtcgc gaggcccaat caatactatg ctgccaatgc agcaattgag 2400

ggtggatcgt ctcgtctcgg caaaccgctt ccttcgccgt tgcagctcgt cacgtcaaac 2460

tacgagattg gtacttcaac tagacattct cattcctatt catctcctca gcactgcatc 2520

tga 2523

<210> 22
<211> 1308
<212> DNA
<213> Ustilago cynodontis

<400> 22
atgctttcct ccagcgcagg atcatctata cgtaagaagc gcaacttcaa agggctgcaa 60

ctggccgagt caccactgtc gagcccagca gctgccacaa cgccatctca aaacacgggt 120

gaaggatcag ctaactcgaa tgcatcgacc atcggcaagt catcgaccgt aacaccagga 180

ggatccctgt ccctcacagc aaagaacggt ctcgacacag agcccaactc gggcgccaac 240

tatcacaaca agttgaccca gcagctggca aatttggagc taggcgtcga atataagctc 300

gacttgaaga atgaggatct caaaacacta tcagagcttg gagcagggaa cggcggtacc 360

gtcaccaagg tgctccacga gaagagcggg acagtcatgg ccaagaaggt cgtcttcatt 420

gatgccaagc caagtgtgcg aaagcaaatc ttgagagagc tgcagatctt gcacgagtgc 480

aactcgccat acattgtctc gttttacggc gcctacctca gcgagccaca catctgtatg 540

tgcatggagt tcatggaaaa ggactcgctc gatggtatct acaagaaata cggtcctatc 600

gctccggaga tctgcggcaa gatcgcagtg gtcgtagcac acggactcac ctacttgtat 660

gacgtgcatc gcatcatcca ccgtgatgtc aagccaagca acatcctcgt caacggcgcc 720

ggacagatca aaatctgcga ctttggcgtc agcggcgagc ttatcaacag tatcgcagac 780

acgtttgtcg gcacgagtac ctacatgtca cctgagcgaa ttcagggtga tcagtacagt 840

gtcaaatctg acgtttggag tttgggtgtc tcgattatcg agttggcttt aggacgattc 900

ccttttgcag agaacgagga cgactttgac tcggacgacg acgatgccag cgacttgctc 960

ggcgacgatc ttagcggtac actttctcct acgaaacctg ctcccaaatt gcatgtggcc 1020

aacaaggaga agcagaatcg caggaagtcg aaagcagccg gtgtcagttt ggaaggatca 1080

agtcatcaaa tgtcaatctt ggatctcttg caacacattg tcaatgagcc accgccgaag 1140

ctgccagaag gaaggttccc gaagttgatg gaagagtttg tcaacctgtg tctagtcaag 1200

gatcctgcaa agcgaccgac acccaaggac ttgaccaagc atcgatacgt gatcgaggca 1260

gacgctgcca aggtggactt gcaaggatgg gtcgatgcga tgaagtag 1308

<210> 23
<211> 576
<212> DNA
<213> Ustilago cynodontis

<400> 23
atgagcggca agatgatgat ctacaagctc gtcgtactcg gagacggtgg tgtcggcaaa 60

accgctctca caattcaact ctgtctcaac cacttcgtcg agacctacga ccctaccatc 120

gaagattcgt accgcaagca gaccgtcatt gatgatcaac cctgtatgct cgaagttctc 180

gacactgcgg gtcaagaaga gtacactgca cttcgcgatc aatggattcg cgaaggtgaa 240

ggtttcttgc tggtctactc tatctcggca agagcgactt ttgaaagggt tgagaggttc 300

aggagtcaaa tctcgagggt gaaggatcag gagccacata cggtacctat catgttggtt 360

gggaataagt gtgataaggt gaatgaaagg gaggtttcca gggaggaggg tcaggcgctg 420

gcgcataggt tgggatgcaa gttcatcgag agtagtgcga aaacttgtgt taatgtggaa 480

agagcgtatt acacagtggt gaggatgatc agggagcaga gggaggggac gacaacacat 540

aagaaggaga agaagaagag tcggtgcaat attctc 576

<210> 24
<211> 1058
<212> DNA
<213> Ustilago cynodontis

<400> 24

atggcgcacg gtcaacaatc caatcaatct gcctcgttca acgtcggcga gacctacaag 60

atcgtcgatg tggtcggtga aggagcctac ggagtcgtct gttccgccat tcacgtcccc 120

tcaagttccc gagtagccat caagaagatc acacccttcg accactccat gttctgtctc 180

cgaactctcc gcgagatcaa gctgcttcgt catttcaacc acgaaaacat catttccatt 240

cttgacattg tcaagcccac cgactatgac agcttttccg aagtctacct cattcaagag 300

ctcatggaga ccgatatgca ccgagtcatc cgtacccagg aactttcgga tgaccattgc 360

caatacttca tctaccaaac actacgaggc ttgaaggctt tgcattcggc tcaagtgctg 420

catcgtgatt tgaagccatc caatctgttg ttgaacgcca actgcgattt gaagatctgc 480

gattttggtc ttgcacgatc agctaaccaa ccggaagcag aaggaacagg gttcatgacc 540

gaatacgttg ctacccgatg gtaccgagca ccagagatca tgcttacgtt caaagagtac 600

accaaagcca tcgacgtttg gtccgtaggt tgcatcttgg cagagatgct ttccggtaaa 660

ccacttttcc ctggccgcga ttatcaccac caactttcgc ttacgctgga aatcttgggc 720

acaccttctt tggacgactt ttacgcaatc acttcgactc gttctcgaga ctatattcga 780

gctttacctt tccgcaagaa acgtaatttc tctcagatgt tccccaatgc aaacccctta 840

gcggtggatc tcatggaaag gtgccttaca ttcagtccaa ggaaaagaat cacggtggaa 900

gaggcattgg cgcatcccta tttggaaccg tatcacgatc cagaagatga gccaacagca 960

gaacctttag atcctagctt ctttgacttt gattactgta aagaacaatt gagtagatcg 1020

gagttgaaga gattgatcta taacgagatc atgcgcta 1058

<210> 25
<211> 1089
<212> DNA
<213> Ustilago cynodontis

<400> 25
atgggagacg ctcgcttgag aaggatcaac aaggagatca acgattgcaa gaatgacaag 60

gtttctggta ttcaagtgga attaatagat ggtaggttta tatcagctgg ttaagatcgg 120

tcgttaggac agcagctgac ttttagcatg acgaatgcca acagagtctc catttcacct 180

agttggtacc tttccaggac cggagaactc accgtacgaa aagggcacct ttgatgtggt 240

aagtatcccc caatgcagtg gactgcccct agctggctta ttgcgttgtt cgccgagtct 300

atcatgagac tgtccctctt gacaaactca ctctttccgc atcgctactt gttccgcacc 360

acgaacaatc atgcttcgcc aaaccaacct cacaggacat cgtcgttccc gaaggctatc 420

cattccagcc tatcaagatg aagttcatca ctcgtgtcta ccatcccaac atctcgtcac 480

aatccggagc catctgtctc gacatcttga aagaccagtg gtcacccgtc ctcacgctca 540

aatctacgct catgtcgctc cgatccttgc tatgctctcc cgagcccaat gatccgcaag 600

atgccgaagt ggcaaagcat tacctccgtg acaaagacga ctttgagaag acagccaggt 660

attggaccga aatctatgcc aacggctccg agtctgcaca accgccatcg aaagggcaaa 720

tcgacgacaa agcggacccg atcctgctgg cagggttgaa gaaggagcac gttgaccaat 780

tcgtcaacat ggtgagtgtc tcgcaagttc tgtagggctg cctgggggtt taagaagagt 840

tgaatgctga catcgtacca atctttgctg cacgtctgcg tacatatgca tcacagggct 900

tcgaaagcaa caaggtcatt gaagtcctca agaagctcaa ctaccgtggc gcgaagtaag 960

tgtcctggta gcaactacca cccgctccgt tcggctatga tataaacggc aaggactgac 1020

acgcatctca ttgctcctct caacagcgtc cgcaacatca ccgacgacac ggtcctcaat 1080

gagcttctc 1089

<210> 26
<211> 2352
<212> DNA
<213> Ustilago cynodontis

<400> 26
atgaccacac acagcccaaa gtcgtcaccg aagatccacg tcaacaagtg gaccgaacaa 60

caggtcgtcg actggctctc cactgtcggt ctctccaagt acgccagaga cttcaaatca 120

aatggcatca ccggagacgt cctcgttctt ctcgacgatg aagcgctcaa agacatcggc 180

gttgtgacca ttggtcaaag gcttgcgctt ctttcggcta tccaccgtct caagcagcaa 240

tttggcatcc ccattcagga tggcgactgg gtcccaaaag cagttgaggc tcaagagcaa 300

gcgtccaatg ctctcactac agcccacatg gctgatgcgc tccgtcagcg agatgatcgt 360

attcgtctcc tggagtcgca gattcttcga cttgccgact accttgcccg cttccaacaa 420

gatatggctt ccgttgctcg tcacgttggt gtcaaggcgc actcgatcga cacacccatc 480

acactcgttt cccattcgtt ccattcatac aactcttccg agagcagcat accctccatg 540

tcgacatacg gctccaatcc ggacatcgct tccaccgcta catccggcct gccactcgag 600

tcgccgacgt atcgtaatgt tgccggcctc gtctcgccca ctcgatctgc tggcttcacc 660

tatcctgcaa gccatgctat gagcgggttc aagcctgcca ccgtcacttc tgctcgctca 720

gatagtatcg gcggcaactc gcccatgaca cccactaccg cagctcaatc tgacgctgcc 780

ggctcctctc acgcagctgg tcccaccgac ctttcgccaa cacagtcgac accgtctgca 840

acaccgacgt cgcttggctc ccagcccgga ttcccacacc agcttccaca gcaacagcag 900

cagcagcaac aaccgcagcc atccagtgca ggctctggtt ccatggtcag cccgactcgt 960

cgactcgctc atcagaacgc gctttcacag ccagacactt ccgggccttc tcgagctcgt 1020

gcaggctctt tgggcacttc gacagcattc tccgccaacg gcagctcgcc cttcgctagt 1080

ggcagcgcaa gcaccagcaa cgaagctacc agcagcgcga atgctggcaa tatagctcct 1140

cggtcggcta cagatcgtga ggaacgcaaa gacacctcaa cagccagctc atcggaacca 1200

aatgcaaaga cagtcgctgc aactgcaagt tccacctcct cgaccacctc gacttcagca 1260

tcagccaact cggtttcatc cgatagcaat ccctacaagt catttcgcgt caccctcgat 1320

gatccttgct ataaagtgct tccagccgca cttaagaagt acaagatcaa cgacgattgg 1380

cgtaagtatg cgctcttcat ctgctacggc aagacagagc ggtgtctcag ctacgatgag 1440

aagccgcttc ttcttttcca gaagctcaag gagaatgaac aaagcccggt cttcatgctg 1500

cggcacatcc gcgacgtcaa gtcgcccatc gctattgctg aggccaaggc ggcgtccagg 1560

ctacaagagc gtgagggcga tggcaacgcc cctgccaaga agcgggcagc gccaagagcg 1620

gatcgacgcc gggttatcgc tggcacacca ccaccaggag cgttggtcac aaacacttcg 1680

aatccagtcg aagttggccc cgccgcagat ctgcctgacc gaaacaagat caccaagacg 1740

tacgcagtcg ccatttatcc ttacatgcca gaacgcgacg acgagttcga tgtcaatgtg 1800

ggtgatacct ttgtcgtcat caacaaggcc aagggctggt ggatcgtgca gagggatgcc 1860

aagggcgacg gagcaggtga tgtggtatat agcgtacctg ctagcgaagg aaacccagcg 1920

gatgacgaca gcatcaccta ctccaattat cgagcagaac atgcttcggg ctgggtacct 1980

gcaggttgtc ttctcgagac cagccgtcca ttgggtagta tcgtcgatgc cgaagtggtt 2040

tcatcgcgca tgcgatcagg accaagtacc atctcgaacc cgagcacgcc gaccttctca 2100

accatctccg gcagtagcgg cagcaccagc ggaggcaaga tggatgccaa gcgcgcttca 2160

atcccgccag cactcatcac gagcacgagc acaccaggta tcatgttgat ggactaccaa 2220

agtgctgaag gcgacttgga cttgaggaag gacgaacgat tgagagtgtt caagcggtac 2280

aatcactggt cgtactgcgt tcaggaacga gatggacatg cgagaggatg ggtgccaagc 2340

tggtatattg gc 2352

<210> 27
<211> 432
<212> DNA
<213> Ustilago cynodontis

<400> 27
atggctatga agagaatcaa caaggagctc gccgatctcg gccgtgatcc tccctcctcc 60

tgctccgctg gtcctaccgg cgacaacatg ttccaatggc aagcaacaat catgggcccc 120

ggtgactctc cctacgccgg tggtgtcttc ttcctttcca tcaccttccc taccgactac 180

cccttcaagc cacccaaggt ctcgttctcc accaagatct accaccccaa catcaacagc 240

aacggcagca tctgtctcga cattctcagg gatcagtgga gtcctgcgct caccatctct 300

aaggtcttgc tctcgatctg ctccatgctc actgacccaa atcccgatga cccgctcgta 360

cccgagattg ccaacttgta caagaccgac cgtcagcgat acgagagcac tgctcgtgaa 420

tggaccagga ag                                                          432

<210>  28
<211>  2493
<212>  DNA
<213>  Ustilago cynodontis

<400>  28
atgcctccga caacgacacg cctccagacc aggcaagccg gtctaagcgc acaagaggca      60

gcttcgtctg actctgggcg tcgccatcat atctctgtaa acctcttcca tggctggtca     120

acagaaacac ctgccagtgg cagctcgaca tcaaggcagg aaagtatcat ggtcaagcga     180

tccctttctc aagaacatct ggatatggtc atgccagcga gccagtcagc acccatcttt     240

cagaagatct cattggcaaa gggcgacccg ggctcttcta gcctgtcgca ggatgatgag     300

gaagaagctg aaaggatcaa gcacctaccg ccatccaagc gtcctggtgt ctcgcacgcc     360

aagaaaactc cgcccaacca catcaagcgc cctcgcaacg cctacatcat ctttcgatct     420

cacactgtct cacaaaagct catccctaaa gaggtcgaaa acgatcatcg caacatcagc     480

cgtatcattg cgcatatgtg gaagagtctg gatccggaag accgtgcgta ctatgaggag     540

atcgcccggg aggagaagga acgccacaag aggctcttcc ccaactacag gtaccagccc     600

accacgcgtc gtggtgacgt caagcaacgc aacgtcaaga agctcgagaa cggtgaagag     660

gaatgtcaag agatcgcaga catcattctc aaggcgcaag gcaaagaagg cgtcgttgtt     720

cgaccagagc cgtccaaacc tatgaaaagg gcacgcgagg cgacgcgtcc tcgcagcaag     780

cgctactcga cggcaactcc agttcgtaag cgagtcaaga cagcaaagac gccggaaagc     840

gatgctcaaa gctcggtgca gacgcttcag accgtcttcc tccatccttc accttcgaca     900

tcgccaacaa cggcacccag cagcggcggt gttggagctg tctcgacgcc ttcgccccag     960

tctttccggc tgggttcgag aacgccttcg atcgacaata gcatgcctca agcaccttca    1020

gcagagcctc tccctatcac ggaagacgtg ttcggacgca gagcgtcctc cgtccctgtt    1080

ctacgacctt gctcgccccc accagctttt gtttcagaat cgcagccatc tcctgtgttg    1140

gaatctcgcg acttggtctc gtcggcagag gcgtctggtc gcgaaagggc gatgcaggat    1200

ggtatcagat cgagctcagt acaaagccga gtaggctttg ccgatgagca ggggatgccg    1260

gctccctcct ggaaaggtcg caaagcgctg cctcctccca tcccgaacac ttggcaacat    1320

tgctccttcg acgatcagtc gctcccttcg cctcgctcgt ttgatgccat gggggctgct    1380

ggtaagccgg caatggctcg acctcgcacc gcgtaccctt cgacgccaag ttcgggttcg    1440

ttccgcggct tcttccatcc ctgggctttc gacgatggta ccgagtcgat gctcatctct    1500

cccatgactg cctcattcca agacacgagg aggcggagct ccttggtccg atccggcttg    1560

gtcgctggtc gacgacctgg atcgcttggc ttggatgcgc acaacaactt aggcgtccgt    1620

cctgatccgg tcgatggatc gcatgccttc gtttctgggc ccgattcgca tatctcagac 1680

gttcagcttt tcgacgaggc gactcgagct gcagaaatca gtctgggaat agagtcatcc 1740

cacggcgagc acgtagacgt cagtacattt gcttttgatc ccgcgctcga gggcgaagga 1800

ggaggtcaag cgcagttgtc ttcgtttcat ggccaagtgg ctcttcgcga gcaccaagcc 1860

ttaccgtcgc ccgttgcatc tccacgattg atggcagata gcccccgccc ttctttctcg 1920

ggcagcacgc tggctgctgc tgcgcgcgac tgggctagca tgaaaaagcg tcgaagtcac 1980

catgagcatc gaagcgtcct cgatccggtc caggactcga tttcggtacc taccgagggc 2040

atcatggcac cggctgaagc aagagtcgga gcagcaacaa attcgagcca gcaaaggtcc 2100

gccctggagg agtcgattga gcgggccgtt ctgcttgcgc tcggcagaga ctcgggtgat 2160

ggcgccgaca gtggcgaccg caactcacaa atcgttcagc aaatcttgtc ttcgctcaat 2220

gccgagctct cgcttcagca aggttcagcc catggcatcg ccacggctgg caaggcctcg 2280

tcgccgatcc tcgagccagg tcgcaggtct tcgctccgcg gatccgttgt tggtcctcct 2340

tacagatcgg cgccatactt ctctgggctc agctccattg atcatcaaag gaatctcaag 2400

ccgcttccgt caccattgca gcttgtcatg tcgaaccatg agatctcttc ggaggcttat 2460

tctcactctt atcccacttc ccagcattgc atc 2493

<210> 29
<211> 45
<212> DNA
<213> artificial sequence

<220>
<223> oligonucleotide primer HT-168

<400> 29
ctcgagtttt tcagcaagat cttctacaag cgtgatttac aaagg 45

<210> 30
<211> 28
<212> DNA
<213> Artificial sequence

<220>
<223> oligonucleotide primer HT-169

<400> 30
acttctggcc gtggtcagtc agagggcg 28

<210> 31
<211> 29
<212> DNA
<213> Artificial sequence

<220>
<223> oligonucleotide primer HT-172

<400>  31
acttctggcc gttgggcaac actgtattc                                          29


<210>  32
<211>  39
<212>  DNA
<213>  Artificial sequence

<220>
<223>  oligonucleotide primer HT-173

<400>  32
aggagatctt ctagaaagat gtctctctct caagcacac                               39


<210>  33
<211>  40
<212>  DNA
<213>  Artificial sequence

<220>
<223>  oligonucleotide primer HT-180

<400>  33
ctcgagtttt tcagcaagat tccgctttga ggtacagttg                              40


<210>  34
<211>  29
<212>  DNA
<213>  Artificial sequence

<220>
<223>  oligonucleotide primer HT-181

<400>  34
acttctggcc ggtcgcccct gtgatagtg                                          29


<210>  35
<211>  28
<212>  DNA
<213>  Artificial sequence

<220>
<223>  oligonucleotide primer HT-184

<400>  35
acttctggcc gatggcagcc ctaatgag                                           28


<210>  36
<211>  38
<212>  DNA
<213>  Artificial sequence

<220>
<223>  oligonucleotide primer HT-185

<400>  36
aggagatctt ctagaaagat gcccacatta gcaggtgg                                38

<210>  37
<211>  38
<212>  DNA
<213>  Artificial sequence

<220>
<223>  oligonucleotide primer HT-174

<400>  37
ctcgagtttt tcagcaagat ccaaatccgc cgtcggag                                38


<210>  38
<211>  34
<212>  DNA
<213>  Artificial sequence

<220>
<223>  oligonucleotide primer HT-175

<400>  38
acttctggcc aagtggaagt gttggaagca tttc                                    34


<210>  39
<211>  29
<212>  DNA
<213>  Artificial sequence

<220>
<223>  oligonucleotide primer HT-178

<400>  39
acttctggcc tgagcaagag agtgtgttc                                          29


<210>  40
<211>  38
<212>  DNA
<213>  Artificial sequence

<220>
<223>  oligonucleotide primer HT-179

<400>  40
aggagatctt ctagaaagat cacgatcaca cttccatc                                38


<210>  41
<211>  28
<212>  DNA
<213>  Artificial sequence

<220>
<223>  oligonucleotide primer HT-170

<400>  41
gactgaccac ggccagaagt tcctattc                                           28


<210>  42
<211>  28

<212>  DNA
<213>  Artificial sequence

<220>
<223>  oligonucleotide primer HT-171

<400>  42
gttgcccaac ggccagaagt tcctatac                                          28


<210>  43
<211>  28
<212>  DNA
<213>  Artificial sequence

<220>
<223>  oligonucleotide primer HT-176

<400>  43
acttccactt ggccagaagt tcctattc                                          28


<210>  44
<211>  28
<212>  DNA
<213>  Artificial sequence

<220>
<223>  oligonucleotide primer HT-177

<400>  44
ctcttgctca ggccagaagt tcctatac                                          28


<210>  45
<211>  28
<212>  DNA
<213>  Artificial sequence

<220>
<223>  oligonucleotide primer HT-182

<400>  45
aggggcgacc ggccagaagt tcctattc                                          28


<210>  46
<211>  28
<212>  DNA
<213>  Artificial sequence

<220>
<223>  oligonucleotide primer HT-183

<400>  46
ggctgccatc ggccagaagt tcctatac                                          28


<210>  47
<211>  20
<212>  DNA
<213>  Artificial sequence

<220>
<223>  oligonucleotide primer HT-100

<400>  47
aagggtggca tcgaggagag                                                   20


<210>  48
<211>  20
<212>  DNA
<213>  Artificial sequence

<220>
<223>  oligonucleotide primer HT-101

<400>  48
ctcgagccac ttagcctttc                                                   20


<210>  49
<211>  20
<212>  DNA
<213>  Artificial sequence

<220>
<223>  oligonucleotide primer HT-109

<400>  49
tccgctttga ggtacagttg                                                   20


<210>  50
<211>  20
<212>  DNA
<213>  Artificial sequence

<220>
<223>  oligonucleotide primer HT-110

<400>  50
ccacattagc aggtggtatc                                                   20


<210>  51
<211>  20
<212>  DNA
<213>  Artificial sequence

<220>
<223>  oligonucleotide primer HT-117

<400>  51
gacaagctca gtgccctctc                                                   20


<210>  52
<211>  20
<212>  DNA
<213>  Artificial sequence

<220>
<223>  oligonucleotide primer HT-118

<400>  52
cacttccatc ggaacatgtg                                                    20


<210>  53
<211>  18
<212>  DNA
<213>  Artificial sequence

<220>
<223>  oligonucleotide primer HT-119

<400>  53
cgatcccgcg attatcac                                                      18


<210>  54
<211>  18
<212>  DNA
<213>  Artificial sequence

<220>
<223>  oligonucleotide primer HT-120

<400>  54
ccagtcactc gctcattc                                                      18


<210>  55
<211>  18
<212>  DNA
<213>  Artificial sequence

<220>
<223>  oligonucleotide primer HT-121

<400>  55
acgattgtgc caagcttc                                                      18


<210>  56
<211>  18
<212>  DNA
<213>  Artificial sequence

<220>
<223>  oligonucleotide primer HT-122

<400>  56
gcagctgatt gatgttgg                                                      18


<210>  57
<211>  18
<212>  DNA
<213>  Artificial sequence

<220>
<223>  oligonucleotide primer HT-123

<400>  57
ccatgtctag ctcctttc                                                      18

<210>  58
<211>  18
<212>  DNA
<213>  Artificial sequence

<220>
<223>  oligonucleotide primer HT-124

<400>  58
cgagacaggt tgactttc                                                    18


<210>  59
<211>  39
<212>  DNA
<213>  Artificial sequence

<220>
<223>  oligonucleotide primer HT-228

<400>  59
ctcgagtttt tcagcaagat ttacaattct ttgcctctc                             39


<210>  60
<211>  29
<212>  DNA
<213>  Artificial sequence

<220>
<223>  oligonucleotide primer HT-229

<400>  60
acttctggcc ctcgctatac ttgcaaatg                                        29


<210>  61
<211>  31
<212>  DNA
<213>  Artificial sequence

<220>
<223>  oligonucleotide primer HT-232a

<400>  61
acttcccggg gaacatgttt cagaatgctg c                                     31


<210>  62
<211>  39
<212>  DNA
<213>  Artificial sequence

<220>
<223>  oligonucleotide primer HT-233

<400>  62
aggagatctt ctagaaagat tcggggatac catcacgag                             39


<210>  63
<211>  28

<212>  DNA
<213>  Artificial sequence

<220>
<223>  oligonucleotide primer HT-230

<400>  63
gtatagcgag ggccagaagt tcctattc                                          28


<210>  64
<211>  28
<212>  DNA
<213>  Artificial sequence

<220>
<223>  oligonucleotide primer HT-231a

<400>  64
aaacatgttc ggccagaagt tcctatac                                          28


<210>  65
<211>  20
<212>  DNA
<213>  Artificial sequence

<220>
<223>  oligonucleotide primer HT-234

<400>  65
tgatcttctg cgagccgaac                                                   20


<210>  66
<211>  20
<212>  DNA
<213>  Artificial sequence

<220>
<223>  oligonucleotide primer HT-235

<400>  66
gcttggagga agccgatctg                                                   20


<210>  67
<211>  20
<212>  DNA
<213>  Artificial sequence

<220>
<223>  oligonucleotide primer HT-236

<400>  67
tttgcctgcc gtcaacactg                                                   20


<210>  68
<211>  20
<212>  DNA
<213>  Artificial sequence

<220>
<223>  oligonucleotide primer HT-237

<400>  68
ttcgcccaca cgatgatcgg                                                    20


<210>  69
<211>  39
<212>  DNA
<213>  Artificial sequence

<220>
<223>  oligonucleotide primer HT-204

<400>  69
ctcgagtttt tcagcaagat ccgatcgctg ttaggacac                               39


<210>  70
<211>  30
<212>  DNA
<213>  Artificial sequence

<220>
<223>  oligonucleotide primer HT-205

<400>  70
acttctggcc cgtgaaacgt tgcaaaacag                                         30


<210>  71
<211>  30
<212>  DNA
<213>  Artificial sequence

<220>
<223>  oligonucleotide primer HT-208

<400>  71
acttctggcc ccgactgaga gattatggtc                                         30


<210>  72
<211>  39
<212>  DNA
<213>  Artificial sequence

<220>
<223>  oligonucleotide primer HT-209

<400>  72
aggagatctt ctagaaagat aatcggaacc gtgtacctg                               39


<210>  73
<211>  28
<212>  DNA
<213>  Artificial sequence

<220>
<223>  oligonucleotide primer HT-206

<400>  73
acgtttcacg ggccagaagt tcctattc                                          28


<210>  74
<211>  28
<212>  DNA
<213>  Artificial sequence

<220>
<223>  oligonucleotide primer HT-207

<400>  74
tctcagtcgg ggccagaagt tcctatac                                          28


<210>  75
<211>  20
<212>  DNA
<213>  Artificial sequence

<220>
<223>  oligonucleotide primer HT-210

<400>  75
tcgctgttag gacacaactg                                                   20


<210>  76
<211>  20
<212>  DNA
<213>  Artificial sequence

<220>
<223>  oligonucleotide primer HT-211

<400>  76
ccgtgtacct ggctgtgtag                                                   20


<210>  77
<211>  20
<212>  DNA
<213>  Artificial sequence

<220>
<223>  oligonucleotide primer HT-210a

<400>  77
tcggtgtgcg gcgatttctg                                                   20


<210>  78
<211>  20
<212>  DNA
<213>  Artificial sequence

<220>
<223>  oligonucleotide primer HT-4a

<400>  78
acagacgtcg cggtgagttc                                                   20

<210>  79
<211>  18
<212>  DNA
<213>  Artificial sequence

<220>
<223>  oligonucleotide primer JB-9

<400>  79
cttcaaggcc accacaac                                                    18


<210>  80
<211>  20
<212>  DNA
<213>  Artificial sequence

<220>
<223>  oligonucleotide primer JB-64

<400>  80
tgctgccatg gtgctactcc                                                  20